# EUROPEAN PATENT APPLICATION

(11) **EP 3 875 585 A1**
(43) Date of publication of application: **08.09.2021**
(21) Application number: 19879622.9
(22) Date of filing: 01.11.2019
(51) Int. Cl.: C12N 15/10, C12Q 1/6806, C12Q 1/6883, G01N 33/50, G01N 33/68, G01N 33/92

(54) **METHOD FOR PREPARING NUCLEIC ACID DERIVED FROM SKIN CELL OF SUBJECT**

(30) Priority: 01.11.2018 JP 2018206938
(71) Applicant: Kao Corporation, Chuo-ku, Tokyo 103-8210 (JP)
(72) Inventor: INOUE, Takayoshi, Haga-gun, Tochigi 321-3497 (JP); UEHARA, Yuya, Haga-gun, Tochigi 321-3497 (JP); YAJIMA, Kotomi, Odawara-shi, Kanagawa 250-0002 (JP)
(74) Representative: Vossius & Partner Patentanwälte Rechtsanwälte mbB
(86) International application number: PCT/JP2019/043040
(87) International publication number: WO 2020/091044

(57) **Abstract**

Provided is a method for analyzing RNA of a subject with high accuracy. The present invention provides a method for preparing a nucleic acid derived from a skin cell of a subject, the method comprising preserving at 0°C or lower an RNA-containing skin surface lipid collected from the subject; and a method for preparing a nucleic acid derived from a skin cell of a subject, the method comprising converting RNA has been contained in a skin surface lipid of the subject into cDNA, then amplifying the cDNA by multiplex PCR, and purifying the resulting reaction product.

## Description

### Field of the Invention

The present invention relates to a method for preparing a nucleic acid derived from a skin cell of a subject, and a method for analyzing a skin of a subject using the nucleic acid.

### Background of the Invention

Techniques for examining current and even future internal physiological conditions of human living bodies by analyzing molecules in biological samples (e.g. nucleic acids, proteins and metabolic substances) have been developed. In particular, analysis using nucleic acid molecules has the advantage that an abundance of information can be obtained by one analysis because an exhaustive analysis method has been established, and that it is easy to functionally link analysis results on the basis of many study reports related to single-nucleotide polymorphisms, RNA functions and the like.

Among various tissues of living bodies, skins receive attention as tissues which contact the outside, and therefore enable collection of biological samples with low invasiveness. As a method for non-invasively collecting a nucleic acid from a skin and analyzing the nucleic acid, a method has been reported in which a human skin sample is collected by wiping the skin surface with a wetted cotton swab, and RNA profiling is performed (Non-Patent Literature 1). Patent Literature 1 indicates that a nucleic acid derived from a skin cell of a subject, such as RNA, is separated from a skin surface lipid, and used as a sample for analysis of a living body.

(Patent Literature) International Publication No. WO2018/008319
(Non-Patent Literature) Forensic Sci Int Genet, 2012, 6(5): 565-577

### Summary of the Invention

In an embodiment, the present invention provides a method for preparing a nucleic acid derived from a skin cell of a subject, the method containing preserving at 0°C or lower an RNA-containing skin surface lipid collected from the subject.

In another embodiment, the present invention provides a method for preparing a nucleic acid derived from a skin cell of a subject, the method containing: converting RNA which has been contained in a skin surface lipid of the subject into cDNA by reverse transcription, and then subjecting the cDNA to multiplex PCR; and purifying a reaction product of the PCR.

In another embodiment, the present invention provides a method for analyzing a condition of a skin, a part other than the skin or the whole body of a subject, the method containing analyzing the nucleic acid prepared by the above-described method.

In another embodiment, the present invention provides a method for evaluating the effect or the efficacy of a skin external preparation, an intracutaneously administered preparation, a patch, an oral preparation or an injection on a subject, the method containing analyzing the nucleic acid prepared by the above-described method.

In another embodiment, the present invention provides a method for analyzing a concentration of a component in the blood of a subject, the method containing analyzing the nucleic acid prepared by the above-described method.

### Brief Description of the Drawings

[Figure 1] Figure 1 shows an effect of the preservation temperature on the stability of RNA in SSL. 18S: 18S ribosomal RNA and 28S: 28S ribosomal RNA.
[Figure 2] Figure 2 shows expression of atopic dermatitis-related marker in SSL-derived RNA.
[Figure 3] Figure 3 shows a predicted value of a blood testosterone concentration from a SSL-derived RNA expression level based on a machine learning model. The ordinate represents the predicted value, and the abscissa represents a measured value.
[Figure 4] Figure 4 shows predicted results of the concentrations of various components in the blood from the SSL-derived RNA expression level based on the machine learning model.
[Figure 5] Figure 5 shows a grouping of subjects based on the expression levels (day 0) of 22 types of RNAs whose expression is increased by use of a facial cleanser.
[Figure 6] Figure 6 shows a change in horny cell layer moisture content by use of the facial cleanser.
[Figure 7] Figure 7 shows a proportion of persons feeling the moisturizing effect after use of the facial cleanser, which is based on a questionary result.
[Figure 8] Figure 8 shows expression of BSG and HCAR2 in a group with a high sebum secretion volume and a group with a low sebum secretion volume.
[Figure 9] Figure 9 shows expression of ASPRV1 and PADI3 in a group with a high moisture content and a group with a low moisture content.
[Figure 10] Figure 10 shows expression of SOCS3, JUNB and IL-1B in a group with high skin redness and a group with low skin redness.
[Figure 11] Figure 11 shows a predicted value of a skin condition from the SSL-derived RNA expression level based on the machine learning model. The ordinate represents the predicted value, and the abscissa represents a measured value.
[Figure 12] Figure 12 shows a predicted value of a blood cortisol concentration from the SSL-derived RNA expression level based on the machine learning model. The ordinate represents the predicted value, and the abscissa represents a measured value.
[Figure 13] Figure 13 shows a predicted value of a cumulative ultraviolet exposure time from the SSL-derived RNA expression level based on the machine learning model. The ordinate represents the predicted value, and the abscissa represents a calculated value based on the questionary results from subjects.

### Detailed Description of the Invention

All the patent literatures, non-patent literatures and other publications cited in the present description are incorporated herein by reference in their entirety.

The names of the genes disclosed in the present description follow Official Symbol described in NCBI ([www.ncbi.nlm.nih.gov/]). On the other hand, with regard to the gene ontology (GO), the names of the genes follow Pathway ID described in String ([string-db.org/]).

The present invention relates to a method for preparing a nucleic acid derived from a skin cell of a subject, and a method for analyzing a skin of a subject using the nucleic acid.

The method of the present invention enables stable preservation of a nucleic acid sample derived from a skin cell has been contained in a skin surface lipid of a subject. Therefore, the present invention improves the accuracy of analysis using the nucleic acid sample (e.g. gene analysis and diagnosis). Further, since the concentration of a specific marker gene-derived component has been contained in the skin cell-derived nucleic acid sample prepared by the method of the present invention correlates to the concentrations of various components present in the blood, use of the nucleic acid sample enables non-invasive measurement of the concentration of a component in the blood.

RNA has the property of being easily decomposed, and is therefore usually preserved under a particular low-temperature condition of -80°C except when the RNA is specifically treated. When a sample having a reduced amount of RNA due to decomposition is used, the accuracy of analysis decreases. Even when RNA is converted into cDNA by reverse transcription reaction and preserved, the accuracy of analysis decreases because a sufficient amount of cDNA cannot be obtained if the original RNA is unstable.

Previously, the present inventors found that a lipid present on a skin surface (skin surface lipid) contains RNA derived from a skin cell of a subject, and use of the RNA enables biological analysis, and the present inventors applied for a patent (Patent Literature 1). Here, further, the present inventors found that RNA has been contained in the skin surface lipid can be preserved under a general low-temperature condition, and can be stably preserved under a condition other than a conventional particular low-temperature condition of - 80°C. Further, the present inventors found that by subjecting RNA separated from the skin surface lipid to reverse transcription reaction and PCR under predetermined conditions, and then purifying the RNA, a sufficient amount of a nucleic acid sample for analysis can be obtained even from a skin surface lipid having a low RNA content.

Accordingly, in an aspect, the present invention provides a method for preparing a nucleic acid derived from a skin cell of a subject. In an embodiment, the method for preparing a nucleic acid derived from a skin cell of a subject according to the present invention comprises preserving at 0°C or lower an RNA-containing skin surface lipid collected from a subject. In another embodiment, the method for preparing a nucleic acid derived from a skin cell of a subject according to the present invention comprises converting RNA has been contained in the skin surface lipid of a subject into cDNA by reverse transcription, then subjecting the cDNA to multiplex PCR, and purifying a reaction product of the PCR.

In the present description, the "skin surface lipid (SSL)" refers to a lipid-soluble fraction present on a skin surface, and is sometimes referred to as sebum. In general, SSL mainly contains secretions secreted from an exocrine gland such as a sebaceous gland on the skin, and is present on the skin surface in the form of a thin layer covering the skin surface.

In the present description, the "skin" is a generic term for regions including tissues of the surface skin, the dermis, the follicle, the sweat gland, the sebaceous gland and other glands of the body surface, unless otherwise specified.

Examples of the nucleic acid derived from a skin cell of a subject and prepared by the method of the present invention include, without limitation, DNA and RNA, and RNA or DNA prepared from the RNA is preferable. Examples of RNA include mRNA, tRNA, rRNA, small RNA (e.g. microRNA (miRNA), small interfering RNA (siRNA) and Piwi-interacting RNA (piRNA)) and long intergenic non-coding (linc) RNA. The mRNA is RNA encoding a protein, and often has a length of 1,000 nt or more. Each of the miRNA, the siRNA, the piRNA and the lincRNA is non-coding (nc) RNA which does not encode a protein. The miRNA is small RNA having a length of from 19 to 30 nt among ncRNAs. The lincRNA is long non-coding RNA having poly-A like mRNA, and has a length of 200 nt or more (Non-Patent Literature 1). More preferably, the RNA prepared in the method of the present invention is RNA having a length of 200 nt or more. Still more preferably, the RNA prepared in the method of the present invention is at least one selected from the group consisting of mRNA and lincRNA. Examples of the DNA prepared in the present invention include cDNA prepared from the aforementioned RNA, and reaction products (e.g. PCR products and clone DNA) from the cDNA.

The subject in the method of the present invention may be an organism having SSL on the skin. Examples of the subject include mammals including humans and non-human mammals, with humans being preferable. Preferably, the subject is a human or a non-human mammal needing or desiring analysis of its nucleic acid. Preferably, the subject is a human or a non-human mammal needing or desiring analysis of gene expression on the skin, or analysis of the condition of the skin or a part other than the skin using a nucleic acid.

SSL collected from a subject includes RNA expressed on a skin cell of the subject, preferably RNA expressed on any of the surface skin, the sebaceous gland, the follicle, the sweat gland and the dermis of the subject, more preferably RNA expressed on any of the surface skin, the sebaceous gland, the follicle and the sweat gland (see Patent Literature). Therefore, the RNA derived from a skin cell of a subject and prepared by the method of the present invention is preferably RNA derived from at least one part selected from the group consisting of the surface skin, the sebaceous gland, the follicle, the sweat gland and the dermis of the subject, more preferably RNA derived from at least one part selected from the group consisting of the surface skin, the sebaceous gland, the follicle and the sweat gland.

In an embodiment, the method of the present invention may further comprise collecting SSL from a subject. Examples of the part of the skin, from which SSL is collected, include, but are not limited to, skins of any part of the body such as the head, the face, the neck, the body trunk or the limb, skins having a disease such as atopy, acne, dryness, inflammation (redness) or a tumor, and skins having a wound. Preferably, the part of the skin from which SSL is collected does not include the palm, the back, the sole of the foot, or the finger skin.

For collection of SSL from the skin of a subject, any means used for collecting or removing SSL from the skin can be employed. Preferably, a SSL absorbing, a SSL bonding material or a device for scraping off SSL from the skin as described below can be used. The SSL absorbing material or the SSL bonding material is not limited as long as it is a material having affinity for SSL, and examples thereof include polypropylene and pulp. Specific examples of the procedure for collecting SSL from the skin include a method in which SSL is absorbed into a sheet-shaped material such as an oil blotting paper or an oil blotting film; a method in which SSL is bonded to a glass plate, a tape or the like; and a method in which SSL is scraped off with a spatula or a scraper. A SSL absorbing material containing a solvent with high lipid solubility beforehand may be used for improving the SSL adsorption property. On the other hand, when the SSL absorbing material contains a solvent with high water solubility or moisture, adsorption of SSL is inhibited, and therefore the content of a solvent with high water solubility or moisture is preferably low. It is preferable that the SSL absorbing material be used in a dried state.

In an embodiment of the present invention, the collected RNA-containing SSL is preserved under a low-temperature condition of 0°C or lower. It is preferable that the collected RNA-containing SSL be preserved under a predetermined low-temperature condition as soon as possible after the collection for suppressing decomposition of RNA as much as possible. The temperature condition for preservation of the RNA-containing SSL in the present invention may be 0°C or lower, and is preferably from -20 ± 20°C to -80 ± 20°C, more preferably from -20 ± 10°C to -80 ± 10°C, still more preferably from -20 ± 20°C to -40 ± 20°C, even more preferably from -20 ± 10°C to -40 ± 10°C, even more preferably -20 ± 10°C, even more preferably -20 ± 5°C. This temperature condition is much milder than a conventional general RNA preservation condition (e.g. - 80°C). Therefore, preservation of the RNA-containing SSL in the present invention under a preferred low-temperature condition does not require use of special equipment such as an ultracold freezer or a dedicated preservation container, and can be performed by using a usual freezer or a freezing chamber of a refrigerator. The period of preservation of the RNA-containing SSL in the present invention under the low-temperature condition is preferably 12 months or less, for example 6 hours or more and 12 months or less, more preferably 6 months or less, for example 1 day or more and 6 months or less, still more preferably 3 months or less, for example 3 days or more and 3 months or less, without limitation.

For separation of RNA from the collected RNA-containing SSL, a method which is normally used for extraction or purification of RNA from a biological sample can be used, for example a phenol/chloroform method, an AGPC (acid guanidinium thiocyanate-phenolchloroform extraction) method, a method using a column such as TRIzol (registered trademark), RNeasy (registered trademark) or QIAzol (registered trademark), a method using special magnetic particles coated with silica, a method using solid phase reversible immobilization magnetic particles, or extraction with a commercially available RNA extraction reagent such as ISOGEN can be used.

In another embodiment of the present invention, RNA separated from the RNA-containing SSL (SSL-derived RNA) can be used as it is for various analyses. In a preferred embodiment, the SSL-derived RNA is converted into DNA. Preferably, the SSL-derived RNA is converted into cDNA by reverse transcription, the cDNA is then subjected to PCR, and the resulting reaction product is purified. For the reverse transcription, a primer targeting specific RNA to be analyzed, and it is preferable to use a random primer for more comprehensive preservation and analysis. In the PCR, only the specific DNA may be amplified using a primer pair targeting specific DNA to be analyzed, and a plurality of DNAs may be amplified using a plurality of primer pairs. Preferably, the PCR is multiplex PCR, which is a method for simultaneously amplifying a plurality of gene regions by simultaneously using a plurality of primer pairs in the PCR reaction system. The multiplex PCR can be performed using a commercially available kit (e.g. Ion AmpliSeqTranscriptome Human Gene Expression Kit; Life Technologies Japan Ltd.).

For the reverse transcription of RNA, a common reverse transcriptase or reverse transcription reagent kit can be used. Preferably, a reverse transcriptase or reverse transcription reagent kit with high accuracy and efficiency is used, and examples thereof include M-MLV reverse transcriptase and modified products thereof, or commercially available reverse transcriptases or reverse transcription reagent kits, for example PrimeScript (registered trademark) Reverse Transcriptase series (Takara Bio Inc.) and SuperScript (registered trademark) Reverse Transcriptase series (Thermo Scientific). SuperScript (registered trademark) III reverse Transcriptase and SuperScript (registered trademark) VILO cDNA Synthesis kit (each from Thermo Scientific), etc. are preferably used.

By adjusting the reaction conditions for the reverse transcription and PCR, the yield of the PCR reaction product is further improved, and hence the accuracy of analysis using the PCR reaction product is further improved. It is preferable that in elongation reaction in the reverse transcription, the temperature be adjusted to preferably 42°C ± 1°C, more preferably 42°C ± 0.5°C, still more preferably 42°C ± 0.25°C, and the reaction time be adjusted to preferably 60 minutes or more, more preferably from 80 to 100 minutes. Preferably, the temperature for annealing and elongation reaction in PCR is preferably 62°C ± 1°C, more preferably 62°C ± 0.5°C, still more preferably 62°C ± 0.25°C. Therefore, it is preferable that in the PCR, annealing and elongation reaction be carried out in one step. The time for the step of annealing and elongation reaction can be adjusted according to the size of DNA to be amplified, etc., and is preferably from 14 to 18 minutes. The condition for degeneration reaction in the PCR can be adjusted according to DNA to be amplified, and is preferably from 10 to 60 seconds at 95 to 99°C. Reverse transcription and PCR with the above-described temperature and time can be carried out using a thermal cycler which is commonly used in PCR.

It is preferable that purification of the reaction product obtained by the PCR be performed by size separation of the reaction product. The size separation enables separation of a desired PCR reaction product from the primer and other impurities contained in the PCR reaction liquid. The size separation of DNA can be performed with, for example, a size separation column, a size separation chip, magnetic beads usable for size separation, or the like. Preferred examples of the magnetic beads usable for size separation include solid phase reversible immobilization (SPRI) magnetic beads such as Ampure XP. When Ampure XP is mixed with a DNA solution, DNA is adsorbed to carboxy groups coated on the surfaces of the magnetic beads, and only the magnetic beads are recovered with a magnet to purify the DNA. When the mixing ratio of the Ampure XP solution to the DNA solution is changed, the molecular size of DNA adsorbed to the magnetic beads changes. By utilizing this principle, DNA with a specific molecular size, which is to be captured, can be recovered on the magnetic beads, while DNA with other molecular sizes and impurities are purified.

The purified PCR reaction product may be subjected to further treatment necessary for performing subsequent analysis. For example, for sequencing or fragment analysis, an appropriate buffer solution may be prepared from the purified PCR reaction product, PCR primer regions contained in DNA subjected to PCR amplification may be cut, or an adaptor sequence may be further added to the amplified DNA. Libraries for various analyses can be prepared by, for example, preparing a buffer solution from the purified PCR reaction product, subjecting the amplified DNA to removal of the PCR primer sequence and adaptor ligation, and amplifying the resulting reaction product if necessary. These operations can be carried out by, for example, using 5XVILO RT Reaction Mix attached to SuperScript (registered trademark) VILO cDNA Synthesis Kit (Life Technologies Japan Ltd.), 5XIon AmpliSeq HiFi Mix attached to Ion AmpliSeq Transcriptome Human Gene Expression Kit (Life Technologies Japan Ltd.), and Ion AmpliSeq Transcriptome Human Gene Expression Core Panel and following the protocols attached to the kits.

The SSL-derived RNA which is subjected to the reverse transcription and PCR may be RNA derived from RNA-containing SSL immediately after collection from a living body, or RNA derived from RNA-containing SSL preserved at room temperature or refrigerated after collection from the living body, and is preferably RNA derived from RNA-containing SSL preserved at 0°C or lower after collection from the living body. The preservation at 0°C or lower may be preservation at -80°C, and is preferably preservation at -20 ± 10°C, more preferably preservation at -20 ± 5°C. The SSL-derived RNA may be used for the reverse transcription or PCR immediately after being separated from SSL, or may be stored by a usual method until being used.

A nucleic acid derived from a skin cell of a subject and prepared from SSL-derived RNA by the method of the present invention can be used for various analyses or diagnoses using nucleic acids. Accordingly, the present invention also provides a method for analyzing a nucleic acid, the method containing analyzing a nucleic acid prepared by the method for preparing a nucleic acid according to the present invention. The nucleic acid is a nucleic acid prepared by the method for preparing a nucleic acid according to the present invention. Examples of analysis and diagnosis which can be performed using the nucleic acid prepared according to the present invention include:
(i) analysis of gene expression related to the skin of the subject, analysis of other gene information, analysis of functions related to the skin of the subject, which is based on the above-mentioned analyses, and the like;
(ii) analysis of a disease or a condition of the skin of the subject, for example evaluation of a health condition of the skin (skin condition such as sebum secretion, moisture content, redness, atopic dermatitis or sensitive skin), estimation of a current skin condition or prediction of a future skin condition, prediction of past histories of the skin such as a cumulative ultraviolet exposure time, diagnosis or prognosis of skin disease, diagnosis or prognosis of skin cancer, evaluation of subtle change of the skin, and the like;
(iii) evaluation of effects or efficacy of a skin external preparation, an intracutaneously administered preparation, a patch, an oral preparation or an injection with the utilization of the analysis of a disease or a condition of the skin of the subject;
(iv) analysis of a condition of a part other than the skin, or the whole body of the subject, for example evaluation of a general health condition or prediction of a future general health condition, diagnosis or prognosis of various diseases such as neural disease, cardiovascular disease, metabolic disease and cancer, and the like; and
(v) analysis of the concentration of a component in the blood of the subject.

More specific examples of analysis and diagnosis using the nucleic acid prepared according to the present invention are described below.

### Analysis of gene expression

As disclosed in Patent Literature 1, SSL contains an abundance of high-molecular-weight RNA such as mRNA derived from the subject. SSL, which is a supply source of mRNA which can be non-invasively collected from the subject, is useful as a biological sample for analysis of gene expression. Further, the mRNA in SSL reflects gene expression profiles of the sebaceous gland, the follicle and the surface skin (see Examples 1 to 4 of Patent Literature 1). Therefore, the nucleic acid prepared according to the present invention is suitable as a biological sample for analysis of gene expression of the skin, particularly the sebaceous gland, the follicle and the surface skin.

### Analysis of skin

The skin of the subject can be analyzed by using as a sample the nucleic acid prepared according to the present invention. Examples of the analysis of the skin include the analysis of gene expression and the analysis of a skin condition. Examples of the analysis of a skin condition include detection of a skin with or a predetermined disease or condition or a skin without predetermined disease or condition. Examples of the predetermined disease or condition include, but are not limited to, deficiency or excess in amount of sebum, deficiency or excess in skin moisture content, redness, atopic dermatitis, and sensitive skin. For example, analysis of the expression level of a marker gene for a predetermined disease or condition such as an amount of sebum, a skin moisture content, redness, atopic dermatitis or sensitive skin in the skin of the subject from the nucleic acid prepared according to the present invention enables determination of whether or not the skin of the subject has the predetermined disease or condition. Preferably, comparison of the expression level of a marker gene for a predetermined disease or condition, which is obtained for the subject, with the expression level of the marker gene in the nucleic acid prepared by the method of the present invention from SSL of a group with the predetermined disease or condition (positive control) or a group without the predetermined disease or condition (negative control) enables determination of whether or not the skin of the subject has the predetermined disease or condition. As the marker gene, a known skin condition-related marker gene can be used.

Another example of analysis of the skin is prediction of a skin condition, and examples of prediction the skin condition include prediction of a skin physical property, prediction of visual or palpatory evaluation of the skin, and prediction of a sebum composition. Examples of the skin physical property include the horn cell layer moisture content, the transepidermal water loss (TEWL), the amount of sebum, the amount of melanin and the amount of erythema. Examples of the visual or palpatory evaluation of the skin include evaluation of a skin condition which is usually performed visually or on palpation by a professional evaluator. More specific examples of the visual evaluation include evaluation of the existence or non-existence or the degree of "cleanness", "clearness", "lightness", "luster", "flecks", "conspicuous dark circles", "yellowness", "overall redness", "textured wrinkles on the cheek", "drooping corners of the mouth", "scale", "acne", "conspicuous pores on the cheek", "conspicuous pores on the nose" and the like, and examples of the palpatory evaluation include evaluation of the existence or non-existence or the degree of "rough feeling", "moist feeling" and the like. Examples of the sebum composition include the amounts of components such as free fatty acid (FFA), wax ester (WE), cholesterol ester (ChE), squalene (SQ), squalene epoxide (SQepo), squalene oxide (SQOOH), diacylglycerol (DAG) and triacylglycerol (TAG).

As shown in Examples below, by correlational analysis of an RNA expression profile obtained from analysis of SSL-derived RNA and data for the measured values and evaluation values of various skin conditions linked to the RNA expression profile, genes closely related to various skin conditions can be selected and used for construction of a prediction model. Specific related genes used for prediction of a skin condition include genes shown in Table 8.

When a large number of gene data are analyzed as expression data of closely related genes used for construction of the prediction model, the prediction model may be constructed after the data are compressed by analysis of main components if necessary.

As an algorism in construction of the prediction model, a known algorism such as one that is used for machine learning. Examples of the machine learning algorism include algorisms such as those of linear regression model (Linear model), Lasso regression (Lasso), random forest (Random Forest), neural network (Neural net), linear kernel support vector machine (SVM (linear)) and rbf kernel support vector machine (SVM (rbf)). Data for verification is input to constructed prediction models to calculate predicted values. A model giving the smallest root-mean-square-error (RMSE) of a difference between a predicted value and a measured value can be selected as an optimum model.

Another example of analysis of the skin is prediction of a cumulative ultraviolet exposure time of the skin. In general, the cumulative ultraviolet exposure time is calculated with the ultraviolet exposure time predicted on the basis of questionary studies on the lifestyle habit and outdoor leisure activity. As shown in Examples below, by correlational analysis of an RNA expression profile obtained from analysis of SSL-derived RNA and calculated data of the cumulative ultraviolet exposure time linked to the RNA expression profile, genes closely related to the cumulative ultraviolet exposure time can be selected to construct a prediction model. The procedure for constructing the model is the same as described above.

Alternatively, the expression level of the nucleic acid prepared from SSL of a group with the predetermined disease or condition (positive control) or a group without the predetermined disease or condition (negative control) is analyzed. A gene for which there is a significant difference in expression level between both the groups can be used as a skin condition-related marker gene. Specifically, as the marker gene for atopic dermatitis, mention is made of one or more genes selected from a group of 1911 genes ((A) of Tables 7-1 to 7-24) whose expression is significantly lower in atopic dermatitis patients than in healthy persons in Test Example 6 below; and one or more genes selected from a group of 370 OR genes ((B) of Tables 7-1 to 7-11) whose expression is lower in atopic dermatitis patients than in healthy persons and a group of 368 OR genes ((C) of Tables 7-1 to 7-11) and a group of 284 OR genes ((D) of Tables 7-1 to 7-11) whose expression decreases in response to the severity of dermatitis, among olfactory receptors (ORs) contained in GO: 0050911 which is a biological process (BP) found to be closely related to atopic dermatitis. As the marker gene for sensitive skin, mention is made of one or more genes selected from a group of 693 genes ((E) of Tables 7-1 to 7-20) whose expression is significantly lower in a group with subjective symptoms of sensitive skin than in a group without subjective symptoms of sensitive skin in Test Example 7 below; and one or more genes selected from a group of 344 OR genes ((F) of Tables 7-1 to 7-10) whose expression is lower in a group with subjective symptoms than in a group without subjective symptoms, among olfactory receptors (ORs) contained in GO: 0050911 which is a biological process (BP) found to be closely related to sensitive skin. As the marker gene for redness, mention is made of one or more genes selected from a group of 703 genes ((G) of Tables 7-1 to 7-20) for which there is a significant difference in expression between a group with intense skin redness and a group with mild skin redness in Test Example 8 below. As the marker gene for the skin moisture content, mention is made of one or more genes selected from a group of 553 genes ((H) of Tables 7-1 to 7-16) for which there is a significant difference in expression between a group with a high horn cell layer moisture content and a low horn cell layer moisture content in Test Example 8 below. As the marker gene for the amount of sebum, mention is made of one or more genes selected from a group of 594 genes ((I) of Tables 7-1 to 7-17) for which there is a significant difference in expression between a group with a large amount of sebum and a group with a small amount of sebum in Test Example 8 below.

Further, on the basis of the analysis of a disease or a condition of the skin of the subject, the effect or efficacy of a given skin external preparation, an intracutaneously administered preparation, a patch, an oral preparation, an injection or the like on the subject can be evaluated. For example, by examining expression of a marker gene for a disease or a condition of the skin of the subject, the effect or efficacy of use of the skincare product on the skin of the subject can be evaluated. The marker for a disease or a condition of the skin, which is used for the evaluation, is, for example, one or more genes selected from the group consisting of BNIP3, CALML3, GAL, HSPA5, JUNB, KIF13B, KRT14, KRT17, KRT6A, OVOL1, PPIF, PRDM1, RBM3, RPLP1, RPS4X, SEPT9, SOAT1, SPNS2, UBB, VCP, WIPI2 and YPEL3.

### Analysis of the concentrations of various components in the blood

The concentrations of various components present in the blood of the subject can be analyzed by using as a sample the nucleic acid prepared according to the present invention. As shown in Examples below, it was possible to predict the concentration of a component in the blood of the subject from the expression level of related marker gene-derived RNA in SSL-derived RNA of the subject by using a machine learning model constructed on the basis of the expression level of related marker gene-derived RNA in SSL-derived RNA and data of the concentrations of various components in the blood. Therefore, the concentrations of various components in the blood can be determined on the basis of the expression level of related marker gene-derived RNA in SSL-derived RNA. The machine learning model can be constructed in accordance with the procedure for constructing a prediction model for the skin condition. Examples of various components present in the blood, which are analyzed according to the present invention, include hormones, insulin, neutral fat, γ-GTP and LDL-cholesterol. Examples of the hormone in the blood include androgens such as testosterone, dihydrotestosterone, androstenedione and dehydroepiandrosterone, estrogens such as estrone and estradiol, progesterone and cortisol. Of these, testosterone or cortisol is preferable. The related marker gene-derived RNA in SSL-derived RNA which is used for determination of the concentrations of various components in the blood can be selected from the group consisting of RNAs whose expression level has a relatively high correlation with the concentration of a component in the blood. Preferably, the expression level of SSL-derived RNA and the concentration of a target component in the blood are measured on a population, a correlation of the expression level of each RNA with the concentration of the component in the blood is examined, and RNA having a relatively high correlation is selected.

As an example of related marker gene-derived RNA in SSL-derived RNA which is used for determination of the concentration of each of, for example, testosterone, insulin, neutral fat, γ-GTP and LDL-cholesterol in the blood, mention is made of at least one selected from a group of RNAs derived from human genes shown below, preferably all of the RNAs.
(Testosterone) SCARNA16, PRSS27, RDBP, PSMB10, SBNO1, EMC3, MAR9, C20orf112, C14orf2 and CCDC90B;
(Insulin) EAPP, SDE2, LYAR, ZNF493, PSMB10, FAM71A, GPANK1, FGD4, MRPL43 and CMPK1; (Neutral fat) CCDC9, C6orf106, CERK, HSD3B2, SUN2, FNDC4, GRAMD1C, DGAT2, ALPL, HOMER3, MTHFS, ADIPOR1, RBM3, EXOC8 and ARHGEF37;
(y-GTP) TMEM38A, BTN3A2, NAP1L2, ABCA2, ALPL, SECTM1, C17orf62, GNB2, R3HDM4, LRG1, SBNO2, CD14, MLLT1, NINJ2 and LIMD2; and
(LDL-cholesterol) THTPA, LOC100506023, ZNF700, TAB3, PLEKHA1, ZNF845, FXC1, CUL4A, NDUFV1 and AMZ2.

A preferred procedure for determining the concentration of a component in the blood using SSL-derived RNA will be described below with determination of the blood testosterone concentration taken as an example. First, by machine learning in which data of the expression level of RNA of each of the 10 genes (SCARNA16, PRSS27, RDBP, PSMB10, SBNO1, EMC3, MAR9, C20orf112, C14orf2 and CCDC90B) having a high correlation with the blood testosterone concentration and contained in SSL-derived RNA obtained from a human population serves as an explanatory variable and data of the concentration of the blood testosterone obtained from the population serves as an objective variable, an optimum prediction model for predicting the blood testosterone concentration is constructed from the expression level of the RNA. On the other hand, SSL-derived RNA is collected from a human subject whose blood testosterone concentration is to be examined. On the basis of the constructed model, the predicted value of the blood testosterone concentration of the subject can be calculated from the data of the expression level of RNA of each of the 10 genes in the SSL-derived RNA of the subject.

### Pathological diagnosis

It has been recently reported that about 63% of a group of RNAs whose expression changes in cancer cells are mRNA encoding proteins (Cancer Res. 2016, 76, 216-226). Therefore, by measuring the expression state of mRNA, a change in physiological condition of cells due to a disease such as cancer can be more exactly detected, so that it is possible to more accurately diagnose a physical condition. SSL contains an abundance of mRNA, and contains mRNA of SOD2 reported to be related to cancer (Physiol genomics, 2003, 16, 29-37; Cancer Res, 2001, 61, 6082-6088). Therefore, SSL is useful as a biological sample for diagnosis or prognosis of cancers such as skin cancer.

In recent years, it has been reported that expression of molecules in the skin varies in patients with diseases in tissues other than the skin, such as obesity, Alzheimer's disease, breast cancer and cardiac disease, and therefore "the skin can be a window to body's health (Eur. J. Pharm. Sci. 2013, 50, 546-556). Thus, it may be possible to analyze a physiological condition at a part other than the skin or a general physiological condition in the subject by measuring the expression state of mRNA in SSL.

### non-coding RNA analysis

In recent years, the involvement of non-coding (nc) RNA such as miRNA and lincRNA in gene expression in cells has been given attention, and actively studied. Non-invasive or low-invasive methods for diagnosing cancer or the like using miRNA in the urine or serum have been heretofore developed (e.g. Proc. Natl. Acad. Sci. USA, 2008, 105, 10513-10518; Urol Oncol, 2010, 28, 655-661). ncRNA prepared from SSL, such as miRNA and LincRNA, can be used as a sample for the studies and diagnoses.

### Screening or detection of nucleic acid marker

A nucleic acid marker for a disease or a condition can be screened or detected by using as a sample the nucleic acid prepared from SSL. In the present description, the nucleic acid marker for a disease or a condition is a nucleic acid serving as an index for determination of a given disease or condition or determination of a risk thereof. Preferably, the nucleic acid marker is an RNA marker, and the RNA is preferably mRNA, miRNA or lincRNA. Examples of the disease or condition targeted by the nucleic acid marker include, but are not limited to, various skin diseases (e.g. atopic dermatitis); skin health conditions (sensitive skin, photoaging, inflammation (redness), dryness, moisture content or oil content, skin tenseness and dullness); and cancers such as skin cancer and diseases in tissues other than the skin, such as obesity, Alzheimer's disease, breast cancer and cardiac disease, as described in the section "Pathological diagnosis". Analysis of expression of a nucleic acid can be performed by known means such as analysis of RNA expression using real-time, PCR, microarrays or a next-generation sequencer.

An example is a method for selecting a nucleic acid marker for a disease or a condition. In the method, a population with a predetermined disease or condition or a risk thereof is taken as a subject, and a nucleic acid derived from a skin cell of the subject is prepared by the method for preparing a nucleic acid according to the present invention. The expression (e.g. expression level) of the nucleic acid prepared from the population is compared to the expression of a control. Examples of the control include a population without the predetermined disease or condition or a risk thereof, and associated data. A nucleic acid whose expression is different from that of the control can be selected as a marker for the predetermined disease or condition or a candidate thereof. Examples of the nucleic acid marker or candidate selected in this manner include marker genes described in Tables 7-1 to 7-24.

Another example is a method for detecting a nucleic acid marker for a disease or a condition, or a method for determining a disease or a condition on the basis of the detection of the marker, or determining a risk thereof. In the method, from a subject desiring or needing determination of a predetermined disease or condition or a risk thereof, a nucleic acid derived from a skin cell of the subject is prepared by the method for preparing a nucleic acid according to the present invention. Subsequently, a nucleic acid marker for the predetermined disease or condition is detected from the prepared nucleic acid. The disease or condition of the subject or a risk thereof is determined on the basis of existence or non-existence or the expression level of the nucleic acid marker.

Analysis of the nucleic acid prepared according to the present invention can be performed by a usual method used for analysis of nucleic acids, such as Real-time, PCR, RT-PCR, microarrays, sequencing and chromatography. The method for analyzing a nucleic acid according to the present invention is not limited thereto.

The present description discloses the following substances, production methods, uses, methods and the like as illustrative embodiments of the present invention. The present invention is not limited to these embodiments.
[1] A method for preparing a nucleic acid derived from a skin cell of a subject, the method containing preserving at 0°C or lower an RNA-containing skin surface lipid collected from the subject.
[2] The method according to [1], wherein the temperature for the preservation is preferably from -20 ± 20°C to -80 ± 20°C, more preferably from -20 ± 10°C to -80 ± 10°C, still more preferably from -20 ± 20°C to -40 ± 20°C, even more preferably from -20 ± 10°C to -40 ± 10°C, even more preferably -20 ± 10°C, even more preferably -20 ± 5°C.
[3] The method according to [1] or [2], wherein the period for the preservation is preferably 12 months or less, for example 6 hours or more and 12 months or less, more preferably 6 months or less, for example 1 day or more and 6 months or less, still more preferably 3 months or less, for example 3 days or more and 3 months or less.
[4] A method for preparing a nucleic acid derived from a skin cell of a subject, the method containing: converting RNA has been contained in a skin surface lipid of the subject into cDNA by reverse transcription, and then subjecting the cDNA to multiplex PCR; and purifying a reaction product of the PCR.
[5] The method according to [4], wherein a temperature for annealing and elongation reaction in the multiplex PCR is preferably 62°C ± 1°C, more preferably 62°C ± 0.5°C, still more preferably 62°C ± 0.25°C.
[6] The method according to [4] or [5], wherein preferably, the elongation reaction in the reverse transcription is carried out under the following conditions:
   42°C ± 1°C for 60 minutes or more;
   42°C ± 1°C for from 80 to 100 minutes;
   42°C ± 0.5°C for 60 minutes or more;
   42°C ± 0.5°C for from 80 to 100 minutes;
   42°C ± 0.25°C for 60 minutes or more; or
   42°C ± 0.25°C for from 80 to 100 minutes.
[7] The method according to any one of [4] to [6], wherein preferably, the purification of the reaction product of the PCR is purification by size separation.
[8] The method according to any one of [4] to [7], wherein the RNA has been contained in the skin surface lipid of the subject is prepared by separating the RNA from the skin surface lipid of the subject.
[9] The method according to any one of [4] to [8], wherein the skin surface lipid of the subject is one preserved at preferably 0°C or lower, more preferably from -20 ± 20°C to -80 ± 20°C, still more preferably from -20 ± 10°C to -80 ± 10°C, even more preferably from -20 ± 20°C to -40 ± 20°C, even more preferably from -20 ± 10°C to -40 ± 10°C, even more preferably -20 ± 10°C, even more preferably -20 ± 5°C.
[10] The method according to [9], wherein the skin surface lipid of the subject is one preserved for preferably 12 months or less, for example 60 hours or more and 12 months or less, more preferably 6 months or less, for example 1 day or more and 6 months or less, still more preferably 3 months or less, for example 3 days or more and 3 months or less.
[11] A method for analyzing a condition of a skin, a part other than the skin, or the whole body in the subject, the method containing analyzing a nucleic acid prepared by the method according to any one of [1] to [10].
[12] The method according to [11], wherein the analysis is preferably analysis of a disease or a condition of the skin, more preferably detection of a skin with redness, sensitive skin or atopic dermatitis or a skin without redness, sensitive skin or atopic dermatitis; detection of a skin with a small or large amount of sebum or skin moisture content; estimation or prediction of a skin condition, for example prediction of a skin physical property, estimation or prediction of visual or palpatory evaluation of the skin, or estimation or prediction of the sebum composition; or estimation or prediction of the cumulative ultraviolet exposure time of the skin.
[13] The method according to [11], wherein
   the analysis is detection of a skin with atopic dermatitis or a skin without atopic dermatitis, and the nucleic acid is at least one selected from the group consisting of the genes described in (B), (C) and (D) of Tables 7-1 to 7-11, more preferably all of the genes;
   the analysis is detection of a skin with mild or moderate atopic dermatitis or without atopic dermatitis, and the nucleic acid is at least one selected from the group consisting of the genes described in (C) and (D) of Tables 7-1 to 7-11, more preferably all of the genes;
   the analysis is detection of a skin with sensitive skin or a skin without sensitive skin, and the nucleic acid is at least one selected from the group consisting of the genes described in (E) of Tables 7-1 to 7-20, more preferably all of the genes;
   the analysis is detection of a skin with sensitive skin or a skin without sensitive skin, and the nucleic acid is at least one selected from the group consisting of the genes described in (F) of Tables 7-1 to 7-10, more preferably all of the genes;
   the analysis is detection of a skin with redness or a skin without redness, and the nucleic acid is at least one selected from the group consisting of the genes described in (G) of Tables 7-1 to 7-20, more preferably all of the genes;
   the analysis is detection of a skin with a large or small moisture content, and the nucleic acid is at least one selected from the group consisting of the genes described in (H) of Tables 7-1 to 7-16, more preferably all of the genes;
   the analysis is detection of a skin with a large or small amount of sebum, and the nucleic acid is at least one selected from the group consisting of the genes described in (I) of Tables 7-1 to 7-17, more preferably all of the genes; or
   the analysis is estimation or prediction of a skin physical property, estimation or prediction of visual or palpatory evaluation of the skin, or estimation or prediction of the sebum composition, and the nucleic acid is at least one selected from the group consisting of the genes described in Table 8, more preferably all of the genes.
[14] A method for evaluating an effect or efficacy of a skin external preparation, an intracutaneously administered preparation, a patch, an oral preparation or an injection on a subject, the method containing analyzing a nucleic acid prepared by the method according to any one of [1] to [10].
[15] The method according to [14], wherein the effect or efficacy of the skin external preparation, the intracutaneously administered preparation, the patch, the oral preparation or the injection on the subject is preferably an improving effect of a skincare product on a skin condition of the subject, and the nucleic acid is preferably at least one selected from the group consisting of BNIP3, CALML3, GAL, HSPA5, JUNB, KIF13B, KRT14, KRT17, KRT6A, OVOL1, PPIF, PRDM1, RBM3, RPLP1, RPS4X, SEPT9, SOAT1, SPNS2, UBB, VCP, WIPI2 and YPEL3, more preferably all of the genes.
[16] A method for analyzing a concentration of a component in the blood of a subject, the method containing analyzing a nucleic acid prepared by the method according to any one of [1] to [10].
[17] The method according to [16], wherein preferably, the component in the blood is a hormone, insulin, neutral fat, γ-GTP or L-cholesterol.
[18] The method according to [17], wherein the hormone is preferably testosterone, dihydrotestosterone, androstenedione, dehydroepiandrosterone, estrone, estradiol, progesterone or cortisol, more preferably testosterone or cortisol.
[19] The method according to [16], wherein the component in the blood is preferably testosterone, and the nucleic acid is preferably at least one selected from the group consisting of 10 RNAs derived from 10 genes consisting of SCARNA16, PRSS27, RDBP, PSMB10, SBNO1, EMC3, MAR9, C20orf112, C14orf2 and CCDC90B, more preferably the 10 RNAs.
[20] The method according to [16], wherein the component in the blood is preferably insulin, and the nucleic acid is preferably at least one selected from the group consisting of 10 RNAs derived from 10 genes consisting of EAPP, SDE2, LYAR, ZNF493, PSMB10, FAM71A, GPANK1, FGD4, MRPL43 and CMPK1, more preferably the 10 RNAs.
[21] The method according to [16], wherein the component in the blood is preferably neutral fat, and the nucleic acid is preferably at least one selected from the group consisting of 15 RNAs derived from 15 genes consisting of CCDC9, C6orf106, CERK, HSD3B2, SUN2, FNDC4, GRAMD1C, DGAT2, ALPL, HOMER3, MTHFS, ADIPOR1, RBM3, EXOC8 and ARHGEF37, more preferably the 15 RNAs.
[22] The method according to [16], wherein the component in the blood is preferably γ-GTP, and the nucleic acid is preferably at least one selected from the group consisting of 15 RNAs derived from 15 genes consisting of TMEM38A, BTN3A2, NAP1L2, ABCA2, ALPL, SECTM1, C17orf62, GNB2, R3HDM4, LRG1, SBNO2, CD14, MLLT1, NINJ2 and LIMD2, more preferably the RNAs.
[23] The method according to [16], wherein the component in the blood is preferably LDL-cholesterol, and the nucleic acid is preferably at least one selected from the group consisting of 10 RNAs derived from 10 genes consisting of THTPA, LOC100506023, ZNF700, TAB3, PLEKHA1, ZNF845, FXC1, CUL4A, NDUFV1 and AMZ2, more preferably the 10 RNAs.
[24] A method for analyzing a concentration of a component in the blood of a subject, the method containing:
   obtaining an expression level of RNA derived from a gene having a high correlation with the concentration of a component in the blood from the nucleic acid of a subject, which is prepared by the method according to any one of [1] to [10]; and
   analyzing the concentration of the component in the blood of the subject by a machine learning model on the basis of the expression level of RNA derived from the gene having a high correlation with the concentration of the component in the blood,
   the machine learning model being a machine learning model constructed so that the data of the expression level of RNA derived from the gene having a high correlation with the concentration of the component in the blood and has been contained in skin surface lipid-derived RNA obtained from a human population serves as an explanatory variable and the data of the concentration of the component in the blood obtained from the human population serves as an objective variable.
[25] The method according to [24], wherein preferably, the component in the blood is a hormone, insulin, neutral fat, γ-GTP or LDL-cholesterol, and the hormone is preferably testosterone or cortisol.
[26] The method according to [25], wherein
   the component in the blood is preferably testosterone, and the gene having a high correlation with the concentration of the component in the blood is preferably at least one selected from the group consisting of SCARNA16, PRSS27, RDBP, PSMB10, SBNO1, EMC3, MAR9, C20orf112, C14orf2 and CCDC90B, more preferably all of the genes;
   the component in the blood is preferably insulin, and the gene having a high correlation with the concentration of the component in the blood is preferably at least one selected from the group consisting of EAPP, SDE2, LYAR, ZNF493, PSMB10, FAM71A, GPANK1, FGD4, MRPL43 and CMPK1, more preferably all of the genes;
   the component in the blood is preferably neutral fat, and the gene having a high correlation with the concentration of the component in the blood is preferably at least one selected from the group consisting of CCDC9, C6orf106, CERK, HSD3B2, SUN2, FNDC4, GRAMD1C, DGAT2, ALPL, HOMER3, MTHFS, ADIPOR1, RBM3, EXOC8 and ARHGEF37, more preferably all of the genes;
   the component in the blood is preferably γ-GTP, and the gene having a high correlation with the concentration of the component in the blood is preferably at least one selected from the group consisting of TMEM38A, BTN3A2, NAP1L2, ABCA2, ALPL, SECTM1, C17orf62, GNB2, R3HDM4, LRG1, SBNO2, CD14, MLLT1, NINJ2 and LIMD2, more preferably all of the genes; or
   the component in the blood is preferably LDL-cholesterol, and the gene having a high correlation with the concentration of the component in the blood is preferably at least one selected from the group consisting of THTPA, LOC100506023, ZNF700, TAB3, PLEKHA1, ZNF845, FXC1, CUL4A, NDUFV1 and AMZ2, more preferably all of the genes.
[27] A database for constructing a machine learning model for analyzing a concentration of a component in the blood, the database containing:
   data of the expression level of RNA derived from a gene having a high correlation with the concentration of the component in the blood and has been contained in skin surface lipid-derived RNA obtained from a human population; and
   data of the concentration of the component in the blood obtained from the human population, wherein
   the component in the blood is preferably testosterone, and the gene having a high correlation with the concentration of the component in the blood is preferably at least one selected from the group consisting of SCARNA16, PRSS27, RDBP, PSMB10, SBNO1, EMC3, MAR9, C20orf112, C14orf2 and CCDC90B, more preferably all of the genes;
   the component in the blood is preferably insulin, and the gene having a high correlation with the concentration of the component in the blood is preferably at least one selected from the group consisting of EAPP, SDE2, LYAR, ZNF493, PSMB10, FAM71A, GPANK1, FGD4, MRPL43 and CMPK1, more preferably all of the genes;
   the component in the blood is preferably neutral fat, and the gene having a high correlation with the concentration of the component in the blood is preferably at least one selected from the group consisting of CCDC9, C6orf106, CERK, HSD3B2, SUN2, FNDC4, GRAMD1C, DGAT2, ALPL, HOMER3, MTHFS, ADIPOR1, RBM3, EXOC8 and ARHGEF37, more preferably all of the genes;
   the component in the blood is preferably γ-GTP, and the gene having a high correlation with the concentration of the component in the blood is preferably at least one selected from the group consisting of TMEM38A, BTN3A2, NAP1L2, ABCA2, ALPL, SECTM1, C17orf62, GNB2, R3HDM4, LRG1, SBNO2, CD14, MLLT1, NINJ2 and LIMD2, more preferably all of the genes; or
   the component in the blood is preferably LDL-cholesterol, and the gene having a high correlation with the concentration of the component in the blood is preferably at least one selected from the group consisting of THTPA, LOC100506023, ZNF700, TAB3, PLEKHA1, ZNF845, FXC1, CUL4A, NDUFV1 and AMZ2, more preferably all of the genes.
[28] A program for carrying out the method according to any one of [24] to [26].
[29] An apparatus for carrying out the method according to any one of [24] to [26].

### Examples

Hereinafter, the present invention will be described in more detail on the basis of Examples, which should not be construed as limiting the present invention.

### Test Example 1: Effect of preservation temperature on stability of RNA in SSL

### 1) Stability of RNA in SSL

Sebum was collected from the entire face of a healthy person using an oil blotting film (5 x 8 cm, made of polypropylene, 3M Ltd.). The oil blotting film was transferred into a glass vial, and left standing at 4°C for several hours, and RNA in SSL contained in the film was then purified. In the purification of RNA, the oil blotting film was cut to an appropriate size, and RNA was extracted in accordance with an attached protocol using QIAzol (registered trademark) Lysis Reagent (Qiagen). The extracted RNA was subjected to reverse transcription at 42°C for 30 minutes with SuperScript (registered trademark) VILO cDNA Synthesis kit (Life Technologies Japan Ltd.) to synthesize cDNA. As a primer for the reverse transcription reaction, a random primer attached to the kit was used. From the obtained cDNA, a library containing cDNA derived from the 20802 gene was prepared by multiplex PCR. The multiplex PCR was performed under the condition of [99°C, 2 min→(99°C, 15 sec→60°C, 16 min) x 20 cycles→4°C, Hold] using Ion AmpliSeqTranscriptome Human Gene Expression Kit (Life Technologies Japan Ltd.). The prepared library was measured using TapeStation (Agilent Technologies) and High Sensitivity D1000 ScreenTape (Agilent Technologies), and the results showed that a peak derived from the library was not detected. The reason why the peak was not detected was that the amount of sebum collected from the subject was small; and leaving the library standing at 4°C after the collection and before the purification had accelerated decomposition, so that the amount of RNA purified was small.

### 2) Effect of preservation temperature

For examining the effect of the preservation temperature on human RNA in SSL, the oil blotting film used for collecting the sebum in 1) was coated with 40 ng of a human surface skin cell-derived RNA solution as RNA, and then preserved for 4 days at (i) room temperature (RT), (ii) 4°C, (iii) -20°C or (iv) -80°C. As the human surface skin cell-derived RNA solution, one obtained by dissolving RNA extracted from frozen NHEK (NB) (KURABO INDUSTRIES LTD.) in a 50% (v/v) ethanol solution was used. The oil blotting film after the preservation was cut to an appropriate size, and RNA was extracted in accordance with an attached protocol using QIAzol (registered trademark) Lysis Reagent (Qiagen). The extracted RNA was measured with TapeStation (Agilent Technologies) and High Sensitivity RNA Screen Tape (Agilent Technologies).

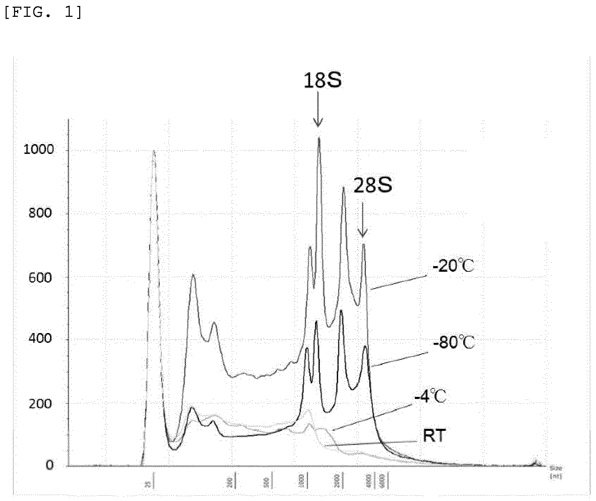

Figure 1 shows the results of the measurement. Among RNAs in SSL preserved at room temperature or 4°C, human-derived RNAs (28S and 18S ribosomal RNAs) showed markedly low peaks, and the peaks for other RNAs were not substantially detected. On the other hand, for RNAs in SSL preserved at -20°C or -80°C, the peaks for 28S and 18S ribosomal RNAs were detected, and therefore it was shown that the RNAs had been stably preserved. Further, since the peak areas of 28S and 18S ribosomal RNAs were larger in preservation at -20°C than in preservation at - 80°C, it was thought that for preservation of RNA in SSL, preservation at -20°C was more suitable than preservation at -80°C, a temperature heretofore commonly employed for preservation of RNA.

### Test Example 2: Preparation of nucleic acid from SSL-derived RNA by multiplex PCR

### 1) Optimization of reverse transcription reaction conditions

Using an oil blotting film (3M Ltd.), sebum was collected from the entire face of a healthy person with a small amount of sebum. From the oil blotting film, RNA was extracted in accordance with the same procedure as in Test Example 1. The extracted RNA was subjected to reverse transcription to synthesize cDNA. The reverse transcription reaction was carried out using SuperScript (registered trademark) VILO cDNA Synthesis kit (Thermo Scientific). As a primer for the reverse transcription reaction, a random primer attached to the kit was used. The condition of the elongation temperature and the time for the reverse transcription was set to (i) 40°C for 60 minutes, (ii) 40°C for 90 minutes, (iii) 42°C for 60 minutes or (iv) 42°C for 90 minutes (temperature accuracy: ±0.25°C). Using the obtained cDNA, multiplex PCR was performed under the same conditions as in Test Example 1. The obtained PCR product was purified with Ampure XP (Beckman Coulter Inc.). The concentration of a PCR product in the solution of the obtained purified product was determined with TapeStation (Agilent Technologies) and High Sensitivity D1000 Screen Tape (Agilent Technologies). Table 1 shows the results. The PCR product was obtained in the largest amount when reverse transcription was performed at 42°C for 90 minutes.

**[Table 1]**

| **Temperature (°C)** | **Time (min)** | **PCR product concentration (pM)** |
|---|---|---|
| **40** | **60** | **3680** |
| **40** | **90** | **4070** |
| **42** | **60** | **3750** |
| **42** | **90** | **8040** |

### 2) Optimization of PCR conditions

Using an oil blotting film (3M Ltd.), sebum was collected from the entire face of a healthy person with a small amount of sebum. From the oil blotting film, RNA was extracted in accordance with the same procedure as in Test Example 1. Using the extracted RNA, synthesis of cDNA and multiplex PCR were performed in the same manner as in 1) except that the temperature for annealing and elongation in PCR was changed. The condition for reverse transcription was set to 42°C for 90 minutes. The temperature for annealing and elongation was set to (i) 60°C, (ii) 62°C, (iii) 63°C or (iv) 64°C (temperature accuracy: ±0.25°C). The obtained PCR product was purified with Ampure XP (Beckman Coulter Inc.), and determined with TapeStation (Agilent Technologies) and High Sensitivity D1000 Screen Tape (Agilent Technologies). Table 2 shows the results. The PCR product was obtained in the largest amount when the temperature for annealing and elongation was 62°C.

**[Table 2]**

| **Annealing and elongation temperature (°C)** | **PCR product concentration (pM)** |
|---|---|
| **60** | **Undetectable** |
| **62** | **139** |
| **63** | **Undetectable** |
| **64** | **Undetectable** |

### 3) Purification of PCR product

Using an oil blotting film (3M Ltd.), sebum was collected from the entire face of a healthy person with a small amount of sebum. From the oil blotting film, RNA was extracted in accordance with the same procedure as in Test Example 1. Using the extracted RNA, synthesis of cDNA and multiplex PCR were performed in the same manner as in 1). The condition for reverse transcription was set to 42°C for 30 minutes. The temperature for annealing and elongation was set to (i) 60°C (temperature accuracy: ±0.25°C). The obtained PCR product was divided into two parts. One part was purified with Ampure XP (Beckman Coulter Inc.), and the other part was not purified. Each sample solution, 5XVILO RT Reaction Mix attached to SuperScript (registered trademark) VILO cDNA Synthesis kit, 5XIon Ampliseq HiFi Mix attached to Ion AmpliSeqTranscriptome Human Gene Expression Kit (Life Technologies Japan Ltd.), and Ion AmpliSeq Transcriptome Human Gene Expression Core Panel were mixed to reconstruct the buffer composition, and in accordance with protocols attached to kits, digestion of the primer sequence, adaptor ligation and purification, and amplification were performed to prepare a library. The concentration of the obtained library was determined with TapeStation (Agilent Technologies) and High Sensitivity D1000 Screen Tape (Agilent Technologies). Table 3 shows the results. In samples which were not purified, the library was not detected.

**[Table 3]**

| **Purification** | **Library concentration (pM)** |
|---|---|
| **No** | **Undetectable** |
| **Yes** | **71.8** |

The results in 1) and 2) showed that when a nucleic acid sample was prepared from RNA in SSL, the optimum condition for reverse transcription reaction was approximately 42°C for 90 minutes, and the optimum condition of the annealing and elongation temperature for multiplex PCR was approximately 62°C. It was considered that by performing multiplex RT-PCR under these conditions, the yield of the nucleic acid sample from RNA in SSL was increased. The results in 3) showed that addition of a purification step after PCR increased the yield of the nucleic acid sample, so that it was possible to prepare of a nucleic acid sample even from SSL with a small RNA amount. Further, it was considered that as shown in Test Example 1, when RNA in SSL collected from the subject was preserved at -20°C until being used for preparation of the nucleic acid sample, RNA was inhibited from denaturing, so that it was possible to further increase the yield of the nucleic acid sample.

The reverse transcriptase and the primer used during the reverse transcription reaction are SuperScript (registered trademark) III Reverse Transcriptase and random Primers, respectively, and the enzyme and the primer used at the time of performing PCR are AmpliSeq HiFi Mix Plus and AmpliSeq Transcriptome Panel Human Gene Expression CORE, respectively.

### Test Example 3: Detection of atopic dermatitis using SSL-derived RNA

### Collection of SSL

20 healthy persons (20 to 39-year-old males, BMI: 18.5 or more and less than 25.0) and 11 atopic dermatitis patients (ADs) (20 to 39-year-old males, BMI: 18.5 or more and less than 25.0) were selected as subjects. The healthy persons were confirmed to have no abnormality of the skin by a dermatologist in advance, and ADs were diagnosed as atopic dermatitis by a dermatologist in advance. Sebum was collected from the entire face of each subject using an oil blotting film (3M Ltd.) after the entire face was photographed. The oil blotting film was transferred into a glass vial, and preserved at -80°C for about 1 month until being used for extraction of RNA. In the following test examples as well as this test example, SSL collected from the subject was preserved at -80°C, i.e. a common preservation condition, until being used for extraction of RNA. If the SSL is preserved under a condition enabling more stable preservation of RNA in SSL (at -20°C) as shown in Test Example 1, at least comparable analysis results may be obtained because RNA expression analysis data can be more stably obtained.

### Preparation of RNA and sequence analysis

From the preserved oil blotting film, RNA was extracted in accordance with the same procedure as in Test Example 1. The extracted RNA was subjected to reverse transcription at 42°C for 90 minutes, and multiplex PCR was performed at an annealing and elongation temperature of 62°C. The obtained PCR product was purified with Ampure XP (Beckman Coulter Inc.), followed by performing reconstruction of the buffer, digestion of the primer sequence, adaptor ligation and purification, and amplification in accordance with the same procedure as in Test Example 2 and 3) to prepare a library. The prepared library was loaded into Ion 540 Chip, and subjected to sequencing using Ion S5/XL System (Life Technologies Japan Ltd.).

### RNA expression analysis

The expression levels of SSL-derived RNA species confirmed to be expressed through the sequencing were compared between the healthy persons and the ADs. As RNA species to be compared, 19 immune response-related RNAs and 17 keratinization-related RNAs were used. It is reported in a document (J Allergy Clin Immunol, 2011, 127: 954-964) that for these RNA species, the ratio of the expression level in AD to the expression level in the healthy person varies between an affected part and a non-affected part of the skin tissue of AD.

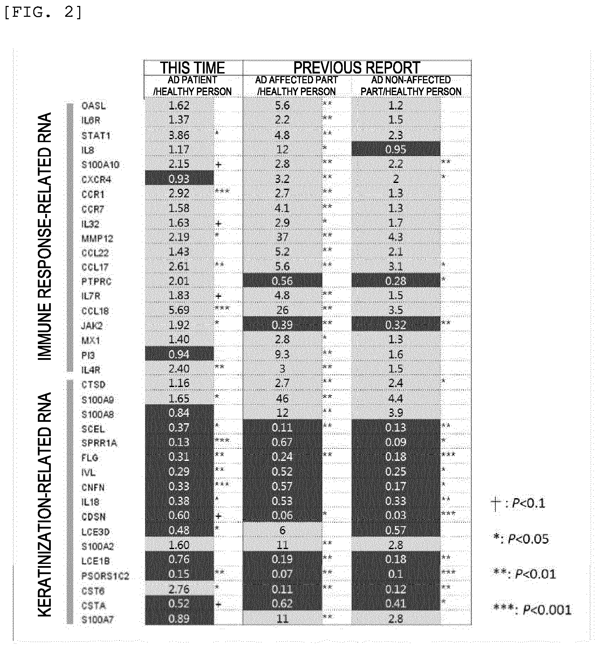

Figure 2 shows the results. The values in the figure represent ratios of the expression level in AD to that in the healthy person as measured in this test example and ratios of the expression level in each of the affected part and the non-affected part of AD to the expression level in the healthy person as measured in the document, for the RNAs. In the figure, RNA whose expression increased in AD is indicated in light gray, and RNA whose expression decreased in AD is indicated in dark gray. As a significance test, the Student's t-test was conducted. For many of the immune response-related RNAs in SSL-derived RNAs, the expression level was higher in AD than in the healthy person as in the report in the document. On the other hand, for many of the keratinization-related RNAs in SSL-derived RNAs, the expression level was lower in AD than in the healthy person as in the report in the document. These results show that SSL-derived RNA contains an atopic skin dermatitis-related marker indicating an enhanced inflammation condition, a decreased barrier function or the like and that an atopic dermatitis patient can be discriminated on the basis of expression of the marker in these SSL-derived RNAs.

### Test Example 4: Prediction of concentration of component in blood using SSL-derived RNA

### Subjects

38 healthy males (20 to 50-year-old, BMI: 18.5 or more and less than 25.0) confirmed to have no abnormality of the skin by a dermatologist in advance were selected as subjects.

### Collection of blood and determination of concentration of component in blood

3 mL of blood was collected from the arm of each subject using a vacuum blood collection tube, and serum was separated and preserved at -80°C. From the preserved serum, the serum testosterone concentration was determined in accordance with an attached protocol using Testosterone ELISA Kit (Cayman Chemical). An external inspection organization (LSI Medience Corporation) was commissioned to determine the serum concentrations of insulin, neutral fat, γ-GTP and LDL-cholesterol.

### Preparation of SSL-derived RNA and sequence analysis

Sebum was collected from the entire face of each subject using an oil blotting film (3M Ltd.) after the entire face was photographed. The oil blotting film was transferred into a glass vial, and preserved at -80°C for about 1 month. From the preserved oil blotting film, RNA in SSL was extracted in accordance with the same procedure as in Test Example 3, a library was prepared, RNA species were identified through sequencing, and the expression levels of the RNA species were measured.

### Prediction of blood testosterone concentration using SSL-derived RNA

Data of the measured expression levels of SSL-derived RNAs from the subjects (reads per million mapped reads: RPM values) was randomly divided into data for 33 subjects and data for 5 subjects. On the basis of the SSL-derived RNA expression levels (RPM values) and the serum testosterone concentrations for a total of 33 subjects, a serum testosterone concentration prediction model based on machine learning was constructed. First, 10 RNAs having the highest correlation with the serum testosterone concentration (RNAs derived from SCARNA16, PRSS27, RDBP, PSMB10, SBNO1, EMC3, MAR9, C20orf112, C14orf2 and CCDC90B) were selected on the basis of the RPM values.

As learning data, the expression levels (RPM values) of SSL-derived RNA for the selected 10 RNAs for the 33 subjects were used as explanatory variables, and the serum testosterone concentrations for the 33 subjects were used as objective variables to perform construction and selection of an optimum prediction model with Visual Mining Studio Software (NTT DATA Mathematical System Inc.).

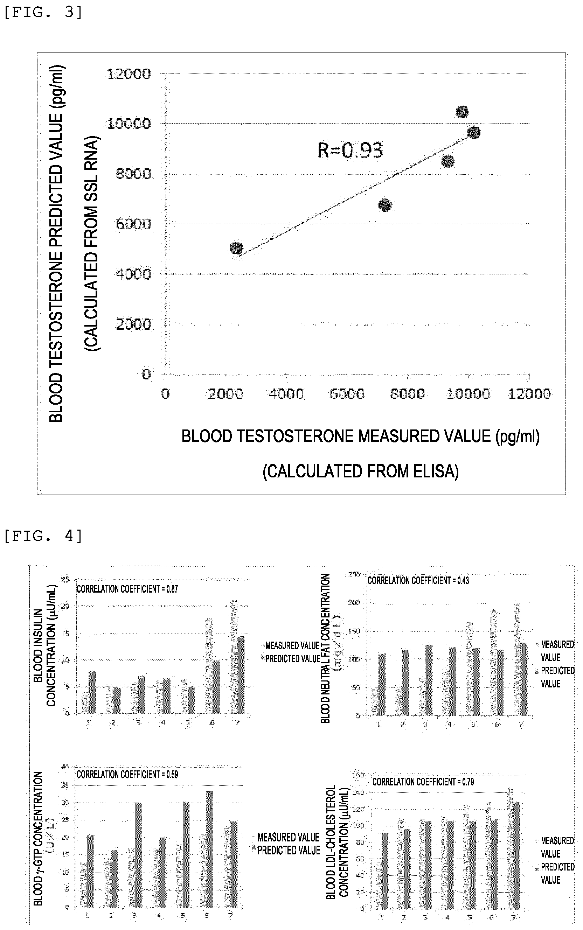

Using the selected prediction model, predicted values of blood testosterone concentrations were calculated from the SSL-derived RNA expression levels for the other 5 subjects. The results showed that the calculated predicted values had a high correlation with the measured values of serum testosterone concentrations (correlation coefficient = 0.93) as shown in Figure 3. This revealed that the SSL-derived RNA had important information for predicting the blood testosterone concentration.

### Prediction of concentrations of insulin, neutral fat, γ-GTP and LDL-cholesterol in blood using SSL-derived RNA

Data of the measured expression levels of SSL-derived RNAs from the subjects (RPM values) was randomly divided into data for 31 subjects and data for 7 subjects. On the basis of the SSL-derived RNA expression levels (RPM values) and the serum concentrations of insulin, neutral fat, γ-GTP and LDL-cholesterol for a total of 31 subjects, prediction models for the serum concentrations of insulin, neutral fat, γ-GTP and LDL-cholesterol, which are based on machine learning, were constructed. First, on the basis of the RPM values, RNAs derived from the following molecules were selected as RNAs having the highest correlation with the serum concentrations of 1) insulin, 2) neutral fat, 3) γ-GTP and 4) LDL-cholesterol:
1) insulin: EAPP, SDE2, LYAR, ZNF493, PSMB10, FAM71A, GPANK1, FGD4, MRPL43 and CMPK1;
2) neutral fat: CCDC9, C6orf106, CERK, HSD3B2, SUN2, FNDC4, GRAMD1C, DGAT2, ALPL, HOMER3, MTHFS, ADIPOR1, RBM3, EXOC8 and ARHGEF37;
3) γ-GTP: TMEM38A, BTN3A2, NAP1L2, ABCA2, ALPL, SECTM1, C17orf62, GNB2, R3HDM4, LRG1, SBNO2, CD14, MLLT1, NINJ2 and LIMD2; and
4) LDL-cholesterol: THTPA, LOC100506023, ZNF700, TAB3, PLEKHA1, ZNF845, FXC1, CUL4A, NDUFV1 and AMZ2.

As learning data, the expression level (RPM value) of SSL-derived RNA for each of the selected RNAs for the 31 subjects was used as an explanatory variable, and the concentration of insulin, neutral fat, γ-GTP or LDL-cholesterol for the 31 subjects was used as an objective variable to perform construction and selection of an optimum prediction model with Visual Mining Studio Software (NTT DATA Mathematical System Inc.).

Using the selected prediction model, predicted values of concentrations of insulin, neutral fat, γ-GTP and LDL-cholesterol in the blood were calculated from the SSL-derived RNA expression levels for the other 7 subjects. The results showed that the calculated predicted values had a positive correlation with the measured values of concentrations of insulin, neutral fat, γ-GTP and LDL-cholesterol in the serum, as shown in Figure 4. This revealed that SSL-derived RNA was a useful index for predicting the concentrations of insulin, neutral fat, γ-GTP and LDL-cholesterol in the blood.

### Test Example 5: Evaluation of skin external preparation (facial cleanser)

### (1) Identification of RNA species used for prediction of effect of facial cleanser on skin

### Subject and collection of SSL

9 healthy males (20 to 39-year-old) were selected as subjects. As a test product, a facial cleanser having an effect of decomposing and removing horn plugs (Biore Ouchi de Esute, Kao corporation) was used. The subjects each washed the entire face twice a day (morning and night) for 1 week using an appropriate amount (about 1 g) of the test product. Before the start of use of the facial cleanser as the test product (day 0) and 2 days after the start of use of the facial cleanser, SSL was collected from the entire face of the subject using an oil blotting film (3M Ltd.).

### Preparation of SSL-derived RNA and sequence analysis

The oil blotting film containing the collected SSL was transferred into a glass vial, and preserved at -80°C for about 1 month. From the preserved oil blotting film, RNA in SSL was extracted in accordance with the same procedure as in Test Example 3, a library was prepared, RNA species were identified through sequencing, and the expression levels of the RNA species were measured.

### Data analysis

As RNAs in which with respect to the measured SSL-derived RNA expression levels (RPM values) before the start of use of the cleanser and 2 days after the start of use of the cleanser, the p value in the Student's t-test 2 days after the start of use of the cleanser was 0.05 times or less of that on day 0 and the RPM value 2 days after the start of use of the cleanser was 2 times or more of that on day 0, RNAs derived from 22 molecules consisting of BNIP3, CALML3, GAL, HSPA5, JUNB, KIF13B, KRT14, KRT17, KRT6A, OVOL1, PPIF, PRDM1, RBM3, RPLP1, RPS4X, SEPT9, SOAT1, SPNS2, UBB, VCP, WIPI2 and YPEL3 were identified (Table 4). These molecules included molecules related to terminal keratinization of the skin, such as BNIP3, OVOL1, KRT14 and KRT17, and molecules related to anti-inflammation action, such as JUNB and PRDM1. It was suggested that these molecules could serve as markers indicating an improvement in skin condition because use of the cleanser increased the expression levels of the molecules.

**[Table 4]**

| (Group of RNAs whose expression is increased by use of cleanser) | | | | |
|---|---|---|---|---|
| **RNA name** | **0day (RPM)** | **2day (RPM)** | **P value** | **Fold change (2days/0day)** |
| BNIP3 | 168.2 | 379.7 | 0.005 | 2.3 |
| CALML3 | 368.2 | 931.6 | 0.048 | 2.5 |
| GAL | 353.9 | 734.9 | 0.028 | 2.1 |
| HSPA5 | 314.0 | 702.9 | 0.046 | 2.2 |
| JUNB | 1472.3 | 3156.7 | 0.012 | 2.1 |
| KIF13B | 47.1 | 186.5 | 0.047 | 4.0 |
| KRT14 | 1027.6 | 2921.0 | 0.046 | 2.8 |
| KRT17 | 3504.4 | 8752.1 | 0.011 | 2.5 |
| KRT6A | 1500.8 | 3355.9 | 0.027 | 2.2 |
| OVOL1 | 103.5 | 214.1 | 0.042 | 2.1 |
| PPIF | 1239.1 | 2528.6 | 0.008 | 2.0 |
| PRDM1 | 223.3 | 471.3 | 0.028 | 2.1 |
| RBM3 | 715.0 | 1813.7 | 0.001 | 2.5 |
| RPLP1 | 352.5 | 998.5 | 0.039 | 2.8 |
| RPS4X | 748.8 | 1501.9 | 0.010 | 2.0 |
| SEPT9 | 94.1 | 257.5 | 0.038 | 2.7 |
| SOAT1 | 209.6 | 509.3 | 0.030 | 2.4 |
| SPNS2 | 301.3 | 712.4 | 0.016 | 2.4 |
| UBB | 284.8 | 833.7 | 0.006 | 2.9 |
| VCP | 275.7 | 826.1 | 0.031 | 3.0 |
| WIPI2 | 750.2 | 1674.2 | 0.027 | 2.2 |
| YPEL3 | 168.3 | 362.1 | 0.019 | 2.2 |

### (2) Prediction of effect of facial cleanser Subject, skin condition data and collection of SSL

18 healthy males (20 to 48-year-old) were selected as subjects. As a test product, a facial cleanser having an effect of decomposing and removing horn plugs (Biore Ouchi de Esute, Kao corporation) was used. The subjects each washed the entire face twice a day (morning and night) for 1 week using an appropriate amount (about 1 g) of the test product as in "(1) Identification of RNA species used for prediction of effect of facial cleanser on skin" above. Before the start of use of the facial cleanser as the test product (day 0) and 1 week after the start of use of the facial cleanser, SSL was collected from the subject and a skin condition was measured as described below.
i) SSL was collected from the entire face using an oil blotting film (3M Ltd.).
ii) The face was washed, and then conditioned in a constant-temperature room (20 ± 5°C, 40%RH) for 15 minutes.
iii) A magnified image of the cheek was taken, and the horn cell layer moisture content of the left part of the cheek was then measured at one point using Corneometer (MPA580, Courage + Khazaka Electronic GmbH, Germany).
iv) Questionnaire studies on the skin condition were conducted.

### Preparation of SSL-derived RNA and sequence analysis

The oil blotting film containing the collected SSL was transferred into a glass vial, and preserved at -80°C for about 1 month. From the preserved oil blotting film, RNA in SSL was extracted in accordance with the procedure as in Test Example 3, a library was prepared, RNA species were identified by sequencing, and the expression levels of the RNA species were measured.

### Data analysis

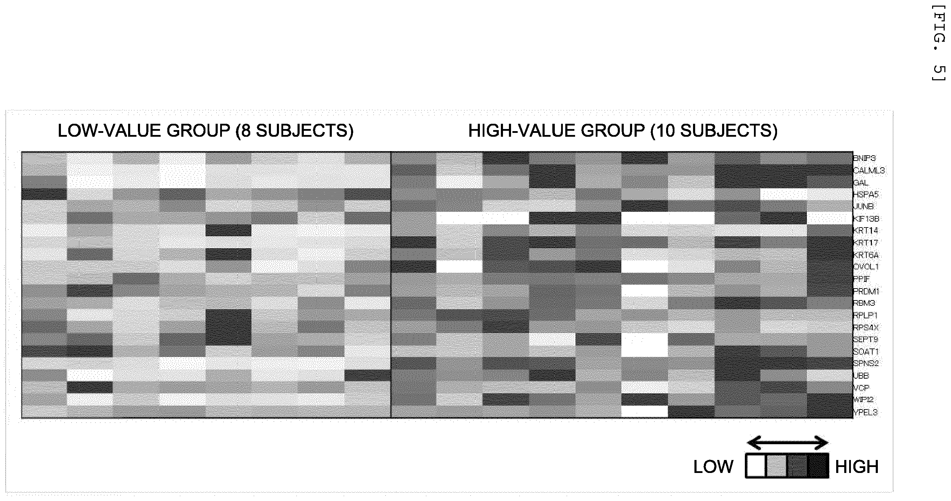

On the basis of the RPM values on day 0 for the group of 22 RNAs (Table 4) selected in "(1) Identification of RNA species used for prediction of effect of facial cleanser on skin" above, the subjects were classified into two groups: a group in which expression of the 22 RNAs tended to be generally high (high-value group); and a group in which expression of the 22 RNAs tended to be generally low (low-value group) (Figure 5). For the grouping, the self-organizing map of Gene Spring (Tomy Digital Biology Co., Ltd.) software was used.

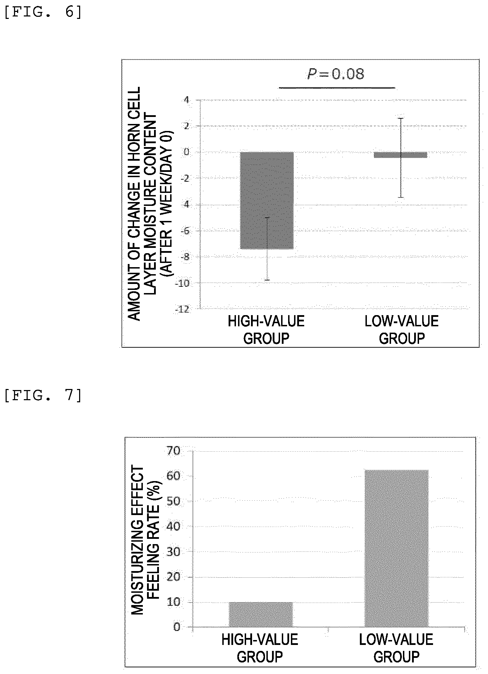

Francesco Iorio et al. reported "Signature reversion" as a method for improving a disease, containing applying a drug etc. having an effect of increasing expression of a group of RNAs whose expression decreases due to a disease or the like (Drug Discov Today, 2013, 18 (7-8): 350-357). This method was thought to ensure that in the low-value group, expression of the group of 22 RNAs would be increased more markedly by use of the test product than that in the high-value group, leading to improvement of a skin condition. In practice, Figure 6 shows the results of comparing the amounts of change in horn cell layer moisture content 1 week after use of the test product (value 1 week after use - value on day 0). On the test day 1 week after the start of use of the test product, the amount of change in horn cell layer moisture content assumed a negative value due to a considerable decrease in atmospheric temperature, so that the horn cell layer moisture content tended to be lower than the horn cell layer moisture content on the test day 0 day after the start of use of this test product. However, in the low-value group, the decrease in horn cell layer moisture content was smaller than the decrease in horn cell layer moisture content in the high-value group, so that the decrease in moisture content tended to be suppressed. Further, in the questionnaire studies on the feeling of usefulness, the population of persons feeling an improvement in skin moisturizing condition was higher in the low-value group than in the high-value group (Figure 7).

These results suggest that use of a SSL-derived RNA analysis technique enables prediction of the effect of a skin external preparation before the start of use of the product. For example, when SSL-derived RNAs (e.g. 22 RNAs found in this example) which are expressed or are not expressed characteristically in persons who can easily enjoy the effect of a certain skin external preparation, and the expression levels of the SSL-derived RNAs in a subject are then examined, it is possible to predict whether or not the effect can be obtained when the subject uses the skin external preparation.

### Test Example 6: Detection of novel atopic skin dermatitis marker molecules using SSL-derived RNA

### Subjects

55 healthy persons (20 to 49-year-old males, BMI: 18.5 or more and less than 25.0), 15 mild atopic skin dermatitis patients (20 to 39-year-old males, BMI: 18.5 or more and less than 25.0) and 25 moderate atopic skin dermatitis patients (20 to 39-year-old males, BMI: 18.5 or more and less than 25.0) were selected as subjects. The healthy persons were confirmed to have no abnormality of the skin by a dermatologist in advance, and the atopic dermatitis patients were diagnosed as atopic dermatitis by a dermatologist in advance.

### Preparation of SSL-derived RNA and sequence analysis

Sebum was collected from the entire face of each subject using an oil blotting film (3M Ltd.). The oil blotting film was transferred into a glass vial, and preserved at -80°C for about 1 month until being used for extraction of RNA. From the preserved oil blotting film, RNA in SSL was extracted in accordance with the same procedure as in Test Example 3, a library was prepared, RNA species were identified through sequencing, and the expression levels of the RNA species were measured.

### Data analysis

On the basis of SSL-derived RNA information (RPM value), a RPM value converted into a base-2 logarithmic value was subjected to data analysis. A group of 1911 genes were extracted in which the RPM value converted into the base-2 logarithmic value in the atopic dermatitis patients was half or less of that in the healthy persons and the p value in the Student's t-test in the atopic dermatitis patients was 0.05 times or less of that in the healthy persons ((A) of Tables 7-1 to 7-24). Subsequently, on the basis of a value obtained by converting the RPM value into a base-2 logarithmic value, and with the false discovery rate (FDR) level set to 5%, searching of biological processes (BPs) by gene ontology (GO) enrichment analysis was performed in accordance with a published document (Nature Protoc, 2009, 4: 44-57; Nucleic Acids res, 2009, 37: 1-13). As a result, 19 BPs related to a group of genes whose expression decreased in the atopic dermatitis patients. Of these, GO: 0050911 (detection of chemical stimulus involved in sensory perception of smell) was shown to be most closely related (Table 5). RNAs forming GO: 0050911 included about 400 olfactory receptors (ORs), and expression of 370 ORs was statistically significantly lower in the atopic dermatitis patients than in the healthy persons. Such a decrease in expression contributed to the significance of GO. This suggested that the expression levels of the 370 ORs in the SSL-derived RNA information could serve as a useful marker for discriminating healthy persons from atopic dermatitis patients. Further, the results of comparing the healthy persons with mild atopic dermatitis patients for RNA expression of OR and comparing the mild atopic dermatitis patients with the moderate atopic dermatitis patients showed that the expression of 368 ORs was lower in the mild atopic dermatitis patients than in the healthy persons, and expression of 284 ORs was lower in the moderate dermatitis patients than in the healthy persons ((B) to (D) of Tables 7-1 to 7-11) . These results revealed that the expression levels of the ORs shown in (B) to (D) of Tables 7-1 to 7-11 in SSL decreased as the symptom of atopic dermatitis worsened, and it was suggested that the severity of atopic dermatitis could be known by using the expression levels of the ORs as an index.

**[Table 5]**

| **(Biological processes difference between healthy persons and atopic der matitis patients)** | | |
|---|---|---|
| Accession | Name | FDR |
| GO:0050911 | detection of chemical stimulus involved in sensory perception of smell | 5E-229 |
| GO:0007186 | G-protein coupled receptor signaling pathway | 3E-164 |
| GO:0007608 | sensory perception of smell | 9E-53 |
| GO:0050907 | detection of chemical stimulus involved in sensory perception | 4E-43 |
| GO:0002323 | natural killer cell activation involved in immune response | 5E-09 |
| GO:0033141 | positive regulation of peptidyl-serine phosphorylation of STAT protein | 5E-09 |
| GO:0006334 | nucleosome assembly | 9E-09 |
| GO:0002286 | T cell activation involved in immune response | 1E-07 |
| GO:0001580 | detection of chemical stimulus involved in sensory perception of bitter taste | 5E-07 |
| GO:0042100 | B cell proliferation | 8E-05 |
| GO:0043330 | response to exogenous dsRNA | 0.0002 |
| GO:0018149 | peptide cross-linking | 0.0003 |
| GO:0060338 | regulation of type I interferon-mediated signaling pathway | 0.0004 |
| GO:0031424 | keratinization | 0.0008 |
| GO:0016339 | calcium-dependent cell-cell adhesion via plasma membrane cell adhesion molecules | 0.0083 |
| GO:0030216 | keratinocyte differentiation | 0.0096 |
| GO:0071880 | adenylate cyclase-activating adrenergic receptor signaling pathway | 0.0098 |
| GO:0050909 | sensory perception of taste | 0.0124 |
| GO:0007192 | adenylate cyclase-activating serotonin receptor signaling pathway | 0.0396 |

### Test Example 7: Detection of novel sensitive skin marker molecules using SSL-derived RNA

### Subjects

42 healthy females confirmed to have no abnormality of the skin by a dermatologist in advance (20 to 59-year-old, BMI: 18.5 or more and less than 25.0) were selected as subject candidates. For these candidates, questionary studies were conducted on whether or not there are subjective symptoms of sensitive skin (one of the four feelings: "bothered", "not so bothered", "not bothered" and "not bothered at all"). 10 candidates showing the feeling of "not bothered" or "not bothered at all" were classified as a group without subjective symptoms of sensitive skin, and 13 candidates showing the feeling of "bothered" was classified as a group with subjective symptoms of sensitive skin. These candidates were selected as subjects.

### Preparation of SSL-derived RNA and sequence analysis

Sebum was collected from the entire face of each subject using an oil blotting film (3M Ltd.). The oil blotting film was transferred into a glass vial, and preserved at -80°C for about 1 month until being used for extraction of RNA. From the preserved oil blotting film, RNA in SSL was extracted in accordance with the same procedure as in Test Example 3, a library was prepared, RNA species were identified through sequencing, and the expression levels of the RNA species were measured.

### Data analysis

On the basis of SSL-derived RNA information (RPM value), a RPM value converted into a base-2 logarithmic value (Log₂ RPM value) was subjected to data analysis. A group of 693 genes were extracted in which the Log₂RPM value in the group with subjective symptoms of sensitive skin was half or less of that in the group without subjective symptoms of sensitive skin and the p value in the Student's t-test in the group with subjective symptoms was 0.05 or less of that in the group without subjective symptoms ((E) of Tables 7-1 to 7-20). Subsequently, on the basis of the Log₂RPM value, and with the FDR level set to 5%, searching of BPs by gene ontology enrichment analysis was performed in accordance with the above-described published document. As a result, 4 BPs related to the group of genes whose expression decreased in the group with subjective symptoms of the sensitive skin were obtained, and it was shown that GO: 0050911 was most closely related (Table 6). Expression of 344 ORs ((F) of Tables 7-1 to 7-10), among about 400 PRs in GO: 0050911, was statistically significantly lower in the persons with subjective symptoms of sensitive skin than in the persons without subjective symptoms of sensitive skin. Such a decrease in expression contributed to the significance of GO. This suggested that the expression levels of these ORs in the SSL-derived RNA information could serve as a useful marker for detecting a subjective symptom of sensitive skin.

**[Table 6]**

| **(Biological processes difference between persons with and without subje ctive symptoms of sensitive skin)** | | |
|---|---|---|
| Accession | Name | FDR |
| GO:0050911 | detection of chemical stimulus involved in sensory perception of smell | 2E-28 |
| GO:0007186 | G-protein coupled receptor signaling pathway | 2E-17 |
| GO:0050907 | detection of chemical stimulus involved in sensory perception | 2E-06 |
| GO:0007608 | sensory perception of smell | 1E-05 |

### Test Example 8: Detection of sebum secretion, moisture content and redness-related marker molecules using SSL-derived RNA

### Subjects

38 healthy males (20 to 59-year-old, BMI: 18.5 or more and less than 25) confirmed to have no abnormality of the skin by a dermatologist in advance were selected as subjects.

### Measurement of skin physical properties

The entire face was photographed, and the casual amount of sebum in the forehead of each subject before washing of the face was then measured using Sebumeter (MPA580, Courage + Khazaka Electronic GmbH, Germany). Thereafter, the face was washed, and conditioned for 15 minutes in a variable-environment room (temperature: 20°C (±2°C) and humidity: 50% (±5%)). After completion of the conditioning, the moisture content of the cheek was measured using Corneometer (MPA580, Courage + Khazaka Electronic GmbH, Germany).

### Preparation of SSL-derived RNA and sequence analysis

After the casual amount of sebum was measured in the measurement of the skin physical properties, sebum was collected from the entire surface of each subject using an oil blotting film (3M Ltd.). The oil blotting film was transferred into a glass vial, and preserved at -80°C for about 1 month. From the preserved oil blotting film, RNA in SSL was extracted in accordance with the same procedure as in Test Example 3, a library was prepared, RNA species were identified through sequencing, and the expression levels of the RNA species were measured.

### Data analysis

### 1) Sebum secretion-related molecules

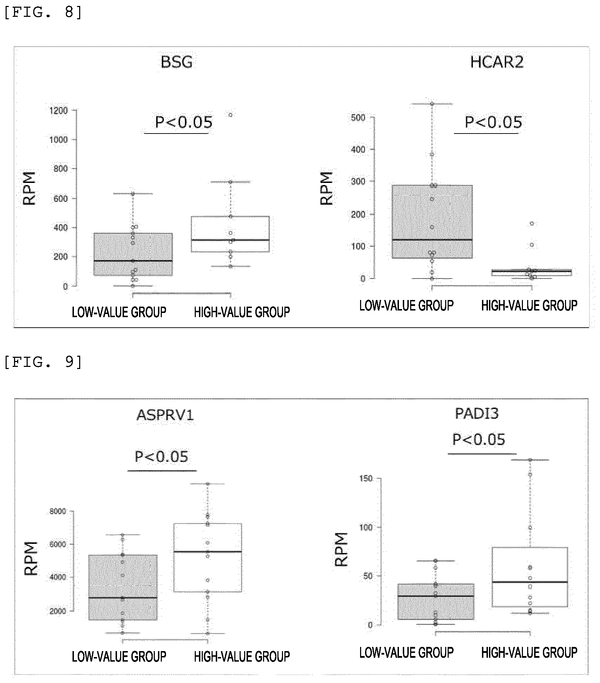

Persons in which the value of the forehead casual amount of sebum measured with Sebumeter was less than 100 were classified as a low-value group, and persons in which the value of the forehead casual amount of sebum was 150 or more were classified as a high-value group. Comparison of the expression level of SSL-derived RNA (RPM value) between the two groups (low-value group and high-value group) was performed in accordance with the same procedure as in Test Example 7, and the result showed that 594 RNAs statistically significantly varied in expression ((I) of Tables 7-1 to 7-17). As shown in Figure 8, it was evident that the expression level of Basigin (BSG) was statistically significantly higher in the sebum secretion high-value group than in the low-value group (Student's t-test, p < 0.05). BSG knockout mice have been reported to show a phenotype in which the meibomian gland biologically and structurally similar to the sebaceous gland shrinks due to a decrease in accumulation of lipids (Cell Death and Disease, 2015, 6: e1726). Further, it was evident that the expression level of hydroxycarboxylic ashid receptor 2 (HCAR2) was statistically significantly lower in the sebum secretion high-value group than in the low-value group. HCAR2 has been reported to have an inhibitory effect on accumulation of lipids in macrophages (J Lipid Res, 2014, 2501-2508). These findings suggest that RNA in SSL serves as an useful index indicating the sebum secretion volume.

### 2) Moisture content-related molecules

On the basis of the results of measurement by Corneometer, the top 15 persons in terms of the horn cell layer moisture content (high-value group) and the bottom 15 persons in terms of the horn cell layer moisture content (low-value group) were selected. Comparison of the expression level of SSL-derived RNA (RPM value) between the two groups (low-value group and high-value group) was performed in accordance with the same procedure as in Test Example 7, and the result showed that 553 RNAs statistically significantly varied in expression ((H) of Tables 7-1 to 7-16). A natural moisturizing factor has been reported to play an important role in maintenance of the skin moisturizing capacity (Dermatol Ther, 17 Suppl, 2004, 1: 43-48). Expression of a factor related to generation of the natural moisturizing factor was examined, and the result revealed that as shown in Figure 9, the expression levels of aspartic peptidase retroviral like 1 (ASPRV1), peptidyl arginine deiminase 3 (PADI3) and the like were statistically significantly lower in the low-value group than in the high-value group (Student's t-test, p < 0.05). Mice lacking ASPRV1 have been reported to have a decreased horn cell layer moisture content in actuality (EMBO Mol Med, 2011, 3: 320-333). PADI3 has been reported to play an important role in generation of the natural moisturizing factor (J Invest Dermatol, 2005, 124: 384-393. These findings suggest that RNA in SSL serves as a useful index indicating the skin moisture content.

### 3) Redness-related molecules

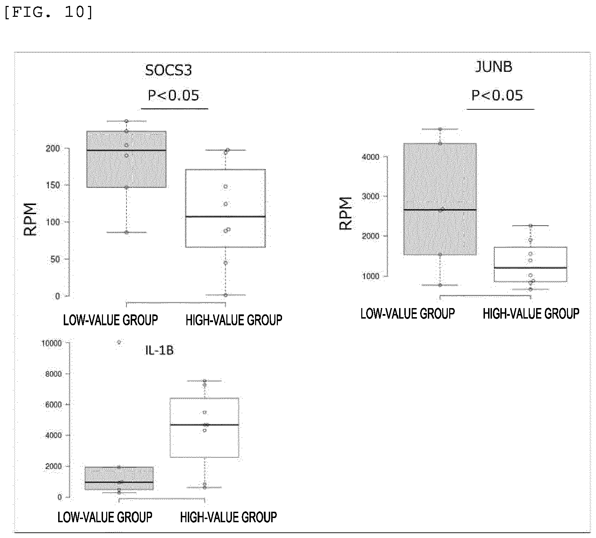

On the basis of the results of visually evaluating face images, 8 persons with intense skin redness (high-value group) and 6 persons with mild skin redness (low-value group) were selected. Comparison of the expression level of SSL-derived RNA (RPM value) between the two groups (low-value group and high-value group) was performed in accordance with the same procedure as in Test Example 7, and the result showed that 703 RNAs statistically significantly varied in expression ((G) of Tables 7-1 to 7-20). It was evident that as shown in Figure 10, the expression levels of suppressor of cytokine shignaling 3 (SOCS3) and JunB proto-oncogene (JUNB), among the above-mentioned RNAs, were statistically significantly lower in the redness high-value group than in the low-value group (Student's t-test, p < 0.05). It has been reported that in knockout mice with JUNB and SOCS3, inflammation is triggered on the skin (Proc. Natl. Acad. Sci. USA, 2009, 106: 20423-20428, PloS One, 2012, 7:e40343). Further, it was evident that the level of IL-1B known to trigger inflammation in the skin tended to be higher in the redness high-value group than in the low-value group although there was no statistically significant difference in the level of IL-1B between the groups. These findings suggest that RNA in SSL serves as a useful index indicating skin redness.

**[Table 7-1]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| ADRA2A | | | OR7E5P | ACADS | OR10A2 | A2M | AASDHPPT | ABCA13 |
| ADRA2B | | | OR8B3 | ACP1 | OR10A3 | ABAT | ACP6 | ABCC1 |
| ADRB1 | OR10A2 | OR10A2 | OR10A2 | ACPP | OR10A5 | ABHD10 | ADAM17 | ABHD12B |
| ADRB2 | OR10A3 | OR10A3 | OR10A3 | ACVR1 | OR10A6 | ABHD13 | ADIPOR1 | ACAD9 |
| APLNR | OR10A4 | OR10A4 | OR10A4 | ADRA2B | OR10A7 | ABHD14B | ADNP2 | ACTR3B |
| AK1 | OR10A5 | OR10A5 | OR10A5 | ADRA2C | OR10AD1 | ABHD8 | AGAP2 | ADAM10 |
| AK4 | OR10A6 | OR10A6 | | AK4 | OR10AG1 | ACAD8 | AGPAT6 | ADAP1 |
| ALDH3A1 | OR10A7 | OR10A7 | | ASCL1 | OR10C1 | ACAT2 | AHCYL2 | AGAP5 |
| ALDH3A2 | OR10AD1 | OR10AD1 | OR10AD1 | ASS1 | OR10G2 | ACO2 | AKAP17A | AGL |
| AKR1B1 | OR10AG1 | OR10AG1 | OR10AG1 | ATP1A2 | OR10G3 | ACOT2 | AKAP9 | AGR2 |
| SLC25A4 | OR10C1 | OR10C1 | OR10G2 | ATP12A | OR10G4 | ACOT4 | AKR1E2 | AHNAK2 |
| ANXA2P1 | OR10G2 | OR10G2 | OR10G3 | BARD1 | OR10G7 | ACOT7 | ALDH16A1 | AKAP11 |
| ANXA2P2 | OR10G3 | OR10G3 | | BBS1 | OR10G8 | ACP1 | ALG1 | ALG1 |
| ANXA2P3 | OR10G4 | OR10G4 | | BPGM | OR10G9 | ACPL2 | AMDHD1 | ALX1 |
| ANXA3 | OR10G7 | OR10G7 | OR10G7 | CDC27 | OR10H1 | ACVR2A | AMIGO1 | ANAPC2 |
| APBB1 | OR10G8 | OR10G8 | | CDKN2B | OR10H2 | ADAM15 | AMMECR1L | ANP32C |
| APOBEC1 | OR10G9 | OR10G9 | | CHEK1 | OR10H3 | ADIPOR2 | AMPD2 | ANXA2 |
| ARG2 | OR10H1 | OR10H1 | | CLNS1A | OR10H4 | ADNP2 | ANKRD33B | AP3B1 |
| ARSF | OR10H2 | OR10H2 | OR10H2 | CNTFR | OR10H5 | AIFM2 | ANKRD54 | AP3M2 |
| ASS1 | OR10H3 | OR10H3 | OR10H3 | COL2A1 | OR10J1 | AJUBA | APLF | APBB2 |
| BDKRB1 | OR10H5 | OR10H5 | OR10H5 | CLDN4 | OR10J3 | AKIRIN1 | ARFGAP1 | APOL1 |
| BMP2 | OR10J1 | OR10J1 | OR10J1 | CRY1 | OR10J5 | AKNA | ARHGEF35 | AQP7 |
| BMP7 | OR10J3 | OR10J3 | OR10J3 | CRY2 | OR10K1 | AKR7A2 | ARID3A | ARG1 |
| BMPR1A | OR10J5 | OR10J5 | OR10J5 | CS | OR10K2 | AKT1 | ARID4B | ARHGAP4 |
| BOK | OR10K1 | OR10K1 | OR10K1 | CSNK2A1 | OR10Q1 | AKT2 | ARMC6 | ARHGEF35 |
| BTF3P11 | OR10K2 | OR10K2 | OR10K2 | DYNC1I2 | OR10R2 | ALG1 | ARPC5L | ARL11 |
| BUB1 | OR10P1 | OR10P1 | OR10P1 | DNMT3A | OR10S1 | AMT | ARTN | ASPRV1 |
| ERC2-IT1 | OR10Q1 | OR10Q1 | | DRD5 | OR10T2 | ANKDD1A | ASB6 | ATP12A |
| MRPL49 | OR10R2 | OR10R2 | OR10R2 | DSC2 | OR10V1 | ANO10 | ASPN | ATP2C2 |
| ZNHIT2 | OR10S1 | OR10S1 | OR10S1 | E2F6 | OR10W1 | ANP32E | ATP6V0D1 | ATP8B3 |
| CACNA2D1 | OR10T2 | OR10T2 | OR10T2 | ELF3 | OR10X1 | AOAH | ATPAF2 | B4GALT1 |
| CACNB1 | OR10V1 | OR10V1 | OR10V1 | FANCB | OR11A1 | APC | ATR | BASP1 |
| CALB2 | OR10W1 | OR10W1 | OR10W1 | FANCF | OR11G2 | APPL1 | ATXN8OS | BBS10 |
| CALD1 | OR10X1 | OR10X1 | OR10X1 | FANCG | OR11H1 | ARF5 | AZIN1 | BBS12 |
| CAV1 | OR10Z1 | OR10Z1 | OR10Z1 | FEN1 | OR11H12 | ARFIP1 | B4GALT7 | BCL2L1 |
| CCIN | OR11A1 | OR11A1 | | FOXD1 | OR11H2 | ARG2 | BATF2 | BCL6B |
| CD59 | OR11G2 | OR11G2 | OR11G2 | FOXL1 | OR11H4 | ARID2 | BAZ1A | BICD2 |

**[Table 7-2]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| CDC25A | OR11H1 | OR11H1 | OR11H1 | FOXO3B | OR11H6 | ARSK | BCL2L15 | BLOC1S3 |
| CDC27 | OR11H12 | OR11H12 | OR11H12 | GPR31 | OR11L1 | ASAP1 | BCL9 | BMP4 |
| CDC34 | OR11H2 | OR11H2 | | GPS2 | OR12D2 | ASCC2 | BEX2 | BNIP3L |
| CDKN1C | OR11H4 | OR11H4 | OR11H4 | HAGH | OR12D3 | ASPRV1 | BEX4 | BZW1 |
| CDKN2C | OR11H6 | OR11H6 | OR11H6 | HMGN2 | OR13C2 | ATF3 | BHMT | C10orf131 |
| CDR1 | OR11L1 | OR11L1 | | HNF4G | OR13C3 | ATG10 | BIRC5 | LINC00619 |
| CDS1 | OR12D2 | OR12D2 | OR12D2 | HNRNPA1 | OR13C4 | ATG3 | BMP8B | C10orf62 |
| CEBPE | OR12D3 | OR12D3 | | HSD17B1 | OR13C5 | ATIC | BRD2 | PLEKHS1 |
| CETN1 | OR13C2 | OR13C2 | OR13C2 | ID1 | OR13C8 | ATP2B1 | C11orf1 | SPATA32 |
| CGA | OR13C3 | OR13C3 | OR13C3 | IDH1 | OR13C9 | ATP5D | C11orf63 | NCMAP |
| CHAD | OR13C4 | OR13C4 | OR13C4 | IFNA16 | OR13F1 | ATP6AP1 | C11orf9 | C1orf131 |
| CHRM4 | OR13C5 | OR13C5 | OR13C5 | IGHMBP2 | OR13H1 | ATP6V1A | C12orf71 | C2orf27B |
| CHRNB1 | OR13C8 | OR13C8 | OR13C8 | IL11 | OR13J1 | ATXN10 | GSKIP | C3orf27 |
| CLNS1A | OR13C9 | OR13C9 | OR13C9 | INPPL1 | OR14A16 | AVPI1 | FAM219B | C6orf226 |
| LTB4R | OR13D1 | OR13D1 | | INSM1 | OR14C36 | AZIN1 | ENTHD2 | C7orf25 |
| CNN3 | OR13F1 | OR13F1 | OR13F1 | IRF6 | OR14I1 | BACH1 | C19orf35 | C8orf86 |
| CNTN1 | OR13H1 | OR13H1 | | ISLR | OR14J1 | BAHD1 | C1orf131 | CAMK1G |
| COL4A1 | OR13J1 | OR13J1 | OR13J1 | KCNA3 | OR1A1 | BANP | SERTAD4-AS1 | CAPN1 |
| COMP | OR14A16 | OR14A16 | OR14A16 | KRT7 | OR1A2 | BCAM | C1QL2 | CASKIN2 |
| COX7A2 | OR14C36 | OR14C36 | OR14C36 | KRT9 | OR1B1 | BCKDHB | C1QTNF6 | CASP1 |
| CLDN3 | OR14I1 | OR14I1 | OR14I1 | LIG1 | OR1C1 | BCL6 | FAM176C | CATSPER2 |
| CRABP1 | OR14J1 | OR14J1 | OR14J1 | SH2D1A | OR1D2 | BIRC2 | C21orf91 | CCDC112 |
| CRYAB | OR1A1 | OR1A1 | OR1A1 | MARS | OR1D4 | BLNK | C3orf14 | CCDC36 |
| CTBP2 | OR1A2 | OR1A2 | OR1A2 | MEST | OR1D5 | BRD3 | C3orf17 | CCDC64B |
| CYB5A | OR1B1 | OR1B1 | OR1B1 | MGAT5 | OR1E1 | BRI3BP | C3orf70 | CCNL2 |
| CYP8B1 | OR1C1 | OR1C1 | OR1C1 | MIPEP | OR1E2 | BRIP1 | C5orf4 | CCPG1 |
| CYP21A2 | OR1D2 | OR1D2 | OR1D2 | MMP13 | OR1F1 | BTN2A1 | C6orf170 | CD200 |
| CYP21A1P | OR1D4 | OR1D4 | OR1D4 | MSH5 | OR1F2P | C11orf35 | C9orf91 | CD82 |
| CYP24A1 | OR1D5 | OR1D5 | OR1D5 | MT1M | OR1G1 | SYNE3 | CA7 | CDKN3 |
| DEFA4 | OR1E1 | OR1E1 | | MUC2 | OR1I1 | C17orf107 | CABLES2 | CDYL |
| DEFB1 | OR1E2 | OR1E2 | | MYH3 | OR1J2 | GID4 | CALCA | CEP55 |
| DHCR24 | OR1F1 | OR1F1 | OR1F1 | NAB2 | OR1J4 | C19orf33 | CAMK1 | CES2 |
| DHFR | OR1F2P | OR1F2P | OR1F2P | NASP | OR1K1 | C19orf38 | CAMKK2 | CGRRF1 |
| NQO1 | OR1G1 | OR1G1 | OR1G1 | NCL | OR1L1 | C1orf115 | CAND1 | CHRM4 |
| DLX3 | OR1I1 | OR1I1 | OR1I1 | NDUFA5 | OR1L4 | C1orf35 | CAPN7 | CKAP2L |

**[Table 7-3]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| DLX5 | OR1J1 | OR1J1 | OR1J1 | NDUFS3 | OR1L6 | C1orf50 | CAPRIN2 | CKMT1A |
| DNASE1 | OR1J2 | OR1J2 | OR1J2 | NKG7 | OR1L8 | SDE2 | CARNS1 | CLCC1 |
| DOCK3 | OR1J4 | OR1J4 | OR1J4 | NONO | OR1M1 | SUCO | CCDC115 | CLEC2A |
| DPH1 | OR1K1 | OR1K1 | OR1K1 | NPAT | OR1N1 | C1QB | CCDC126 | CLEC3A |
| DRD2 | OR1L1 | OR1L1 | OR1L1 | NPM1 | OR1N2 | C1RL | CCDC127 | CLTB |
| DRD5 | OR1L3 | OR1L3 | OR1L3 | NPY1R | OR1Q1 | C2CD2 | SOGA2 | CNGB1 |
| DUSP5 | OR1L4 | OR1L4 | OR1L4 | OR2C1 | OR1S1 | ADTRP | CCDC17 | COG8 |
| DUSP8 | OR1L6 | OR1L6 | | SLC22A18 | OR1S2 | C6orf132 | CCDC28B | COL13A1 |
| E2F1 | OR1L8 | OR1L8 | OR1L8 | SERPINE1 | OR2A1 | C7orf26 | CCDC36 | COL5A3 |
| E2F5 | OR1M1 | OR1M1 | OR1M1 | CHMP1A | OR2A12 | C9orf142 | CCDC71L | COL6A4P2 |
| E2F6 | OR1N1 | OR1N1 | OR1N1 | PFAS | OR2A14 | C9orf169 | TMA7 | COMMD9 |
| S1PR1 | OR1N2 | OR1N2 | OR1N2 | PGM3 | OR2A2 | RABL6 | CCL24 | CPA4 |
| PHC1 | OR1Q1 | OR1Q1 | | PIGR | OR2A20P | CALML3 | CCL28 | CPA5 |
| EFNB2 | OR1S1 | OR1S1 | OR1S1 | PLD2 | OR2A25 | CASP2 | CD163L1 | CRAMP1L |
| CELSR2 | OR1S2 | OR1S2 | OR1S2 | POLRMT | OR2A4 | CASP4 | CDCA8 | CRYGD |
| MEGF9 | OR2A1 | OR2A1 | | POU3F1 | OR2A42 | CASS4 | CDK8 | CRYL1 |
| EIF4B | OR2A12 | OR2A12 | | POU5F1B | OR2A5 | CASZ1 | CEACAM20 | CSNK1A1P1 |
| EML1 | OR2A14 | OR2A14 | | PPOX | OR2A7 | CCDC124 | CEP63 | CSRP2BP |
| EPHB6 | OR2A2 | OR2A2 | OR2A2 | PPP1R3D | OR2A9P | CCDC64B | CES4A | CTBS |
| EPHX2 | OR2A20P | OR2A20P | | PPP3CC | OR2AE1 | CCDC66 | CHCHD4 | CTDSP1 |
| EXTL2 | OR2A25 | OR2A25 | | MAP2K5 | OR2AG1 | CCP110 | CHMP7 | CTSC |
| F2R | OR2A4 | OR2A4 | OR2A4 | PRODH | OR2AG2 | CD274 | CHST8 | CTSH |
| FANCF | OR2A42 | OR2A42 | | PROS1 | OR2AK2 | CD28 | CHUK | CTSL2 |
| EFEMP1 | OR2A5 | OR2A5 | OR2A5 | PSMC6 | OR2AT4 | CD3E | CITED1 | CUTC |
| FCGR2A | OR2A7 | OR2A7 | OR2A7 | TAS2R38 | OR2B11 | CDA | CLCA4 | CWF19L2 |
| GPC4 | OR2A9P | OR2A9P | OR2A9P | PTK7 | OR2B2 | CDC34 | CLDN12 | CXXC4 |
| FGF9 | OR2AE1 | OR2AE1 | OR2AE1 | PWP2 | OR2B3 | CDC40 | CLDND1 | CYP2E1 |
| FGF13 | OR2AG1 | OR2AG1 | OR2AG1 | QARS | OR2B6 | CDC42BPG | CLIP4 | CYP8B1 |
| FOXG1 | OR2AG2 | OR2AG2 | OR2AG2 | RARS | OR2C1 | CDC42EP1 | CLMP | CYYR1 |
| FOXF1 | OR2AK2 | OR2AK2 | OR2AK2 | RB1 | OR2D2 | CDIPT | CLP1 | DAD1 |
| FOXC1 | OR2AT4 | OR2AT4 | OR2AT4 | SNORA62 | OR2D3 | CEACAM5 | CLPS | DAO |
| FOXD1 | OR2B11 | OR2B11 | OR2B11 | RNF2 | OR2F1 | CENPP | CMYA5 | DAPP1 |
| FOXD4 | OR2B2 | OR2B2 | OR2B2 | SNORD15A | OR2F2 | CEP250 | CNOT6 | DARC |
| FOXL1 | OR2B3 | OR2B3 | OR2B3 | MRPL12 | OR2G3 | CERS4 | COL16A1 | DCAKD |
| FOXE3 | OR2B6 | OR2B6 | OR2B6 | RPS26 | OR2G6 | CFD | COR06 | DCHS2 |

**[Table 7-4]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| FOXC2 | OR2C1 | OR2C1 | OR2C1 | SDHC | OR2H1 | CHD1L | CPNE4 | DCTN1 |
| FOXE1 | OR2D2 | OR2D2 | OR2D2 | ST3GAL3 | OR2H2 | CHMP4B | CR1L | DEPDC1 |
| FOXD2 | OR2D3 | OR2D3 | OR2D3 | SKI | OR2J3 | CHMP5 | CRCP | DGCR10 |
| FOXO3B | OR2F1 | OR2F1 | OR2F1 | SLC5A2 | OR2K2 | CIDEA | CREB3L2 | DIP2A |
| FLG | OR2F2 | OR2F2 | | SUMO2 | OR2L1P | CLDN4 | CRIP1 | DNAJB8 |
| FRK | OR2G2 | OR2G2 | OR2G2 | SNCA | OR2L2 | CLEC4E | CRISPLD2 | DNASE2 |
| FTH1P3 | OR2G3 | OR2G3 | OR2G3 | SOX1 | OR2L3 | CNOT6L | CRYZL1 | DPYS |
| FUT2 | OR2G6 | OR2G6 | OR2G6 | SOX9 | OR2L8 | CNPPD1 | CSPG5 | DSTN |
| FUT4 | OR2H1 | OR2H1 | | SRP9 | OR2M1P | COL6A1 | CSTF3 | DTYMK |
| GAS1 | OR2H2 | OR2H2 | OR2H2 | ST13 | OR2M2 | COQ7 | CTAGE5 | DUSP5 |
| OPN1MW | OR2J2 | OR2J2 | OR2J2 | STAR | OR2M3 | CORO1A | CTNNBIP1 | DYRK1B |
| GCSH | OR2J3 | OR2J3 | OR2J3 | SURF1 | OR2M4 | COX6A1 | CTPS1 | EAF2 |
| GDF1 | OR2K2 | OR2K2 | OR2K2 | TBX6 | OR2M5 | CRAT | CTR9 | ECEL1P2 |
| GFRA1 | OR2L1P | OR2L1P | OR2L1P | ICAM5 | OR2M7 | CRIPT | CTSA | ECT2 |
| GK2 | OR2L2 | OR2L2 | | TMPRSS2 | OR2T10 | CS | CXCL14 | EEF1A2 |
| GK3P | OR2L3 | OR2L3 | OR2L3 | TNXB | OR2T11 | CSDC2 | CXorf40B | EEF1G |
| GLI4 | OR2L8 | OR2L8 | OR2L8 | TRAF1 | OR2T12 | CSNK1A1 | CXorf56 | ELF3 |
| GLUD2 | OR2M1P | OR2M1P | OR2M1P | TSPYL1 | OR2T2 | CSTB | CYP4F8 | EPDR1 |
| GMPR | OR2M2 | OR2M2 | OR2M2 | TTC3 | OR2T27 | CSTF3 | DAP | EPHB4 |
| NPBWR1 | OR2M3 | OR2M3 | OR2M3 | TTF1 | OR2T29 | CTNNBIP1 | DBR1 | EPOR |
| NPBWR2 | OR2M4 | OR2M4 | OR2M4 | SUMO1 | OR2T3 | CTNND1 | DCAF17 | EVPLL |
| UTS2R | OR2M5 | OR2M5 | OR2M5 | UPK1B | OR2T35 | CTSC | DCDC2 | EYA4 |
| GPR21 | OR2M7 | OR2M7 | OR2M7 | CORO2A | OR2T4 | CTSH | DCUN1D3 | FAM108A1 |
| GPR25 | OR2S2 | OR2S2 | OR2S2 | ZKSCAN1 | OR2T6 | CTSL1 | DDR2 | FAM111B |
| GPR32 | OR2T1 | OR2T1 | OR2T1 | ZNF230 | OR2T8 | CXCL17 | DDX1 | FAM114A2 |
| FFAR1 | OR2T10 | OR2T10 | | MOGS | OR2V2 | CYB5R4 | DDX52 | FAM122A |
| GPX2 | OR2T11 | OR2T11 | OR2T11 | RASSF7 | OR2W1 | CYBRD1 | DEFB134 | FAM135A |
| GRM2 | OR2T12 | OR2T12 | OR2T12 | HIST1H2AM | OR2W3 | CYFIP1 | DEGS2 | FAM175A |
| GSTA3 | OR2T2 | OR2T2 | OR2T2 | HIST1H2BH | OR2W5 | CYP4F3 | DHTKD1 | FAM206A |
| GSTA4 | OR2T27 | OR2T27 | OR2T27 | HIST1H2BC | OR2Y1 | CYTH2 | DHX37 | FAM65C |
| HIST1H1T | OR2T29 | OR2T29 | OR2T29 | HIST2H2BE | OR2Z1 | CYTIP | DHX58 | FAM70A |
| HIST1H2AE | OR2T3 | OR2T3 | OR2T3 | HIST1H3E | OR3A1 | DAB2IP | DIAPH1 | FAM82A2 |
| HIST1H2AD | OR2T33 | OR2T33 | | HIST1H4C | OR3A2 | DCTD | DMXL1 | FAM89B |
| HIST1H2BB | OR2T35 | OR2T35 | | HIST1H4G | OR3A3 | DDT | DNA2 | FBXO18 |
| HMGCS2 | OR2T4 | OR2T4 | | OR1D5 | OR3A4P | DDX54 | DNAAF2 | FCER1G |

**[Table 7-5]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| HMGA1 | OR2T6 | OR2T6 | OR2T6 | SOX14 | OR4A15 | DEGS2 | DNAJB11 | FCGR1C |
| HMOX1 | OR2T8 | OR2T8 | OR2T8 | SPOP | OR4A16 | DGCR2 | DNAJB8 | FDPS |
| FOXA1 | OR2V2 | OR2V2 | | NSMAF | OR4A47 | DGCR6L | DNAJC6 | FGL2 |
| HNF4G | OR2W1 | OR2W1 | OR2W1 | CDK10 | OR4A5 | DHCR7 | DNTTIP2 | FHDC1 |
| HOXA1 | OR2W3 | OR2W3 | | OR6A2 | OR4B1 | DHPS | DPH5 | FLG2 |
| HP | OR2W5 | OR2W5 | OR2W5 | DCHS1 | OR4C11 | DHX40 | DRGX | FLJ14107 |
| HPD | OR2Y1 | OR2Y1 | | JRKL | OR4C12 | DMC1 | DUSP18 | FLOT1 |
| ERAS | OR2Z1 | OR2Z1 | | B3GALT4 | OR4C13 | DNAJA1 | DUSP4 | FRS3 |
| HRC | OR3A1 | OR3A1 | | MATN4 | OR4C15 | DNAJB1 | EBAG9 | FSCB |
| HSD17B1 | OR3A2 | OR3A2 | OR3A2 | SYNGAP1 | OR4C16 | DNAJB9 | EBF4 | FTH1P3 |
| HSD17B3 | OR3A3 | OR3A3 | | VNN1 | OR4C3 | DNAJC17 | ECRP | FTL |
| HTR1A | OR3A4P | OR3A4P | | EIF2S2 | OR4C46 | DNASE1L2 | ECT2L | FXC1 |
| HTR1B | OR4A15 | OR4A15 | OR4A15 | TIMELESS | OR4C6 | DOPEY2 | EFCAB4B | GABRE |
| HTR1D | OR4A16 | OR4A16 | OR4A16 | BAIAP3 | OR4D1 | DPP3 | EFEMP1 | GADD45A |
| HYAL1 | OR4A47 | OR4A47 | OR4A47 | USP13 | OR4D10 | DUSP23 | EGFL8 | GCGR |
| ID4 | OR4A5 | OR4A5 | OR4A5 | HSPB3 | OR4D11 | DUSP27 | EIF2S3 | GCNT4 |
| IFNA1 | OR4B1 | OR4B1 | OR4B1 | CH25H | OR4D2 | DUSP7 | EIF4A3 | GDPD3 |
| IFNA2 | OR4C11 | OR4C11 | OR4C11 | USP2 | OR4D5 | DVL3 | EIF4H | GGA1 |
| IFNA4 | OR4C12 | OR4C12 | OR4C12 | SMC3 | OR4D6 | DYDC2 | EML6 | GJB4 |
| IFNA5 | OR4C13 | OR4C13 | OR4C13 | ZMYM3 | OR4D9 | DYRK1A | ENDOU | GJB5 |
| IFNA7 | OR4C15 | OR4C15 | | MAGI1 | OR4E2 | EAF1 | ENPP7 | GNA15 |
| IFNA8 | OR4C16 | OR4C16 | OR4C16 | PNMA1 | OR4F15 | ECHDC2 | ENTPD7 | GNG3 |
| IFNA10 | OR4C3 | OR4C3 | OR4C3 | TRIP4 | OR4F17 | EDC3 | EPB41L2 | GNG8 |
| IFNA13 | OR4C46 | OR4C46 | OR4C46 | UBE4A | OR4F21 | EEF1D | EPS8L2 | GOLPH3L |
| IFNA14 | OR4C6 | OR4C6 | OR4C6 | RAB28 | OR4F3 | EEF2K | EREG | GON4L |
| IFNA16 | OR4D1 | OR4D1 | OR4D1 | RAB9BP1 | OR4F4 | EGLN1 | ERI1 | GPN1 |
| IFNA17 | OR4D10 | OR4D10 | OR4D10 | HS6ST1 | OR4F5 | EIF2C3 | ERVK13-1 | GPR110 |
| IFNA21 | OR4D11 | OR4D11 | | RPH3AL | OR4F6 | EIF4A3 | ESM1 | GPR157 |
| IFNA22P | OR4D2 | OR4D2 | OR4D2 | PITPNM1 | OR4K1 | EIF6 | ESRRA | GPR27 |
| IFNB1 | OR4D5 | OR4D5 | OR4D5 | AATK | OR4K13 | ELF3 | ETFA | GRK6 |
| IFNW1 | OR4D6 | OR4D6 | OR4D6 | PHF14 | OR4K14 | ELOVL3 | EYA1 | GSTTP2 |
| CYR61 | OR4D9 | OR4D9 | OR4D9 | KIAA0100 | OR4K15 | ELP2 | FAIM2 | GTPBP6 |
| CXCR2P1 | OR4E2 | OR4E2 | OR4E2 | FAM131B | OR4K2 | EMD | FAM108B1 | GUCA1B |
| IL11 | OR4F15 | OR4F15 | OR4F15 | PHACTR2 | OR4K5 | ENO3 | FAM125B | GUCA2B |
| INSM1 | OR4F17 | OR4F17 | OR4F17 | MED24 | OR4L1 | EPN3 | FAM135A | GYG1 |
| IVL | OR4F21 | OR4F21 | OR4F21 | MAGEC1 | OR4M1 | ERC1 | FAM180B | HBEGF |

**[Table 7-6]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| JUN | OR4F3 | OR4F3 | OR4F3 | SH2D3A | OR4M2 | ERF | FAM3C | HCAR2 |
| KCNA2 | OR4F4 | OR4F4 | OR4F4 | TSPAN2 | OR4N2 | ERGIC3 | FAM46C | HCG26 |
| KCNA10 | OR4F5 | OR4F5 | | TSPAN1 | OR4N3P | EVI2B | FAM65C | HEATR4 |
| KCNF1 | OR4F6 | OR4F6 | OR4F6 | TRIM10 | OR4N4 | EXOSC1 | FAM72A | HECTD1 |
| KCNJ11 | OR4K1 | OR4K1 | OR4K1 | TRIM28 | OR4N5 | EZR | FAM90A1 | HENMT1 |
| KLK1 | OR4K13 | OR4K13 | OR4K13 | LPAR6 | OR4P4 | FABP3 | FAM92A1 | HIGD1A |
| KRT7 | OR4K14 | OR4K14 | OR4K14 | DHRS2 | OR4Q3 | FAM102A | FBXL16 | HIST1H3A |
| KRT8 | OR4K15 | OR4K15 | OR4K15 | STAM2 | OR4S1 | FAM114A2 | FERMT1 | HIST1H3F |
| KRT15 | OR4K17 | OR4K17 | | CWC27 | OR4S2 | FAM123A | FEZF2 | HIST2H2AB |
| KRT18 | OR4K2 | OR4K2 | OR4K2 | B3GNT3 | OR4X1 | FAM129B | FLJ34503 | HK2 |
| KRT19 | OR4K5 | OR4K5 | OR4K5 | PKDREJ | OR4X2 | FAM135A | FMO5 | HK3 |
| LCK | OR4L1 | OR4L1 | OR4L1 | UBE2E3 | OR51A2 | EMC9 | FNBP4 | HMG20B |
| LCN2 | OR4M1 | OR4M1 | OR4M1 | CREB3 | OR51A4 | FAM200B | FOPNL | HMGCL |
| LEPR | OR4M2 | OR4M2 | OR4M2 | SEMA6C | OR51A7 | FAM212B | FOXO4 | HMGCS2 |
| LIPE | OR4N2 | OR4N2 | OR4N2 | SLC19A2 | OR51B2 | FAM214B | FREM1 | HNRNPF |
| LRP6 | OR4N3P | OR4N3P | OR4N3P | ARID3B | OR51B5 | FAM84B | FSCN2 | HOXD12 |
| LTK | OR4N4 | OR4N4 | OR4N4 | NPRL2 | OR51B6 | FAM91A1 | FUS | HS3ST3A1 |
| MAB21L1 | OR4N5 | OR4N5 | OR4N5 | ZMYND11 | OR51D1 | FAM96A | G0S2 | HS3ST3B1 |
| MAGEB4 | OR4P4 | OR4P4 | OR4P4 | OR5I1 | OR51E1 | FAM96B | GABPB1 | HSD3B2 |
| MAK | OR4Q3 | OR4Q3 | | HSPH1 | OR51F2 | FBXL3 | GALC | HSP90AB1 |
| MAS1 | OR4S1 | OR4S1 | | COPS8 | OR51G1 | FBXO31 | GAPDHS | HSPA9 |
| MC1R | OR4S2 | OR4S2 | OR4S2 | DBF4 | OR51G2 | FBXO32 | GCC2 | HSPBP1 |
| MC3R | OR4X1 | OR4X1 | OR4X1 | STIP1 | OR51I1 | FBXW2 | GDI2 | HUWE1 |
| MC4R | OR4X2 | OR4X2 | OR4X2 | CLP1 | OR51I2 | FBXW5 | GEMIN6 | HYAL1 |
| MC5R | OR51A2 | OR51A2 | | COPS6 | OR51L1 | FBXW7 | GEN1 | IDE |
| MCAM | OR51A4 | OR51A4 | OR51A4 | ILVBL | OR51M1 | FDX1L | GGNBP2 | IDH3A |
| MDK | OR51A7 | OR51A7 | OR51A7 | SLC27A5 | OR51Q1 | FGD2 | GNAI1 | IFFO2 |
| MEIS3P1 | OR51B2 | OR51B2 | OR51B2 | DDX52 | OR51S1 | FGF22 | GNAQ | IL27 |
| MFGE8 | OR51B4 | OR51B4 | | ADAM30 | OR51T1 | FIS1 | GOLGA5 | IMPG2 |
| MGAT3 | OR51B6 | OR51B6 | OR51B6 | KATNA1 | OR51V1 | FLII | GOLGA6D | INPP4B |
| SCGB2A2 | OR51D1 | OR51D1 | OR51D1 | CAPN11 | OR52A1 | FPR2 | GPCPD1 | INSC |
| MGST1 | OR51E1 | OR51E1 | | RASSF1 | OR52A5 | FRMD8 | GRAP2 | IQGAP1 |
| MME | OR51F1 | OR51F1 | OR51F1 | CEP250 | OR52B2 | FSTL3 | GSDMC | IRF2BP1 |
| MMP13 | OR51F2 | OR51F2 | | POLI | OR52B4 | FTH1 | HABP2 | IRF2BPL |
| ALDH6A1 | OR51G1 | OR51G1 | | XPOT | OR52B6 | FXR1 | HARS2 | ITGAL |
| MST1 | OR51G2 | OR51G2 | OR51G2 | SNF8 | OR52D1 | G6PD | HAUS6 | JKAMP |

**[Table 7-7]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| MST1R | OR51I1 | OR51I1 | OR51I1 | GPN1 | OR52E2 | GAGE2B | LAMTOR5 | JUP |
| MSX1 | OR51I2 | OR51I2 | | ZNF507 | OR52E4 | GALP | HDAC1 | KANK1 |
| MT1M | OR51L1 | OR51L1 | OR51L1 | ICK | OR52E6 | GCHFR | HDAC9 | KBTBD13 |
| MT1X | OR51M1 | OR51M1 | OR51M1 | RAB3GAP1 | OR52E8 | GDPD3 | HDHD2 | KCNMB4 |
| MUC2 | OR51Q1 | OR51Q1 | | ENDOD1 | OR52H1 | GEMIN8 | HEY1 | KCTD4 |
| MYBPH | OR51S1 | OR51S1 | OR51S1 | NBEAL2 | OR52I1 | GET4 | HGFAC | KCTD5 |
| MYH3 | OR51T1 | OR51T1 | | ZC3H7B | OR52I2 | GGPS1 | HIATL1 | KDM4D |
| MYO5B | OR51V1 | OR51V1 | OR51V1 | EXOC7 | OR52J3 | GHDC | HOXC13 | KIAA0141 |
| NAP1L2 | OR52A1 | OR52A1 | OR52A1 | KLHL18 | OR52K2 | GIPC3 | HSD17B3 | KIAA0930 |
| NAP1L3 | OR52A5 | OR52A5 | OR52A5 | SUN1 | OR52L1 | GJB3 | HSF1 | KIAA1683 |
| NDN | OR52B2 | OR52B2 | OR52B2 | MAU2 | OR52M1 | GJB4 | HSF2 | KIAA1704 |
| NDUFA9 | OR52B4 | OR52B4 | OR52B4 | SLC7A8 | OR52N1 | GLA | HSPA2 | KIAA1958 |
| NEO1 | OR52B6 | OR52B6 | OR52B6 | RHOQ | OR52N2 | GLO1 | HSPA4 | KIF13B |
| NFIA | OR52D1 | OR52D1 | | GCAT | OR52N4 | GMEB1 | HSPH1 | KLHDC2 |
| NFE2 | OR52E2 | OR52E2 | OR52E2 | SCRIB | OR52N5 | GNGT2 | IFFO1 | KLHL21 |
| NFIB | OR52E4 | OR52E4 | | ADAT1 | OR52R1 | GOT1 | IGF2BP3 | KLHL4 |
| NPAS2 | OR52E6 | OR52E6 | OR52E6 | WBP1 | OR52W1 | GPCPD1 | IL17RA | KLK12 |
| NPHP1 | OR52E8 | OR52E8 | OR52E8 | PRG1 | OR56A1 | GPR161 | ILF3 | KLK13 |
| ROR1 | OR52H1 | OR52H1 | OR52H1 | FAM215A | OR56A3 | GPR56 | IMPA2 | KLK6 |
| GPR143 | OR52I1 | OR52I1 | OR52I1 | MKRN2 | OR56A4 | GPT | ING2 | KLK9 |
| OMP | OR52I2 | OR52I2 | OR52I2 | LRRC6 | OR56A5 | GPT2 | IQCF2 | KLRC2 |
| OR1D2 | OR52J3 | OR52J3 | OR52J3 | LDOC1 | OR56B1 | GRB7 | IQCK | KRT17 |
| OR1F1 | OR52K1 | OR52K1 | | EDC4 | OR5A1 | GSDMA | IRX6 | KRT6A |
| OR3A1 | OR52K2 | OR52K2 | OR52K2 | SSBP3 | OR5A2 | GSK3A | ISM1 | KRT6B |
| OTX1 | OR52L1 | OR52L1 | | MAFF | OR5AC2 | GUF1 | ITFG2 | KRTAP4-5 |
| OVGP1 | OR52M1 | OR52M1 | OR52M1 | CIZ1 | OR5AK2 | GUSB | ITGB1 | KRTAP4-6 |
| P2RY4 | OR52N1 | OR52N1 | | DPCD | OR5AK4P | H19 | ITGB3 | LCN2 |
| PABPC3 | OR52N2 | OR52N2 | OR52N2 | THUMPD3 | OR5AN1 | HACL1 | KAT6B | LIMCH1 |
| PAEP | OR52N4 | OR52N4 | OR52N4 | ZNF385A | OR5AP2 | HBD | KCNU1 | LINC00273 |
| PCCA | OR52N5 | OR52N5 | OR52N5 | TENM4 | OR5AR1 | HEATR6 | KCTD1 | LINC00323 |
| PDGFRA | OR52R1 | OR52R1 | OR52R1 | GIGYF2 | OR5AS1 | HGC6.3 | KCTD15 | LINC00442 |
| PDHA2 | OR52W1 | OR52W1 | OR52W1 | DKFZP434H168 | OR5B12 | HIF1A | KDM4C | LMTK3 |
| PDK2 | OR56A1 | OR56A1 | OR56A1 | EPC2 | OR5B17 | HINT2 | KIAA0408 | LOC100129480 |
| PDZK1 | OR56A3 | OR56A3 | OR56A3 | SERBP1 | OR5B2 | HINT3 | KIAA2013 | RSU1P2 |
| PFN2 | OR56A4 | OR56A4 | OR56A4 | OR1C1 | OR5B21 | HIPK3 | KIF1B | LOC100240734 |
| PGK2 | OR56A5 | OR56A5 | | TINF2 | OR5C1 | HIVEP2 | KIF1C | LOC100287042 |

**[Table 7-8]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| SERPINA1 | OR56B1 | OR56B1 | OR56B1 | OR5L2 | OR5D13 | HMGA1 | KLHDC5 | LOC100505716 |
| PIN1P1 | OR56B4 | OR56B4 | | OR10J1 | OR5D14 | HMGCL | KLHL4 | LOC100506013 |
| PIP | OR5A1 | OR5A1 | OR5A1 | OR12D2 | OR5D16 | HMGXB3 | KRT16P2 | LOC100506422 |
| PKP1 | OR5A2 | OR5A2 | OR5A2 | OR7A5 | OR5D18 | HMOX2 | KRT18 | LINC00629 |
| PLA2G2A | OR5AC2 | OR5AC2 | OR5AC2 | OR4F3 | OR5E1P | HRAS | LCA5L | LOC100506888 |
| POLR2K | OR5AK2 | OR5AK2 | | OR4D1 | OR5F1 | HSBP1L1 | LEO1 | MKNK1-AS1 |
| PON3 | OR5AK4P | OR5AK4P | OR5AK4P | TCL6 | OR5H1 | HSD3B2 | LEPREL4 | LOC255512 |
| POU3F1 | OR5AN1 | OR5AN1 | OR5AN1 | SDCBP2 | OR5H14 | HSPA2 | LGI3 | LOC374443 |
| POU5F1B | OR5AP2 | OR5AP2 | OR5AP2 | NDOR1 | OR5H2 | HSPA8 | LHX9 | LOC400752 |
| PPP1R1A | OR5AR1 | OR5AR1 | OR5AR1 | ATAD2 | OR5H6 | ID1 | LIMD1 | LOC401010 |
| PPP1R3D | OR5AS1 | OR5AS1 | | RBM15B | OR5I1 | IER5 | LOC100129534 | LOC554206 |
| PPP1R8 | OR5AU1 | OR5AU1 | OR5AU1 | HCFC2 | OR5K4 | IFI35 | TSTD3 | LOC645166 |
| PPP3R2 | OR5B12 | OR5B12 | OR5B12 | PKN3 | OR5L1 | IFNAR2 | LINC00605 | LOC646471 |
| PRF1 | OR5B17 | OR5B17 | | PADI1 | OR5L2 | IFNGR1 | LOC100133315 | LOC653160 |
| PRKACG | OR5B2 | OR5B2 | | PIK3R4 | OR5M1 | IGSF6 | LOC100144604 | LOXL4 |
| PRKCE | OR5B21 | OR5B21 | OR5B21 | TAS2R3 | OR5M10 | IMPA2 | LOC100289673 | LRFN1 |
| MAPK13 | OR5B3 | | | TAS2R8 | OR5M11 | INPP5K | LINC00629 | LRMP |
| PROC | OR5C1 | OR5C1 | | TAS2R14 | OR5M3 | IRF2BPL | LOC148413 | LRRC43 |
| PRSS8 | OR5D13 | OR5D13 | OR5D13 | FAHD2A | OR5M8 | ISG20 | RPL34-AS1 | LTBP1 |
| KLK6 | OR5D14 | OR5D14 | | CALHM2 | OR5M9 | ITPKC | C2orf91 | LUZP1 |
| TAS2R38 | OR5D16 | OR5D16 | | TMX2 | OR5P2 | IVNS1ABP | LOC439990 | LYPD6B |
| PTPN3 | OR5D18 | OR5D18 | OR5D18 | MRTO4 | OR5P3 | JHDM1D | LIMD1-AS1 | LZTS1 |
| PEX19 | OR5E1P | OR5E1P | OR5E1P | ZDHHC2 | OR5R1 | JMJD1C | LOC645206 | MAD1L1 |
| RAB3B | OR5F1 | OR5F1 | OR5F1 | MZB1 | OR5T1 | JPH4 | FAM227A | MAFB |
| RAB27B | OR5H1 | OR5H1 | OR5H1 | MRPL27 | OR5T2 | JUNB | LOC650368 | MAGEA6 |
| RAC3 | OR5H14 | OR5H14 | OR5H14 | COA4 | OR5T3 | JUP | LRFN3 | MAML2 |
| RARRES1 | OR5H15 | OR5H15 | OR5H15 | GMPR2 | OR5V1 | KAT2B | LRP3 | MANBAL |
| RBP1 | OR5H2 | OR5H2 | OR5H2 | FKBP11 | OR5W2 | KATNB1 | LRRC16A | MAOB |
| RCN1 | OR5H6 | OR5H6 | OR5H6 | PCYOX1 | OR6A2 | KCTD13 | LRRC30 | MAP6 |
| RCVRN | OR5I1 | OR5I1 | OR5I1 | UFC1 | OR6B1 | KDM2A | LRRC57 | MAPK8IP3 |
| SNORA62 | OR5J2 | OR5J2 | OR5J2 | LARS | OR6B2 | KDM4B | LRRC61 | MAR2 |
| SNORD15A | OR5K1 | OR5K1 | | TRIM33 | OR6B3 | KDM5A | LRRC8B | MARK1 |
| RPS26 | OR5K2 | OR5K2 | | TAS2R5 | OR6C1 | KEAP1 | LSM2 | MCOLN3 |
| RSC1A1 | OR5K3 | OR5K3 | OR5K3 | ANKRD16 | OR6C3 | KIAA0317 | LSM5 | MEG8 |
| S100A1 | OR5K4 | OR5K4 | OR5K4 | NDFIP2 | OR6C4 | KIAA1737 | LY6G6F | NTMT1 |
| S100A5 | OR5L1 | OR5L1 | OR5L1 | HES2 | OR6C68 | KIF1C | MAFK | METTL18 |
| SCD | OR5L2 | OR5L2 | | RAB39A | OR6C70 | KIF9 | MAML3 | METTL23 |

**[Table 7-9]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| SCNN1B | OR5M1 | OR5M1 | OR5M1 | KCTD9 | OR6C74 | KLF16 | MATR3 | METTL4 |
| CCL11 | OR5M10 | OR5M10 | OR5M10 | KLHL24 | OR6C75 | KLF5 | MBIP | MFSD2B |
| SH3GL1P1 | OR5M11 | OR5M11 | OR5M11 | HAUS4 | OR6C76 | KLK12 | MBTPS2 | MICB |
| SH3GL1P2 | OR5M3 | OR5M3 | OR5M3 | COMMD4 | OR6F1 | KRAS | MCAT | MIR181D |
| SHMT1 | OR5M8 | OR5M8 | OR5M8 | UCKL1 | OR6K2 | KRT17 | MDC1 | MOGS |
| SLC6A8 | OR5M9 | OR5M9 | OR5M9 | PIGX | OR6K3 | KRT79 | MDN1 | MPPE1 |
| SLC6A11 | OR5P2 | OR5P2 | OR5P2 | SLC25A38 | OR6K6 | LACRT | METTL18 | MRE11A |
| SLC18A3 | OR5P3 | OR5P3 | OR5P3 | HERC6 | OR6M1 | LAMTOR1 | MFGE8 | MRPL11 |
| SLPI | OR5R1 | OR5R1 | | C14orf119 | OR6N1 | LCLAT1 | MIA | MRPL37 |
| SMS | OR5T1 | OR5T1 | OR5T1 | RBM23 | OR6N2 | LCMT1 | MICALL2 | MSC |
| SOD3 | OR5T2 | OR5T2 | | MSTO1 | OR6P1 | LCP2 | MIS18A | MT1X |
| SOX1 | OR5T3 | OR5T3 | OR5T3 | ARMC1 | OR6Q1 | LDHD | MKI67 | MT3 |
| SOX2 | OR5V1 | OR5V1 | | C7orf43 | OR6S1 | LGALS2 | MOGAT2 | MTMR1 |
| SOX4 | OR5W2 | OR5W2 | OR5W2 | NUDT15 | OR6T1 | LIMD2 | MON1B | MUS81 |
| SOX11 | OR6A2 | OR6A2 | | UEVLD | OR6V1 | LIMK1 | MPP7 | MYEOV2 |
| SPAG4 | OR6B1 | OR6B1 | OR6B1 | ABCF3 | OR6W1P | LINC00467 | MPZ | MYO6 |
| SPRR1A | OR6B2 | OR6B2 | OR6B2 | AGPAT5 | OR6X1 | LIPH | MPZL3 | NAP1L2 |
| SPRR2C | OR6B3 | OR6B3 | OR6B3 | TBC1D2 | OR6Y1 | LMAN2 | MRFAP1L1 | NAV3 |
| SPRR2D | OR6C1 | OR6C1 | OR6C1 | STYK1 | OR7A10 | LMBR1L | MRPL10 | NCEH1 |
| SPRR2E | OR6C2 | OR6C2 | OR6C2 | PLXNA3 | OR7A17 | LOC100287177 | MRPL14 | NCS1 |
| SPRR2G | OR6C3 | OR6C3 | OR6C3 | FAM46A | OR7A5 | LOC100505795 | MRPL17 | NDN |
| SSTR4 | OR6C4 | OR6C4 | | SMPD4 | OR7C1 | LOC100505839 | MRPL22 | NDUFA12 |
| STATH | OR6C6 | OR6C6 | OR6C6 | IWS1 | OR7C2 | LINC00641 | MRPL34 | NDUFA3 |
| SULT1E1 | OR6C65 | | | MBNL3 | OR7D4 | LOC283856 | MRPS2 | NDUFS1 |
| ELOVL4 | OR6C68 | OR6C68 | OR6C68 | SCN3B | OR7E37P | LOC731275 | MS4A12 | NDUFV2 |
| AURKAPS1 | OR6C70 | OR6C70 | OR6C70 | LENEP | OR7E5P | LPCAT2 | MS4A14 | NEU2 |
| SULT1A1 | OR6C74 | OR6C74 | OR6C74 | DMAP1 | OR7E91P | LPIN3 | MT1DP | NKTR |
| SYT5 | OR6C75 | OR6C75 | OR6C75 | PCDHB15 | OR7G1 | LRP10 | MUL1 | NLRP1 |
| TAPBP | OR6C76 | OR6C76 | OR6C76 | PCDHB13 | OR7G2 | LRRC57 | MYCT1 | NMT2 |
| TBX6 | OR6F1 | OR6F1 | OR6F1 | PCDHB12 | OR7G3 | LYN | MYH11 | NPBWR1 |
| TBX3 | OR6K2 | OR6K2 | OR6K2 | PCDHB10 | OR8A1 | MAP2K2 | MYOM3 | NPBWR2 |
| TDGF1P3 | OR6K3 | OR6K3 | OR6K3 | PCDHB7 | OR8B12 | MAP2K7 | NAPEPLD | NPFFR1 |
| TEAD3 | OR6K6 | OR6K6 | OR6K6 | MDM1 | OR8B2 | MAP7D1 | NARG2 | NRG2 |
| TERC | OR6M1 | OR6M1 | OR6M1 | XAB2 | OR8B3 | MAPK1IP1L | NAT10 | NSUN5P2 |
| TGFB2 | OR6N1 | OR6N1 | OR6N1 | CHPT1 | OR8B4 | MAPK6 | NCCRP1 | OAZ2 |
| THBS2 | OR6N2 | OR6N2 | | UTP3 | OR8B8 | MAST3 | NDRG4 | OGDH |

**[Table 7-10]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| TCHH | OR6P1 | OR6P1 | OR6P1 | LYRM4 | OR8D1 | SLC25A51 | NDUFA3 | OPN1SW |
| TJP1 | OR6Q1 | OR6Q1 | | ATP8B2 | OR8D2 | MCC | NDUFC1 | OR10G9 |
| TLE2 | OR6S1 | OR6S1 | OR6S1 | THAP11 | OR8D4 | MCM3AP | NDUFS3 | OR10J5 |
| TSPAN8 | OR6T1 | OR6T1 | OR6T1 | CBX8 | OR8G1 | MCM7 | NDUFV3 | OR11L1 |
| TOP1P1 | OR6V1 | OR6V1 | OR6V1 | USP31 | OR8G2 | MEA1 | NEDD1 | OR1F2P |
| TOP1P2 | OR6W1P | OR6W1P | OR6W1P | NLN | OR8H1 | MED21 | NEFL | OR1N2 |
| TSPY1 | OR6X1 | OR6X1 | OR6X1 | GBA2 | OR8H2 | MEF2D | NEK1 | OR2A2 |
| TSPYL1 | OR6Y1 | OR6Y1 | OR6Y1 | RBAK | OR8H3 | METRNL | NEU4 | OR2J3 |
| TYR | OR7A10 | OR7A10 | OR7A10 | C6orf47 | OR8I2 | METTL4 | NFKBIL1 | OR2L3 |
| TYRO3P | OR7A17 | OR7A17 | OR7A17 | PCTP | OR8J1 | MGC16121 | NFS1 | OR2T6 |
| UCN | OR7A5 | OR7A5 | | RPRD1B | OR8J3 | MGEA5 | NGDN | OR4N2 |
| VEGFC | OR7C1 | OR7C1 | OR7C1 | TRMT11 | OR8K1 | MGST2 | NGLY1 | OR51L1 |
| WNT7B | OR7C2 | OR7C2 | | RINT1 | OR8K3 | MKNK2 | NIN | OR52A5 |
| ZNF19 | OR7D4 | OR7D4 | OR7D4 | SNX16 | OR8U1 | MLF2 | NKPD1 | OR52K1 |
| ZNF32 | OR7E24 | | | DIO3OS | OR9A2 | MLPH | NMNAT1 | OR56A1 |
| ZNF45 | OR7E37P | OR7E37P | | OSGEPL1 | OR9A4 | MMP13 | NOL7 | OR5AP2 |
| ZNF80 | OR7E91P | OR7E91P | OR7E91P | STRA6 | OR9G1 | MOGS | NOP14 | OR5B12 |
| MKRN3 | OR7G1 | OR7G1 | OR7G1 | LMBR1 | OR9G4 | MOXD1 | NQO1 | OR5H1 |
| MKRN7P | OR7G2 | OR7G2 | | GREM2 | OR9I1 | MRE11A | NR2E3 | OR5P2 |
| ZNF154 | OR7G3 | OR7G3 | | TPSB2 | OR9K2 | MRPL14 | NSFL1C | OR6C74 |
| ZNF177 | OR8A1 | OR8A1 | OR8A1 | CTAGE1 | OR9Q2 | MRPL23 | NTSR1 | OR8K1 |
| RNF113A | OR8B12 | OR8B12 | OR8B12 | DEPTOR | | MRPL49 | NUP107 | OSBPL9 |
| ZNF224 | OR8B2 | OR8B2 | OR8B2 | NT5DC2 | | MRPL51 | NUP54 | OSCP1 |
| ZXDA | OR8B4 | OR8B4 | | MRPS11 | | MSGN1 | NWD1 | OTOP2 |
| OR2H2 | OR8B8 | OR8B8 | OR8B8 | PLEKHA3 | | MTMR10 | OCEL1 | OTUD7B |
| TUSC3 | OR8D1 | OR8D1 | | YIPF2 | | MTOR | ODF2L | OVOL1 |
| HMGA2 | OR8D2 | OR8D2 | OR8D2 | FASTKD3 | | MYCBP2 | OGFR | P2RX1 |
| COIL | | OR8D4 | | DSCC1 | | MYH10 | OPA3 | PA2G4 |
| HIST3H3 | OR8G1 | OR8G1 | OR8G1 | ZNF426 | | MYH11 | OPRL1 | PACRGL |
| HIST1H4I | OR8G2 | OR8G2 | OR8G2 | TRAPPC6A | | MYH2 | OR10A3 | PAM |
| FZD7 | OR8G5 | OR8G5 | OR8G5 | LILRP2 | | N4BP1 | OR13C5 | PAPD4 |
| FZD8 | OR8H1 | OR8H1 | OR8H1 | RBM42 | | NAA30 | OR7E24 | PAQR7 |
| FZD9 | OR8H2 | OR8H2 | OR8H2 | IRX6 | | NACC1 | ORC5 | PCDH20 |
| HIST1H2AK | OR8H3 | OR8H3 | OR8H3 | DLEU2L | | NAPRT1 | OVCA2 | PCDHB7 |
| HIST1H2AJ | OR8I2 | OR8I2 | OR8I2 | OR2A4 | | NCCRP1 | P2RY12 | PCDHGB8P |
| HIST1H2AL | OR8J1 | OR8J1 | OR8J1 | OR4K1 | | NCOR2 | PACSIN3 | PCMTD2 |

**[Table 7-11]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| HIST1H2AB | OR8J3 | OR8J3 | OR8J3 | C10orf76 | | NDEL1 | PAK2 | PDCD1 |
| HIST1H2BG | OR8K1 | OR8K1 | OR8K1 | HSPBAP1 | | NDUFA11 | PANX2 | PDZD4 |
| HIST1H2BL | OR8K3 | OR8K3 | OR8K3 | SMC6 | | NDUFAF1 | PCNT | PEBP1 |
| HIST1H2BM | OR8K5 | OR8K5 | OR8K5 | ZDHHC14 | | NDUFS1 | PCSK7 | PFDN2 |
| HIST1H2BF | OR8S1 | OR8S1 | OR8S1 | IPO4 | | NDUFS4 | PDE5A | PFKFB1 |
| HIST1H2BE | OR8U1 | OR8U1 | OR8U1 | ELMO3 | | NDUFS7 | PDZD2 | PGM5 |
| HIST1H2BI | OR9A2 | OR9A2 | OR9A2 | CCDC82 | | NFE2L2 | PET117 | PGPEP1 |
| HIST1H2BO | OR9A4 | OR9A4 | OR9A4 | CLMN | | NFKBIE | PFDN1 | PHOSPHO1 |
| HIST1H3D | OR9G1 | OR9G1 | OR9G1 | MAGIX | | NIF3L1 | PFDN4 | PI4KB |
| HIST1H3E | OR9G4 | OR9G4 | OR9G4 | MAP6D1 | | NKAP | PFDN5 | PIEZO1 |
| HIST1H3J | OR9I1 | OR9I1 | OR9I1 | FAM106A | | NKTR | PHC2 | PIK3R1 |
| HIST1H3H | OR9K2 | OR9K2 | OR9K2 | FBXO11 | | NLRP12 | PHF21B | PIP5K1B |
| HIST1H4K | OR9Q1 | OR9Q1 | | ELL3 | | NMT1 | PHPT1 | PKIB |
| HIST1H4J | OR9Q2 | OR9Q2 | | C16orf70 | | NPC1 | PKP2 | PLEKHA7 |
| OR1A1 | | | | FER1L4 | | NPIP | PLA2G4A | PLEKHB2 |
| OR1D5 | | | | HCG4B | | NRADDP | PLP2 | PLEKHG2 |
| OR1E2 | | | | OR4F17 | | NUAK2 | PNO1 | PLEKHM1 |
| OR1G1 | | | | OR51G2 | | NUDT18 | POLA1 | PLOD1 |
| OR3A3 | | | | OR4A16 | | NUFIP2 | POLK | PLP2 |
| PLA2G6 | | | | OR6N2 | | NUP210L | POLR3F | PNKD |
| SOX14 | | | | OR2G3 | | NUP88 | POLR3GL | PNLIPRP3 |
| ANXA9 | | | | MIR600HG | | NUPL1 | PPAPDC1B | POLR3F |
| BCAS1 | | | | FAM83D | | NXPH3 | PPIF | POLR3GL |
| PPFIA3 | | | | KAZALD1 | | O3FAR1 | PPP1R17 | POR |
| MADD | | | | TRMT1L | | NABP2 | PPP1R1C | POTEC |
| OR6A2 | | | | ISCA1 | | ODF3B | PRDM10 | PP14571 |
| DCHS1 | | | | PPP1R14C | | OR10G3 | PREX2 | PPM1M |
| JRKL | | | | ADAMTS10 | | OR1B1 | PRKD1 | PPP1R16B |
| GALNT4 | | | | KRTAP4-6 | | OR1M1 | PRKRIP1 | PPP2R1A |
| B3GALT4 | | | | PLVAP | | OR2H2 | PRM2 | PRMT5 |
| ADAM1A | | | | RBM4B | | OR2T33 | PSKH1 | PROC |
| SCEL | | | | USP26 | | OR6C75 | PTPMT1 | PRSS22 |
| PEX11A | | | | ANGPTL6 | | OSBPL10 | PUS3 | PSMB7 |
| SAP30 | | | | RSPH3 | | OSBPL8 | PUS7L | PSMC4 |
| INPP4B | | | | TBC1D10A | | OTUB1 | QPCTL | PSMD7 |
| FGF16 | | | | BCO2 | | OXSR1 | RAB22A | PSMD8 |

**[Table 7-12]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| ALDH1A2 | | | | KRTAP9-3 | | PADI1 | RAB2B | PSMF1 |
| IER3 | | | | NACAP1 | | PAK1 | RAB9A | PTF1A |
| VNN2 | | | | RACGAP1P | | PAPL | RAP1GAP | PTPLA |
| ENDOU | | | | FRMD8P1 | | PARP15 | RAP1GDS1 | PUSL1 |
| BAIAP3 | | | | CCDC8 | | PATL1 | RASSF9 | QTRTD1 |
| CDK5R2 | | | | TKTL2 | | PCGF3 | RBM34 | R3HDM1 |
| HIST1H2BJ | | | | TMEM164 | | PCIF1 | RHOU | RAB23 |
| HSPB3 | | | | TCHP | | PELI1 | RIF1 | RAB34 |
| SELENBP1 | | | | PSMG3 | | PELP1 | RIOK1 | RAB5B |
| MPZL1 | | | | HAGHL | | PFKP | RIPK2 | RAD51B |
| CH25H | | | | COQ5 | | CPQ | RNF146 | RAP2B |
| TAAR5 | | | | CAPNS2 | | PHLDA3 | ROBO1 | RARRES1 |
| SYT7 | | | | FAM213A | | PHPT1 | RPAP3 | RBFOX2 |
| CLDN8 | | | | MIEN1 | | PI4K2B | RPL34 | RBM25 |
| CLDN9 | | | | THOC3 | | PIAS1 | RPS7 | RBPMS2 |
| KRT75 | | | | KRTAP4-4 | | PIK3CB | RRAGB | RCN2 |
| P2RX6 | | | | ZNF469 | | PIK3CD | RSF1 | REEP6 |
| ZBED1 | | | | MYO18B | | PIK3R1 | RUFY2 | RIIAD1 |
| ZBED1 | | | | GPT2 | | PIM2 | SAG | RIMBP3C |
| TIAF1 | | | | FOXD2-AS1 | | PLA2G2F | SAP30L | RIMS4 |
| LRAT | | | | FAM136A | | PLA2G4D | SAPCD1 | RNASE7 |
| CRLF1 | | | | SNHG7 | | PLA2G7 | SATB1 | RNF103 |
| ATP6V1F | | | | FAM83A | | PLAC2 | SEC14L2 | RNF144A |
| NREP | | | | MGC16025 | | PLCD3 | SEC23A | RNPEP |
| HMGN3 | | | | DGCR6L | | PLEKHM1P | SENP6 | ROPN1 |
| SLC9A3R2 | | | | MYLK2 | | PLIN5 | SERINC2 | ROPN1L |
| SLC22A13 | | | | ZCRB1 | | PLP2 | SERPINH1 | RPL18 |
| CDY2A | | | | TANC1 | | PMVK | SEZ6 | RPL32 |
| GDF15 | | | | MBD3L1 | | PNMA1 | SGMS1 | RPS27L |
| NR1D1 | | | | GALP | | POLG | SHOC2 | RPS6 |
| RIN1 | | | | TRIM4 | | POLR3D | SHPK | S100A1 |
| GDA | | | | HPS4 | | PPBPP2 | SHROOM4 | S100A16 |
| KLK4 | | | | KIAA2013 | | PPP1CA | SIGLEC9 | S100P |
| CLCA2 | | | | COX19 | | PPP1R14A | SLC10A3 | SAMD3 |
| IQCB1 | | | | PPP1R3E | | PPP1R15B | SLC12A3 | SCMH1 |
| USP6NL | | | | BTF3L4 | | PPP2R1A | SLC16A11 | SCN5A |

**[Table 7-13]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| ZNF623 | | | | ZNF830 | | PPP6R1 | SLC22A13 | SDC4 |
| LCMT2 | | | | TTC30A | | PPP6R2 | SLC22A31 | SDHAF1 |
| EPM2AIP1 | | | | HIST3H2A | | DESI2 | SLC24A1 | SDR42E1 |
| TRIL | | | | PGAP3 | | PQLC1 | SLC25A16 | SDR9C7 |
| ARNT2 | | | | HAUS8 | | PRKCSH | SLC25A35 | SEC14L5 |
| XYLB | | | | POM121L2 | | PRSS22 | SLC26A9 | SEC24C |
| ABCB6 | | | | TP531NP1 | | PRSS27 | SLC34A1 | SELK |
| TSPAN5 | | | | GLB1L3 | | PSAPL1 | SLC4A1 | SEMA5A |
| TSPAN1 | | | | PLCD3 | | PSD4 | SLC5A6 | SERPINA1 |
| KIF20A | | | | CHST14 | | PSMA5 | SLC7A4 | SESN2 |
| HNRNPA3P1 | | | | MFSD3 | | PSMB1 | SLC9A2 | SETD1B |
| LPAR6 | | | | OSBPL5 | | PSMB6 | SLX4 | SF3A2 |
| NBR2 | | | | FBXO32 | | PSMD3 | SNRPD3 | SGSH |
| SPRY3 | | | | EVI5L | | PSMG3 | SNX6 | SHFM1 |
| SPRY3 | | | | THEM4 | | PTEN | SOCS4 | SIK3 |
| CNKSR1 | | | | RAB3IP | | PTER | SOX17 | SKP1 |
| FSTL3 | | | | FTSJ3 | | PTGES2 | SOX2 | SLC17A8 |
| PKDREJ | | | | COMTD1 | | PTOV1 | SPATA5 | SLC19A1 |
| SPON2 | | | | NUDT9P1 | | PTPRA | SPATA6 | SLC25A2 |
| LYPLA1 | | | | CPXM2 | | PTPRK | SPNS2 | SLC25A39 |
| RNASEH2A | | | | OR52E2 | | PWWP2A | SPTY2D1 | SLC2A4 |
| AGR2 | | | | OR52J3 | | R3HDM2 | SSNA1 | SLC32A1 |
| TACC2 | | | | OR4X2 | | RAB10 | ST6GALNAC4 | SLC38A5 |
| MAB21L2 | | | | SLC36A4 | | RAB21 | ST8SIA4 | SLC4A11 |
| TXNRD2 | | | | OR2D3 | | RAB32 | STK19 | SLC4A3 |
| LEFTY1 | | | | OR52W1 | | RAB5A | STOX1 | SLC6A8 |
| SPHAR | | | | ERP27 | | RALY | STX4 | SLC7A2 |
| NPRL2 | | | | ASCL4 | | RANGAP1 | TACR3 | SLFN12 |
| CELF1 | | | | SPIC | | RAPGEF6 | TADA1 | SLMO2 |
| CERS1 | | | | C14orf28 | | RASA2 | TAF5L | SMARCAL1 |
| RFPL3 | | | | OR11H6 | | RASA4 | TBCA | SMEK3P |
| PTTG2 | | | | NTAN1 | | RASAL1 | TBCB | SMYD2 |
| OR5I1 | | | | PRSS30P | | RBFA | TCTN1 | SND1 |
| ACTL7B | | | | GPR139 | | RBM10 | TECR | SNORA79 |
| ACTL7A | | | | METTL23 | | RBM27 | THAP3 | SNX25 |
| PPBPP2 | | | | CD300LB | | RBMXL1 | THG1L | SOX12 |

**[Table 7-14]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| OCLM | | | | AFMID | | RBPJ | THUMPD1 | SOX21 |
| RUNDC3A | | | | OR1I1 | | RCE1 | TIE1 | SPATA25 |
| MTMR11 | | | | TCHHL1 | | RER1 | TIMM8A | SPINT1 |
| MORF4L1 | | | | OR2M5 | | RFNG | TMED10P1 | SPRYD7 |
| KCNQ1OT1 | | | | PODN | | RFXANK | TMED3 | ST3GAL1 |
| SPINK5 | | | | HSD3BP4 | | RGS19 | TMEM25 | STARD9 |
| KLK11 | | | | TMEM125 | | RHOQ | TMEM39B | STK11IP |
| KDELR3 | | | | OR6N1 | | RHOT2 | TMEM72 | SUGP1 |
| IFT27 | | | | HMGB3P1 | | RIMBP3 | TMOD1 | SULT2B1 |
| RBPMS | | | | TAAR9 | | RNF11 | TOP2A | SUOX |
| ADAM30 | | | | OR9A2 | | RNF216 | TPH2 | SURF2 |
| ADAM29 | | | | OR13C8 | | RNF39 | TPST1 | SYT1 |
| PRSS23 | | | | OR1L8 | | RNPEP | TRA2B | SYT15 |
| PRDM5 | | | | GAB3 | | RNPEPL1 | TRIM59 | TAAR3 |
| HIBADH | | | | KRTAP13-1 | | RORC | TRIP11 | TAPBPL |
| AP4S1 | | | | PISRT1 | | RPAP3 | TRMT1 | TAS2R3 |
| SOX21 | | | | ITLN2 | | RPL18A | TSN | TAS2R43 |
| LZTS1 | | | | LOC143666 | | RPS4Y1 | TSR2 | TAS2R5 |
| PTENP1 | | | | SPTY2D1 | | RRAGD | EMC2 | TAS2R9 |
| FZD10 | | | | OR2AG1 | | RSL24D1 | TUBG2 | TBC1D8 |
| NXPH4 | | | | ZNF664 | | RWDD1 | U2SURP | TCEB3C |
| GPR45 | | | | PGPEP1L | | RXFP4 | UBA52 | TCIRG1 |
| TREX1 | | | | BEAN1 | | S100A14 | UBE2E1 | TEAD4 |
| FRMPD1 | | | | KRT25 | | S100A16 | UBP1 | TEX2 |
| DOLK | | | | C19orf18 | | SAMD15 | UBXN4 | TFCP2 |
| ZNF507 | | | | ZNRF4 | | SAMD9L | URGCP | TGIF1 |
| ZNF510 | | | | EXOC8 | | SAR1B | USP14 | TGM1 |
| PLEKHA6 | | | | CCDC17 | | SBNO1 | USP33 | TIAM1 |
| SHANK2 | | | | CCT8L2 | | SCAF1 | VAPB | TIMM10 |
| RIMS1 | | | | CCDC117 | | SCNN1A | VAT1 | TIMP1 |
| FAIM2 | | | | SLC16A14 | | SCYL1 | VDAC3 | TINAGL1 |
| PDZRN3 | | | | LBX2-AS1 | | SDCBP2 | VPS28 | TM7SF3 |
| ENDOD1 | | | | LRRC34 | | SDF2L1 | VPS39 | TM9SF4 |
| ARHGAP26 | | | | DAB2IP | | SEC14L6 | WASF1 | TMCO6 |
| ATP10B | | | | CLEC14A | | SEC24B | WASH5P | TMEM139 |
| FRMD4B | | | | PGBD4 | | SEC31A | WDR47 | TMEM164 |

**[Table 7-15]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| NUP205 | | | | ITPRIPL2 | | SEC62 | WDR82 | TMEM180 |
| RRS1 | | | | TMEM92 | | SEMA7A | WIPF2 | TMEM213 |
| TSPYL4 | | | | ZNF383 | | SEPT5 | WWOX | TMEM216 |
| NEDD4L | | | | PRR22 | | SEPW1 | XRCC1 | TMEM229B |
| RRP8 | | | | TTLL9 | | MSRB1 | YEATS2 | TMEM237 |
| GCAT | | | | TIGD2 | | SERAC1 | YEATS4 | TMEM31 |
| CBX7 | | | | RNF133 | | SESN1 | YPEL3 | TMEM38B |
| HEY2 | | | | GPHA2 | | SF3A2 | ZBED5 | TMEM79 |
| ANP32D | | | | LCTL | | SFN | ZBTB46 | TMIE |
| ANP32C | | | | IBA57 | | SFXN1 | ZDHHC3 | TMPRSS11E |
| OR52A1 | | | | RNF215 | | SFXN3 | ZDHHC4 | TNFRSF11B |
| SEC14L2 | | | | OR8U1 | | SGK2 | ZNF121 | TNS1 |
| MAPK8IP2 | | | | OR4C6 | | SGTA | ZNF132 | TOX2 |
| RASD2 | | | | OR8J1 | | SH3BGRL | ZNF192 | TPK1 |
| WBP1 | | | | OR6T1 | | SH3BGRL3 | ZNF197 | TPRN |
| PRG1 | | | | OR5B21 | | SH3BP1 | ZNF277 | TPT1 |
| VSIG2 | | | | OR4D11 | | SH3BP4 | ZNF280C | TRAPPC2L |
| FAM215A | | | | ATOH7 | | SH3GL1 | ZNF383 | TRAPPC3 |
| BRD7P3 | | | | CCDC89 | | SIK1 | ZNF436 | TREML1 |
| LDOC1 | | | | CTAGE10P | | SIRT7 | ZNF441 | TREX2 |
| SSBP3 | | | | LEMD2 | | SLAMF7 | ZNF473 | TRIM11 |
| GSPT2 | | | | ZBTB9 | | SLC12A3 | ZNF599 | TRIM16L |
| OSBP2 | | | | HIST1H2AA | | SLC15A4 | ZNF615 | TRIM31 |
| FJX1 | | | | PRPS1L1 | | SLC16A13 | ZNF695 | TRIT1 |
| SHC2 | | | | BRAT1 | | SLC17A1 | ZNF703 | TSHZ2 |
| DGCR11 | | | | RPL23P8 | | SLC19A1 | ZSCAN22 | TSNAX |
| DGCR9 | | | | CNPY4 | | SLC22A3 | ZSCAN29 | TSPY26P |
| SPDEF | | | | SNX32 | | SLC25A15 | LOC100131067 | TSPYL6 |
| KLK5 | | | | LPCAT4 | | SLC25A28 | LOC100505474 | TSSK6 |
| ABTB2 | | | | HIST1H2BA | | SLC26A9 | PMS2P5 | TTC33 |
| WWTR1 | | | | OR4C3 | | SLC28A3 | NAV2 | TUBBP5 |
| TPGS2 | | | | KANK3 | | SLC38A6 | C21orf88 | TUBGCP3 |
| KANK2 | | | | RNF214 | | SLC39A4 | C7orf55 | UBE2E2 |
| SNED1 | | | | ASPM | | SLC6A8 | EGOT | UBL4B |
| L3MBTL1 | | | | TAS2R39 | | SLC9A8 | FLJ31306 | UGGT1 |
| TMEM98 | | | | TAS2R30 | | SMAGP | GMNN | UNC13D |

**[Table 7-16]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| RAI14 | | | | TAS2R20 | | SMPD3 | HIST1H2AI | UNC93A |
| DKFZP434H168 | | | | BEST4 | | SNRPC | HTA | UPK1B |
| WIPI2 | | | | MAGEA2B | | SNURF | IPCEF1 | UPK3A |
| IRF2BP1 | | | | STK32C | | SNX20 | LINC00263 | VARS2 |
| OR1F2P | | | | OR4C13 | | SOD2 | LINC00277 | VWA1 |
| OR1C1 | | | | OR9G4 | | SOX3 | LOC100132273 | WBP2 |
| OR1A2 | | | | INO80E | | SPATA2L | LOC100506050 | WBSCR16 |
| OR2F1 | | | | LINC00324 | | SPNS2 | LOC338799 | WIZ |
| OR2B6 | | | | FLJ36000 | | SPSB1 | LOC340017 | WNT10B |
| OR1J4 | | | | VSTM1 | | SPSB3 | LOC401109 | WNT7A |
| DGCR10 | | | | ZNF493 | | SRP14 | STXBP5-AS1 | WWC2 |
| FBXW4P1 | | | | LOC284578 | | SRSF1 | NME2 | ZBTB1 |
| OR2L1P | | | | KRTAP13-4 | | SRSF11 | SYNGR1 | ZDHHC8P1 |
| OR2K2 | | | | OR2V2 | | SRSF3 | TTTY17B | ZDHHC9 |
| PTTG3P | | | | HLA-F-AS1 | | SS18L1 | | ZFP82 |
| FBXW8 | | | | TTC3P1 | | ST6GALNAC4 | | ZNF219 |
| TSPAN17 | | | | TAAR6 | | STK19 | | ZNF252P |
| CLDN17 | | | | KRTAP8-1 | | STK3 | | ZNF442 |
| OR7A17 | | | | KRTAP13-2 | | STK40 | | ZNF490 |
| OR5L2 | | | | KRTAP23-1 | | STOML1 | | ZNF582 |
| OR5K1 | | | | OR5AP2 | | SUGP2 | | ZNF598 |
| OR5H1 | | | | OR2AG2 | | SULT2B1 | | ZNF703 |
| OR5E1P | | | | KLHL17 | | SUN2 | | ZNF704 |
| NUPR1 | | | | FAM58BP | | SUV420H1 | | ZNF721 |
| OR10J1 | | | | ACER2 | | SYTL1 | | ZNF737 |
| OR8B8 | | | | OR2W3 | | TAB2 | | ZNF74 |
| OR10A3 | | | | OR2T3 | | TAF7 | | ZNF750 |
| OR10H3 | | | | ACTBL2 | | TAGLN3 | | ZNF778 |
| OR10G3 | | | | QRFP | | TANK | | ZNF841 |
| OR10G2 | | | | ECEL1P2 | | TAS1R3 | | ZRANB1 |
| OR10H2 | | | | SERINC2 | | TAX1BP3 | | LINC00282 |
| OR10H1 | | | | SKA2 | | TBC1D20 | | LOC100286922 |
| GREM1 | | | | LCE1A | | TBCD | | C17orf76-AS1 |
| OR8B2 | | | | KAAG1 | | TBRG1 | | CCDC169 |
| OR7A5 | | | | PCNAP1 | | TCF25 | | DPY19L1P1 |
| OR7C1 | | | | MRPL42P5 | | TECR | | FAM66A |

**[Table 7-17]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| OR4F4 | | | | DND1 | | THAP4 | | FLJ10038 |
| OR4F3 | | | | LOC375196 | | THAP6 | | GPRASP2 |
| OR4D1 | | | | KRTAP10-1 | | TIA1 | | KIAA1908 |
| OR2W1 | | | | KRTAP10-11 | | TIAL1 | | LOC100129034 |
| OR2T1 | | | | LINC00320 | | TIMM17B | | LOC100132832 |
| OR1L1 | | | | GLTPD2 | | TIMM50 | | LOC100216001 |
| OR1J2 | | | | C17orf82 | | TM7SF2 | | LOC144486 |
| SNORA66 | | | | OR6B2 | | TMC1 | | LOC149086 |
| SNORA74A | | | | GTF21RD2B | | TMEM117 | | LOC283663 |
| RANBP6 | | | | ZCCHC13 | | TMEM123 | | LOC644172 |
| FGF22 | | | | UBE2NL | | TMEM134 | | FUT8-AS1 |
| INPP5J | | | | OR51I2 | | TMEM141 | | LOC654342 |
| CPAMD8 | | | | OR52H1 | | TMEM179B | | LOC728024 |
| INTU | | | | OR56A3 | | TMEM185A | | MDS2 |
| INGX | | | | OR5D13 | | TMEM208 | | MRS2P2 |
| IL36A | | | | OR5D16 | | TMEM79 | | PCDHGA1 |
| MOCS3 | | | | OR8H3 | | TMOD3 | | PTCRA |
| BHLHE22 | | | | OR5M9 | | TMPRSS13 | | RPL32P3 |
| RPS6KA6 | | | | OR5M10 | | TNPO2 | | TRIM16 |
| TMEM97 | | | | KDM4E | | TOLLIP | | |
| CECR2 | | | | OR10G7 | | TOP1 | | |
| SLCO4A1 | | | | OR6C75 | | TOX | | |
| DEXI | | | | OR6C70 | | TP531NP2 | | |
| SNX24 | | | | OR11G2 | | TPR | | |
| TMPRSS11E | | | | OR4M2 | | TRAPPC3 | | |
| MAGEH1 | | | | OR6K3 | | TRIM28 | | |
| COMMD5 | | | | OR11L1 | | TRMT2A | | |
| MRPL15 | | | | OR2AK2 | | TRPM7 | | |
| N6AMT1 | | | | OR2L3 | | TRPV3 | | |
| UHRF1 | | | | SUMO1P1 | | TSPAN17 | | |
| PACSIN3 | | | | USP17L6P | | TSPO | | |
| RBM15B | | | | HSP90AB2P | | TSR1 | | |
| PSMC3IP | | | | OR13C2 | | TSSC4 | | |
| PCDHB1 | | | | OR2A5 | | TST | | |
| LINC00312 | | | | MKRN9P | | TSTA3 | | |
| RPA4 | | | | CHCHD10 | | TUBGCP2 | | |

**[Table 7-18]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| DSE | | | | OR2A7 | | TUFM | | |
| PURG | | | | OR2A20P | | TWF1 | | |
| PADI1 | | | | OR51A4 | | UBAC1 | | |
| PSAT1 | | | | OR2T2 | | UBAP2L | | |
| SLC40A1 | | | | SRRD | | UBE2V2 | | |
| KLK12 | | | | RPL21P44 | | UBE2W | | |
| ARHGEF4 | | | | FAM92A1P2 | | UBR2 | | |
| TAS2R3 | | | | LINC00619 | | UBR4 | | |
| TAS2R4 | | | | DDI1 | | UBTD1 | | |
| TAS2R16 | | | | BLID | | UIMC1 | | |
| TAS2R8 | | | | KRTAP5-5 | | UNC119 | | |
| TAS2R13 | | | | H3F3C | | UNC5B | | |
| TAS2R10 | | | | MZT1 | | UNKL | | |
| TAS2R14 | | | | OR11H12 | | UPK3BL | | |
| PAM16 | | | | PRAMEF6 | | URI1 | | |
| BOLA1 | | | | GUSBP5 | | USP6NL | | |
| SH3GLB1 | | | | CRSP8P | | UTP23 | | |
| NDUFAF1 | | | | OR9K2 | | UTRN | | |
| LACTB2 | | | | WBP11P1 | | VAMP3 | | |
| MRPS2 | | | | ANKRD65 | | VASN | | |
| HSD17B14 | | | | OR2B3 | | VILL | | |
| DCXR | | | | UQCRBP1 | | VPS36 | | |
| GLTP | | | | IGIP | | VPS37C | | |
| TMEM216 | | | | LOC494127 | | VPS52 | | |
| TFDP3 | | | | ATXN7L3B | | WAC | | |
| UBAP1 | | | | LOC554206 | | WDTC1 | | |
| ZNF571 | | | | LOC574538 | | WHSC1 | | |
| RXFP3 | | | | SNORA52 | | WIPI2 | | |
| MS4A4A | | | | RPL31P11 | | WNT5A | | |
| PLA1A | | | | LOC641367 | | WWC3 | | |
| VGLL1 | | | | C15orf62 | | XKR8 | | |
| GULP1 | | | | BTBD18 | | XKRX | | |
| NAT8B | | | | LOC643387 | | YEATS2 | | |
| CXXC5 | | | | ZNF862 | | YIPF2 | | |
| GDE1 | | | | KRTAP27-1 | | ZCCHC17 | | |
| RBMX2 | | | | RPL13AP3 | | ZDHHC12 | | |

**[Table 7-19]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| PPIL1 | | | | LINC00520 | | ZDHHC23 | | |
| ZNF44 | | | | C6orf132 | | ZEB2 | | |
| RAB23 | | | | LOC648987 | | ZFP36L2 | | |
| TM6SF2 | | | | LOC650293 | | ZFYVE26 | | |
| GPR84 | | | | RPSAP9 | | ZNF174 | | |
| CDHR5 | | | | OR1D4 | | ZNF385A | | |
| CLDN22 | | | | ABCC6P1 | | ZNF561 | | |
| SETD4 | | | | HIST2H3D | | ZNF655 | | |
| KCNK10 | | | | OCM | | ZNF747 | | |
| DPM3 | | | | SNORA75 | | ZNF750 | | |
| TAS2R5 | | | | PCP4L1 | | ZNF862 | | |
| GAR1 | | | | SCARNA6 | | ZNRF4 | | |
| HCG4 | | | | SCARNA 16 | | ZRSR2 | | |
| SEMA5B | | | | FAM138D | | ZSWIM4 | | |
| NLE1 | | | | SNORA58 | | ZSWIM6 | | |
| TRMT13 | | | | SNORA79 | | SBDSP1 | | |
| ANKRD16 | | | | LOC728024 | | PPP1CC | | |
| ROBO4 | | | | MAGEA9B | | AIFM3 | | |
| RNF186 | | | | USP17L5 | | SOWAHC | | |
| SDK2 | | | | CNTNAP3B | | ATP5O | | |
| MAGEL2 | | | | SKP1P2 | | BAGE2 | | |
| WDR5B | | | | LOC728739 | | BCL2L2 | | |
| UGT1A10 | | | | OC90 | | FAM213A | | |
| UGT1A8 | | | | FAM166B | | CDK11A | | |
| LZTFL1 | | | | PWRN2 | | CLU | | |
| MANSC1 | | | | HAVCR1P1 | | FLJ39639 | | |
| GPN2 | | | | CTAGE4 | | KLHDC3 | | |
| ZNF586 | | | | LOC100128573 | | LETMD1 | | |
| SAMD9 | | | | LOC100129034 | | LOC100507331 | | |
| SYTL2 | | | | NRADDP | | LOC390705 | | |
| PAQR5 | | | | PSORS1C3 | | LOC440944 | | |
| PALMD | | | | TSTD3 | | SMG1P1 | | |
| DCAF16 | | | | LOC100131496 | | LOC728377 | | |
| RPP25 | | | | LOC100287042 | | SEC14L1P1 | | |
| RASIP1 | | | | LOC100288748 | | MALAT1 | | |
| HAUS4 | | | | MIR548I3 | | MAPKAPK3 | | |

**[Table 7-20]**

| (A) | (B) | (C) | (D) | (E) | (F) | (G) | (H) | (I) |
|---|---|---|---|---|---|---|---|---|
| Healthy vs AD | Healthy vs AD | Healthy vs AD mild | AD mild vs AD moderate | Healthy vs sensitive skin | Healthy vs sensitive skin | Redness-related molecule | Moisture content-related molecule | Sebum secretion-related molecule |
| 1911 genes | 370 genes | 368 genes | 284 genes | 693 genes | 344 genes | 703 genes | 553 genes | 594 genes |
| PARP16 | | | | LOC100303749 | | MGC72080 | | |
| PIGX | | | | NDUFAF4P1 | | NDUFA13 | | |
| SLC25A38 | | | | MTRNR2L2 | | NFAT5 | | |
| ZSCAN2 | | | | MTRNR2L4 | | NUDT3 | | |
| TRMT12 | | | | KRTAP16-1 | | PEX19 | | |
| ZCWPW1 | | | | GAS5-AS1 | | PI4KAP1 | | |
| EBLN2 | | | | TPBGL | | PLA2G4B | | |
| TRIM68 | | | | SNHG16 | | PMF1 | | |
| C19orf73 | | | | LOC100507634 | | RASA4CP | | |
| DZANK1 | | | | CAHM | | RPSAP58 | | |
| LGI2 | | | | | | SEPT7 | | |
| RAVER2 | | | | | | SERPINB4 | | |
| NAT10 | | | | | | SUZ12P1 | | |
| CCDC87 | | | | | | TBPL1 | | |
| TMEM40 | | | | | | TEN1 | | |
| BLOC1S4 | | | | | | TEX264 | | |
| YOD1 | | | | | | TNFSF12 | | |
| CAMK2N1 | | | | | | TSTD1 | | |
| RBM12B-AS1 | | | | | | ZNF37BP | | |
| TRPV6 | | | | | | ZNF564 | | |

**[Table 7-21]**

| (A) Healthy vs AD (1911 genes) | | | | | | | |
|---|---|---|---|---|---|---|---|
| MIOX | CD248 | HS3ST6 | ERMP1 | OR12D3 | ACTRT3 | TPD52L3 | GLCCI1 |
| SLC39A4 | RGL3 | IPPK | DENND2D | KRTAP4-6 | CNFN | OR6W1P | SLC52A3 |
| DEPDC1 | VN1R1 | EPS8L2 | GRHL2 | KRTAP2-1 | PARD6B | SAPCD2 | KLHDC7B |
| SYBU | ZNF304 | RMND5A | FAM106A | KIF18A | TTBK1 | CCDC120 | SIGLEC11 |
| SLC48A1 | AMIGO1 | LPIN3 | CEBPA-AS1 | ARHGAP24 | KISS1R | ZNF551 | MAL2 |
| BCAS4 | RIMKLB | MRPL36 | HEXA-AS1 | TAAR8 | GPR174 | ZNF616 | LMTK3 |
| CDC37L1 | MICAL3 | PINK1 | C3orf36 | SOX7 | IL1F10 | ZNF468 | SLITRK1 |
| H2AFJ | SEMA6A | PLEKHA3 | ARL14 | FAM167A | KIF2B | MCU | GNRHR2 |
| ZNF415 | ZNF471 | KCTD14 | LRRC8E | SLC35G5 | LCE3D | LYRM7 | FBXO32 |
| DDX28 | RANBP10 | DBNDD1 | FUZ | DYNLRB1 | EBPL | PPP1R3E | WDR31 |
| GOLGA6B | FBRSL1 | ELOVL6 | OPA3 | SESN2 | SPINK7 | SPOCD1 | VASN |
| ZMAT5 | TNRC6C | MLPH | TM2D3 | SH3BGRL2 | SPZ1 | CCDC149 | GPR146 |
| BAIAP2L1 | MAGEE1 | CHAC1 | C16orf70 | RBM4B | RNASE7 | CCDC34 | FCRL1 |
| PCDHGB8P | SEMA4G | C1orf116 | PPP1R2P9 | USP26 | GLIS2 | LOC91450 | ZNF689 |
| PCDHB15 | PCDHB16 | PPDPF | PNPLA3 | SLC25A2 | INSM2 | BOC | ZNF526 |
| PCDHB14 | KRTAP5-8 | FA2H | LY6G6C | KRTAP9-3 | CCDC54 | C11orf52 | TGM7 |
| PCDHB13 | SLC4A5 | PRR15L | LY6G5C | KRTAP9-8 | ABHD1 | TTC30A | FAM122A |
| PCDHB12 | PLEKHB1 | TTPAL | ITIH5 | KRTAP17-1 | PSRC1 | CHMP4C | TLCD1 |
| PCDHB11 | ZBED5 | BRCC3 | HCG4B | HDAC10 | PLEKHA8 | OTOP2 | OLIG1 |
| PCDHB10 | CREBZF | IRX3 | OR11H1 | TATDN1 | MGC2889 | HIST3H2A | MAS1L |
| PCDHB9 | ZNF77 | OR5H6 | OR4F17 | TSSK1B | TUBA1C | MARS2 | MRGPRD |
| PCDHB8 | OVOL2 | OR5H2 | OR4K15 | NACAP1 | MFSD9 | PEX11G | LRG1 |
| PCDHB6 | PRM3 | OR4K5 | OR8J3 | IFI27L2 | MGC12916 | HTR7P1 | THEM4 |
| PCDHB4 | IL22RA1 | OR51G1 | OR4P4 | TSSK6 | HPDL | PIGM | DCD |
| PCDHB3 | ALOXE3 | OR11H2 | OR4C15 | CCDC8 | C1orf198 | HAUS8 | MRGPRX4 |
| PCDHB2 | PBOV1 | OR51B2 | OR4A5 | PCDHB19P | FAM222A | CAPZA3 | MUCL1 |
| KIAA1217 | PPCDC | RSG1 | OR4A15 | ARMC2 | TIGD5 | POM121L2 | RPL29P2 |
| PARD3 | HPSE2 | ZBED2 | OR10W1 | FSCB | AJUBA | HTRA3 | GPR62 |
| IL36G | PROK2 | DLEU2L | OR2AE1 | TKTL2 | SNHG7 | FTMT | COMTD1 |
| ANKRD7 | PKNOX2 | OR52N1 | OR6K2 | KBTBD7 | COX14 | HSPB9 | NUDT9P1 |
| OR2S2 | OXCT2 | OR4F5 | OR2G3 | ENKD1 | MGC16025 | TMEM203 | GSTO2 |
| NDUFA4L2 | PERP | OR2A4 | OR2G2 | RAB6C | HIST1H2AH | ARHGAP12 | SFR1 |
| PAPOLB | C19orf33 | CCNJL | PTDSS2 | PCGF6 | KRTAP4-1 | CDRT15P1 | OR52E2 |
| THAP10 | CLSTN2 | NEIL1 | MIR600HG | PPP1R1B | KRTAP4-5 | FOXQ1 | OR51L1 |
| SMARCAD1 | FN3K | ZNF668 | PLA2G12A | TMEM191A | RIMBP3 | TP531NP1 | OR51A7 |
| RPL23AP32 | DIO3OS | ZMYM1 | ISCA1 | ATP13A4 | SLC45A3 | BIRC8 | OR51S1 |
| UTP3 | DPEP3 | LINC00115 | AMN | FBXW9 | RHPN2 | KRT71 | OR51F2 |
| HYMAI | ROBO3 | MAGIX | OR2B2 | CAPNS2 | ITPRIP | GLB1L3 | OR52R1 |
| PITHD1 | LINC00244 | SETD6 | LINC00597 | CHCHD6 | MBD3L1 | KTI12 | OR4C46 |
| CYSLTR2 | GPR135 | NANOG | PPP1R14C | TMEM107 | SERPINB12 | PLCD3 | OR4X2 |
| NAT14 | PLA2G2F | C10orf95 | ADAMTS10 | ZNF527 | SIGLEC10 | CHST14 | OR52M1 |

**[Table 7-22]**

| (A) Healthy vs AD (1911 genes) | | | | | | | |
|---|---|---|---|---|---|---|---|
| OR52K2 | KLHDC7A | ANKRD19P | C19orf18 | EID2 | OR5T2 | SLC29A4 | OR51V1 |
| OR5P2 | LINC00628 | OR13C5 | ZNF563 | ZNF100 | OR8H1 | AKR7A2P1 | OR8D1 |
| OR8I2 | HSD3BP4 | OR13C8 | IGFL2 | CITED4 | OR8K3 | STH | OR9G4 |
| OR2D3 | ARHGEF19 | OR13C3 | ZNRF4 | FAM43B | OR5M3 | MTVR2 | KCTD21 |
| OR2D2 | HIST3H2BB | OR13C4 | ZNF738 | UBL4B | OR5M8 | STXBP4 | OR10A4 |
| OR52W1 | OR10T2 | OR13F1 | ZNF569 | OXER1 | OR5M11 | SERHL2 | RPL13P5 |
| OR56A4 | OR6P1 | OR1L8 | TMEM56 | KBTBD12 | OR5AR1 | BPIFC | FGF14-AS2 |
| OR56A1 | OR10X1 | OR1N2 | NBPF4 | TIGD2 | OR5AK4P | PHYHD1 | LINC00346 |
| OR10P1 | OR10Z1 | OR1N1 | PM20D1 | DDX53 | YPEL4 | OR6C74 | C14orf178 |
| OR10AD1 | OR6K6 | VENTXP1 | GCSAML | RNF133 | HYLS1 | OR6C3 | OR4N4 |
| OR10A7 | OR6N1 | MAGEE2 | CCDC24 | CDC14C | OR8B12 | OR2T6 | PLA2G4D |
| HIST4H4 | BHLHE23 | ACTRT1 | FAM71A | BHLHA15 | OR8G5 | ZDHHC23 | GOLGA6L1 |
| ASCL4 | SIRPD | SPIN4 | GTSF1L | PER4 | OR10G8 | OR1L4 | NUDT7 |
| SPIC | TSPY26P | ACTRT2 | WFDC5 | PSORS1C2 | OR10G9 | MPV17L | TMEM114 |
| CSNK1A1L | HMGB3P1 | WFDC3 | RIMBP3C | SPACA4 | OR10S1 | HIST1H2BA | MILR1 |
| ANKRD9 | C7orf13 | ABHD16B | DNAJB7 | SCAMP5 | OR6T1 | OR52B2 | ANKRD20A9P |
| RNASE8 | OR9A4 | SMCR5 | CKAP2L | IMMP1L | OR4D5 | OR4C3 | ANKRD20A9P |
| OR4K14 | CLHC1 | RPL10L | TTC30B | OR56B4 | OR6Q1 | OR4S1 | ACTL9 |
| OR4L1 | FAM3D | KRT72 | FAM117B | DTX3 | OR9I1 | CDRT15L2 | ZNF844 |
| OR11H6 | LRRC15 | SCP2D1 | C3orf22 | CCER1 | OR9Q2 | LOC256880 | SCGB2B2 |
| PLA2G4E | C4orf33 | EIF5AL1 | PYDC2 | LINC00638 | OR1S2 | OR51F1 | OR10H5 |
| ZG16B | TRAM1L1 | OR52B4 | WWC2-AS2 | LCE4A | OR1S1 | C9orf43 | ZNF493 |
| OR4D2 | OR2Y1 | OR52I2 | FAM218A | LINC00466 | OR10Q1 | C2orf72 | OR14A16 |
| AFMID | AFAP1L1 | UBQLNL | DAB2IP | NUDT17 | OR5B17 | MRGPRX1 | LOC284578 |
| ZNF816 | GRPEL2 | LOC143666 | RAET1L | KRTCAP3 | OR5B21 | TAS2R39 | CYB561D1 |
| OR7D4 | GPR151 | KBTBD3 | IQUB | TIGD1 | OR5A2 | TAS2R40 | SIRPB2 |
| OR7G1 | SOWAHA | OR10A5 | CDCA2 | LRRC45 | OR5A1 | TAS2R41 | KRTAP13-4 |
| OR1M1 | TAAR9 | OR2AG1 | FAM84B | FBXO15 | OR4D6 | TAS2R43 | RPL23AP82 |
| COX6B2 | TAAR1 | A2ML1 | C8orf48 | ZBTB7C | OR4D11 | TAS2R46 | TPRG1 |
| EID2B | C6orf141 | ST13P4 | C9orf163 | TMEM184A | ATOH7 | TAS2R30 | DCAF4L1 |
| OR1I1 | HUS1B | ADAM21P1 | KIAA1958 | ADCK5 | CCDC89 | TAS2R19 | RNF175 |
| RINL | OR9A2 | BEAN1 | ZNF782 | HTRA4 | CTAGE10P | TAS2R20 | GPR150 |
| TCHHL1 | TMEM139 | SLC22A31 | ZNF645 | FAM226A | RNF152 | STEAP2 | OR2V2 |
| RPTN | OR2A14 | FLJ30679 | KRT19P2 | CDY2B | ZNF485 | POM121L4P | RNF180 |
| OR2M5 | OR6B1 | SLC35G3 | CLEC14A | PIP5K1P1 | REEP3 | NAP1L5 | PRR18 |
| OR2M3 | OR2F2 | KCTD11 | FITM1 | OR4C16 | HIST1 H2AA | PIPSL | ZNF789 |
| OR2T12 | KLF14 | TRIM16L | TMEM30B | OR4S2 | PRPS1L1 | RNU12 | TPI1P2 |
| OR14C36 | CLDN23 | KRT25 | STRC | OR4C6 | DKFZP586I1420 | IGBP1P1 | TMED10P1 |
| OR2T4 | LYPD2 | TMEM99 | PGBD4 | OR5D14 | NKAPL | OR5J2 | ZNF252P-AS1 |
| OR2T11 | ADHFE1 | SLC25A52 | ZFPM1 | OR5L1 | TOB2P1 | OR4C13 | C9orf47 |
| OR10J5 | DCAF4L2 | LINC00526 | MFSD6L | OR5AS1 | FERD3L | OR4C12 | TPRN |
| LRRC39 | TAF1L | ZNF534 | SPPL2C | OR8K5 | RPL23P8 | KRT8P41 | TUSC1 |

**[Table 7-23]**

| (A) Healthy vs AD (1911 genes) | | | | | | | |
|---|---|---|---|---|---|---|---|
| OR13C9 | DCAF12L2 | LCE1B | KRTAP10-12 | OR51B6 | OR4N5 | ZNF880 | CXADRP3 |
| FOXD4L3 | NANOS1 | LCE1C | MRGPRG | OR51M1 | OR11G2 | C3orf80 | WHAMMP2 |
| LOC286437 | EBLN1 | LCE1F | SLC7A5P2 | OR51Q1 | OR11H4 | LOC401127 | GOLGA6L9 |
| TTC3P1 | OR52B6 | LCE2A | C6orf120 | OR51I2 | OR5AU1 | SAPCD1 | HSBP1L1 |
| P2RY8 | OR2AT4 | LCE2C | KRTAP5-1 | OR52D1 | OR4M2 | OR2A7 | UQCRHL |
| P2RY8 | OR10A2 | LCE2D | KRTAP5-3 | OR52H1 | OR4N3P | OR2A20P | ETV3L |
| FAM223B | OR6C2 | LCE3E | KRTAP5-4 | OR52N4 | KBTBD13 | CD99P1 | ACTR3BP2 |
| VN1R2 | OR6C4 | SLCO4C1 | KRTAP5-10 | OR52N5 | OR4F6 | CD99P1 | MTHFD2L |
| VN1R4 | H1FNT | KRTAP12-2 | TMEM189 | OR52N2 | OR4F15 | ZNF674-AS1 | GUSBP5 |
| VN1R5 | OR6S1 | KRTAP12-1 | CC2D2B | OR52E6 | LOC390705 | OR51T1 | DUX4L4 |
| TAAR6 | SMTNL2 | KRTAP10-10 | CC2D2B | OR52E8 | OR7G2 | OR51A4 | CRSP8P |
| SERPINA9 | NACA2 | PCNAP1 | OR56B1 | OR52E4 | OR7G3 | OR2T2 | C6orf226 |
| HIST2H2BC | KRT27 | MRPL42P5 | GVINP1 | OR56A3 | OR7A10 | OR14I1 | LINC00602 |
| KRTAP7-1 | NCCRP1 | NANOGNB | CLEC12B | OR56A5 | OR10K2 | OR5K2 | ZNRF2P1 |
| KRTAP19-1 | ZNF284 | DND1 | REP15 | OR4X1 | OR10K1 | RPL21P44 | SPDYE7P |
| KRTAP13-2 | OR6F1 | KRT77 | RTL1 | OR5D13 | OR6Y1 | OR2A42 | OR2A9P |
| KRTAP13-3 | OR2W3 | ZNF699 | C2CD4B | OR5D16 | VSIG8 | UFSP1 | OR4F21 |
| KRTAP23-1 | OR2T3 | LOC375196 | GLTPD2 | OR5W2 | OR11L1 | OR2T27 | AARD |
| KRTAP6-2 | OR10R2 | GJB7 | C17orf100 | OR8H2 | OR2L8 | OR2T35 | AQP7P3 |
| KRTAP6-3 | OR2T29 | VWC2 | RPRML | OR8H3 | OR2AK2 | OR4A47 | SPATA31C1 |
| KRTAP19-2 | TGM6 | ENHO | C17orf82 | OR5T1 | OR2L3 | OR5H14 | LOC441454 |
| KRTAP19-3 | MSGN1 | AQP7P1 | ZNF788 | OR8K1 | OR2M2 | OR5H15 | LOC441455 |
| KRTAP19-4 | PRORSD1P | LRRC10 | KRTDAP | OR5M9 | OR2T33 | OR5K3 | PGAM4 |
| KRTAP19-5 | SOWAHB | RNF126P1 | ZNF808 | OR5M10 | OR2M7 | OR6C68 | OR9K2 |
| KRTAP19-6 | PFN3 | SLC27A1 | TRNP1 | OR5M1 | OR2G6 | YY2 | OR4M1 |
| KRTAP19-7 | FBLL1 | RPS10P7 | TMEM81 | OR9G1 | SUMO1P1 | C16orf74 | PGCP1 |
| KRTAP20-3 | ACTBL2 | SDC4P | CAPN8 | OR5AK2 | USP17L6P | LOC407835 | OR10J3 |
| IFNE | OR6V1 | USP17L2 | OR2M1P | OR5AN1 | LOC392196 | C10orf62 | OR2W5 |
| TAS2R60 | OR2A12 | NHLRC1 | TSPYL6 | OR4D10 | OR13J1 | DDI1 | OR2B3 |
| OR5F1 | OR2A1 | RNF148 | BOLA3 | OR4D9 | OR13C2 | BLID | OR2J3 |
| OR5AP2 | SLC10A5 | KRTAP10-4 | FUNDC2P2 | OR10V1 | OR1L6 | KRTAP5-5 | OR14J1 |
| OR52L1 | QRFP | KRTAP10-6 | OR6B2 | LRRC10B | OR5C1 | KRTAP5-2 | ATP6VOCP3 |
| OR2AG2 | KIF24 | KRTAP10-7 | PLSCR5 | OR6X1 | OR1K1 | KRTAP5-6 | SLC25A51P1 |
| C17orf51 | MPC1L | KRTAP10-9 | TMPRSS11F | OR6M1 | OR2A5 | KRTAP5-7 | GSTM2P1 |
| LRRC30 | DCAF8L2 | KRTAP10-1 | PROB1 | OR10G4 | SPRED3 | KRTAP5-11 | OR2A2 |
| RXFP4 | DCAF8L2 | KRTAP10-11 | C5orf46 | OR8A1 | MEX3D | CARD17 | FOXB2 |
| FAM58BP | OR13H1 | KRTAP10-2 | MAFA | OR6C1 | MKRN9P | LINC00167 | DPY19L2P4 |
| CYP27C1 | ECEL1P2 | KRTAP10-5 | IER5L | OR6C75 | ST20 | ZNF705A | H2AFB1 |
| LINC00299 | SERINC2 | KRTAP10-8 | OR52K1 | OR6C76 | FAM174B | FAM66C | GEMIN8P4 |
| IFITM4P | RBPMS2 | KRTAP10-3 | OR52I1 | OR6C70 | TOB1-AS1 | H3F3C | IGIP |
| ACER2 | RTN4RL2 | KRTAP12-3 | OR51D1 | OR4N2 | KCNJ2-AS1 | OR11H12 | RNASE12 |
| PABPC1L2A | LCE1A | KRTAP12-4 | OR52A5 | OR4K13 | SIGLEC16 | LOC440173 | LOC494127 |

**[Table 7-24]**

| (A) Healthy vs AD (1911 genes) | | | | | | | |
|---|---|---|---|---|---|---|---|
| FLJ25758 | RPL13AP3 | SNORA29 | PDZK1P1 | LOC100287042 | | | |
| ARGFXP2 | LINC00520 | SNORA30 | SNORA84 | SH3RF3-AS1 | | | |
| DPRXP4 | BASP1P1 | SNORA36A | SNORA36C | LOC100288069 | | | |
| LOC554206 | LOC646214 | SNORA38 | SNORA70B | KRTAP22-2 | | | |
| ASPDH | CXADRP2 | SNORA46 | SNORA70C | MTRNR2L7 | | | |
| PRR9 | ARIH2OS | SNORA47 | LOC100128164 | REXO1L2P | | | |
| MIR490 | LOC646471 | SNORA53 | LOC100128361 | LOC100288748 | | | |
| MIR181D | FABP9 | SNORA55 | CTAGE4 | LOC100288846 | | | |
| SNORA33 | LOC646903 | SNORA56 | LOC100128573 | LOC100289361 | | | |
| LOC606724 | LOC646999 | SNORA71C | LOC100129046 | LOC100289511 | | | |
| SNORA27 | C6orf132 | SNORA79 | FAM106CP | MIR1307 | | | |
| SNORA21 | PA2G4P4 | SNORA59A | MAPT-IT1 | MIR548I3 | | | |
| SNORA41 | CTAGE11P | SCARNA 14 | CXorf49 | MIR548I1 | | | |
| RPL31P11 | ACTG1P4 | ANP32AP1 | LINC00552 | MIR548I2 | | | |
| FAM138F | PPP1R3G | LINC00163 | LOC100130673 | NDUFAF4P1 | | | |
| LOC641746 | LOC648987 | LOC728024 | SYCE1L | LOC100335030 | | | |
| LOC642361 | RPL23AP64 | TSPY3 | LOC100130992 | SNORA70F | | | |
| SMIM5 | LOC650293 | KRTAP2-2 | DBIL5P | SNORA70D | | | |
| TMEM72 | UCA1 | KRTAP19-8 | LOC100131496 | SNORA70E | | | |
| KIAA0754 | NBPF6 | KRTAP9-1 | LOC100132287 | MTRNR2L1 | | | |
| C15orf62 | RPSAP9 | USP17L5 | LOC100132356 | MTRNR2L3 | | | |
| LOC643339 | OR1D4 | TPI1P3 | FAM157B | MTRNR2L4 | | | |
| LOC643387 | KRTAP4-11 | OPN1MW2 | CLDN24 | MTRNR2L5 | | | |
| SCGB1B2P | ASAH2B | LOC728613 | LOC100132831 | MTRNR2L6 | | | |
| UG0898H09 | FOXD4L6 | ASB9P1 | DPH3P1 | MTRNR2L8 | | | |
| KRTAP24-1 | PPIAL4A | SKP1P2 | FAM223A | LOC100499489 | | | |
| KRTAP27-1 | PPIAL4C | FAM133CP | SBF1P1 | LOC100500773 | | | |
| CTAGE9 | HIST2H3D | LOC728739 | LOC100133331 | MIR3907 | | | |
| SDHAF1 | GOLGA6C | LOC728752 | UBE2Q2P2 | KRTAP16-1 | | | |
| LOC644189 | LOC653653 | PPIAL4F | JMJD7 | GAS5-AS1 | | | |
| LINC00622 | SNORA8 | LOC729080 | KLLN | LOC100506083 | | | |
| KANSL1-AS1 | SNORA75 | PRR23A | KRTAP20-4 | KRBOX1 | | | |
| LOC644656 | LOC654342 | MRS2P2 | DBIL5P2 | LOC100506730 | | | |
| CLDN25 | SCARNA18 | ZNF878 | SRRM5 | TPBGL | | | |
| CDRT15P2 | SCARNA1 | SEC14L1P1 | TPT1-AS1 | MKNK1-AS1 | | | |
| LOC644936 | SCARNA15 | TMEM229A | PP12613 | RAB11B-AS1 | | | |
| SNRPD2P2 | SNORA1 | GSTA7P | FLJ16779 | LOC100507634 | | | |
| TMEM200C | SNORA2A | PPIAL4E | LOC100240734 | MARK2P9 | | | |
| FUT8-AS1 | SNORA2B | SNHG9 | LOC100270746 | CAHM | | | |
| FLJ42627 | SNORA5B | PFN1P2 | LOC100270804 | DNM3OS | | | |
| ASH1L-AS1 | SNORA14A | PSAPL1 | LOC100272217 | | | | |
| POU5F1P4 | SNORA14B | PWRN2 | LOC100286922 | | | | |

### Test Example 9: Prediction of skin condition using SSL-derived RNA

### Subjects

39 healthy females (age: 30s) having no problem on the skin of the face, the fingers or the upper arms were selected as subjects.

### Collection of sebum

Using an oil blotting film (5 cm × 8 cm, 3M Ltd.), sebum was collected from the entire face of each subject before washing of the face, and preserved as a sample for analysis of SSL-derived RNA at -80°C for about 1 month.

### Visual evaluation and palpatory evaluation of skin

After the sebum was collected, the subjects each washed the face using a commercially available facial cleanser, and conditioned in a variable-environment room (temperature: 20°C ± 1°C and humidity: 40% ± 5%). During the conditioning, the skin condition of the face of each of the subjects was evaluated visually and on palpation.
- Visual evaluation items: "cleanness", "clearness", "lightness", "yellowness", "overall redness", "flecks", "scale", "luster", "textured wrinkles on the cheek", "conspicuous dark circles", "drooping corners of the mouth", "acne", "conspicuous pores (cheek)" and "conspicuous pores (nose)"
- Palpatory evaluation: "rough feeling" and "moist feeling"

For each evaluation item, three professional evaluators marked scores on the basis of criteria (3: very heavy, 2: heavy, 1: slightly heavy, 0: none), and an average of the scores by the three evaluators was defined as an evaluation value.

### Measurement of skin physical properties

From each of the subjects after completion of the conditioning, the horn cell layer moisture content was measured with Corneometer (MPA580, Courage + Khazaka Electronic GmbH, Germany) and Skicon (YOYOI Co., Ltd.), the transepidermal water loss (TEWL) was measured with Tewameter (MPA580, Courage + Khazaka Electronic GmbH, Germany), the amount of sebum was measured with Sebumeter (MPA580, Courage + Khazaka Electronic GmbH, Germany), and the amount of melanin and the amount of erythema were measured with CM26000d (KONICA MINOLTA, INC.). The amount of sebum was measured on the forehead, and all the others were measured.

### Sebum composition analysis

After a lapse of 1 hour or more from the washing of the face, the subject was caused to lie supine, and two sheets of cigarette paper (1.7 cm × 1.7 cm, RIZLA: RIZLA BLUE DOUBLE) degreased with chloroform/methanol = 1/1 were arranged near the center of the forehead so as not to overlap each other, and lightly pressed against the forehead for 10 seconds to collect sebum. The cigarette paper containing the sebum was put into a screw tube, methanol was immediately added, and the cigarette paper was cryogenically preserved at -80°C until analysis.

The solvent was removed from the screw tube by distillation under the nitrogen flow, and 1 mL of chloroform/methanol = 1/1 was then added into the screw tube. After it was confirmed that the cigarette paper was sufficiently immersed in the solvent in the screw tube, sebum was extracted by ultrasonic treatment for 5 minutes to obtain a sebum solution. In a very small vial, 20 µL of a lipid internal standard solution for direct-MS/MS measurement at 100 µmol/L was solidified by drying, 100 µL of the sebum solution prepared in accordance with the above-described procedure was added thereto, dissolved and mixed to prepare a sebum sample solution containing an internal standard. From the prepared sample solution, the amounts of free fatty acid (FFA), wax ester (WE), cholesterol ester (ChE), squalene (SQ), squalene epoxide (SQepo), squalene oxide (SQOOH), diacylglycerol (DAG) and triacylglycerol (TAG) were measured for each subject by direct-MS/MS, and absolute amounts were calculated on the basis of the internal standard.

### <Direct-MS/MS measurement conditions>

In accordance with the method described in a document (JP-B-6482215), measurement was performed under the following conditions.

Instrument: LC/Agilent 1200 series, mass spectrometer/6460 triple quadrupole (manufactured by Agilent Technologies)
Mobile phase: 15 mmol/L ammonium acetate-containing chloroform/methanol = 1/1
Flow rate: 0.2 mL/min
Injection volume: 1 µL
Detection conditions: ionization method = ESI, dry gas temperature = 300°C, dry gas flow rate = 5 L/min, nebulizer pressure = 45 psi, sheath gas flow rate = 11 L/min, nebulizer voltage = 0 V, capillary voltage = 3,500 V

### (Detection mode of mass spectrometer)

FFA: Scan (Negative mode)
WE: Precursor Ion Scan for detecting molecules from constituent fatty acid-derived product ions
ChE: Precursor Ion Scan for detecting molecules from cholesterol backbone-derived product ions
SQ, SQepo, SQOOH: MRM
DAG: Neutral Loss Scan for detecting molecules from desorbed hydroxyl groups
TAG: Neutral Loss Scan for detecting molecules from fatty acids desorbed as neutral molecules

### Preparation of SSL-derived RNA and sequence analysis

From the preserved oil blotting film, RNA in SSL was extracted in accordance with the same procedure as in Test Example 3, a library was prepared, RNA species were identified through sequencing, and the expression levels of the RNA species were measured.

### Construction of machine learning model

### - Data used

In the data of the expression levels of SSL-derived RNAs from the subjects (read count values), data with a read count of less than 10 was set as a missing value, and converted into a RPM value corrected for a difference in the total number of reads between samples, and the missing value was then supplemented by singular value decomposition (SVD) imputation. Only genes for which expression data that is not a missing value had been obtained in 80% or more of all the subjects was used for the following analysis. For construction of the machine learning model, RPM values converted into base-2 logarithmic values (Log₂ RPM values) were used for approximating RPM values following a negative binomial distribution to a normal distribution. Evaluation values and measured values for the prediction target items (data from the visual and palpatory evaluation of the skin, the measurement of skin physical properties and the skin composition analysis; Table 8) were converted into deviation values in the target data sets, which were defined as target values.

### - Division of data set

Of the RNA profile data set obtained from the subjects, RNA profile data for 31 subjects, which amounts to 80% of the data set, was used as training data for skin condition prediction models, and RNA profile data for the other 8 subjects, which amounts to 20% of the data set, was used as test data for evaluation of model accuracy. For dividing the data set into the training data and the test data, a division method giving a uniform age distribution (division 1) and a division method giving uniform target values in prediction target items (division 2) were examined.

### - Selection of feature genes

In the training data, target values in the prediction target items and absolute values of Spearman's correlation coefficients (rho) of Log₂ RPM values were calculated, and the top 10 genes (or 5 genes) shown in Table 8 were selected as feature genes in the prediction target items.

### - Model construction

Model construction was performed using the caret package of Statistical Analysis Environment R.
-- The data of the expression level of SSL-derived RNA (Log₂ RPM value) in the training data was used as an explanatory variable, and the target value in each of the prediction target items was used as an objective variable to construct a prediction model.
-- For each prediction target item, the prediction model was made to learn by performing 10-fold cross validation using 6 algorisms which are linear regression model (Linear model), Lasso regression (Lasso), random forest (Random Forest), neural network (Neural net), linear kernel support vector machine (SVM (linear)) and rbf kernel support vector machine (SVM (rbf)).
-- For each algorism, the expression level of SSL-derived RNA (Log₂ RPM value) in the test data was input to the model after learning to calculate the target predicted value in each prediction item.
-- For each prediction item, the root-mean-square-error (RMSE) of a difference between a predicted value and a measured value was calculated, and the model giving the smallest value of RMSE was selected as an optimum prediction model.

**[Table 8]**

| **Prediction target item** | Symbol001 | Symbol002 | Symbol003 | Symbol004 | Symbol005 | Symbol006 | Symbol007 | Symbol008 | Symbol009 | Symbol010 |
|---|---|---|---|---|---|---|---|---|---|---|
| **Horn cell layer moisture content (Comeo)** | HIPK2 | HK2 | DDX5 | ARHGEF10L | ASAP1 | VPS26A | SNORA5C | LONP1 | TMSB10 | NCOA1 |
| **Horn cell layer moisture content (Skicon)** | SNORA5C | ASAP1 | HK2 | ANKRD28 | DDX5 | CHIC2 | HIPK2 | COX4I1 | SNORA71C | LONP1 |
| TEWL | SNORA62 | ACTN1 | DYRK1A | GSTO1 | EIF2AK1 | KIAA1432 | DSCR3 | LSM14A | CCND3 | SLC30A1 |
| **Amount of sebum** | FRMD8 | DPM1 | UBE2J1 | SNORA68 | CCL17 | DRG1 | DYRK1A | SPCS2 | ESYT2 | NR2C2 |
| **Amount of melanin** | ABCA11P | KRT13 | PNN | CIRBP | DIS3 | CHI3L1 | BLOC1S6 | MSRB1 | VIM | FPR3 |
| **Amount of erythema** | TMEM164 | SDF4 | STEAP4 | UBR2 | ACAP2 | TMEM154 | DDX60 | DSCR3 | LOC 1005067 | TCIRG1 |
| **Cleanness** | SPRED2 | VIM | RAB7A | HIST1H3D | YIPF5 | NSMCE1 | RNF6 | POGLUT1 | CCRN4L | C4orf34 |
| **Clearness** | BIRC3 | NR4A3 | RGS1 | POGLUT1 | ARPC2 | SPCS2 | SCARNA16 | CD1E | TMEM66 | HLA.DRA |
| **Lightness** | LOC440173 | ANKLE2 | NPM1 | VIM | ABCA11P | FTX | WDR33 | AHNAK | CD80 | MINK1 |
| **Luster** | SRSF6 | GOLT1B | CCT4 | HSP90AB1 | LIMD1 | ABCA11P | MAGT1 | SDC4 | SPCS3 | KAT7 |
| **Flecks** | PPFIA1 | DCP1A | RLF | CRLF3 | PLEKHF2 | | | | | |
| **Conspicuous dark circles** | SLC20A1 | RNASET2 | PSMB10 | ARL2 | AVL9 | SLMAP | MCC | TSPAN3 | STARD3NL | VMP1 |
| **Yellowness** | STAG1 | PPP6R3 | VCPIP1 | NEDD4L | STXBP3 | SCGB2A2 | FTX | BMP2 | CCDC93 | SCAF11 |
| **Overall redness** | NBEALZ | PDPR | GLRX | TMEM164 | CCDC88B | LMBRDI | DNAJC3 | NAIP | MEAF6 | NBR1 |
| **Textured wrinkles on the cheek** | STX4 | C10orf76 | BCL2L13 | CYTH1 | MAL | BAX | SNORA5C | J0SD1 | ABHD16A | DBNL |
| **Droopinq corners of the mouth** | RTN4 | MKNK2 | RAP1A | ATF6B | CAST | TPT1 | OS9 | C17orf62 | HN1 | ARHGEF2 |
| **Scale** | MTA3 | CYBASC3 | RAP1GAP | CLIC3 | PRR24 | STK17A | GNPAT | GAN | SASH1 | MINA |
| **Acne** | PARP8 | DPYD | LOC1004991 | TLK2 | STAT2 | HIAT1 | TPR | RNF213 | EZH1 | ADAR |
| **Pores (cheek)** | AGAP3 | GBA | SNX27 | MGC12916 | RBM22 | PSMA5 | LOC1005067 | KXD1 | SYAP1 | NUCB1 |
| **Pores (nose)** | TIFA | DCTN2 | HOMER3 | SCYL1 | NAA50 | COPG1 | SPOPL | EPAS1 | DTX2 | PSMD7 |
| **Rough feeling** | FAM83G | ATP11A | SCYL2 | UBE2S | BMP2 | GLRX5 | KIAA0930 | KRT25 | ACAD8 | SERTAD1 |
| **Moist feeling** | LOC349196 | FOX04 | DHRS1 | RNF10 | ACAD8 | MRPL49 | RAB11B | HIST3H2A | KRT72 | REX01L2P |
| **FFA** | RASGEF1B | ABCE1 | PDE8A | SLC7A5P1 | PPP4C | RAP1B | HDAC5 | EIF5B | PAFAH1BZ | INF2 |
| **WC** | DPM1 | DDX27 | GBA | ABCE1 | LYZ | DUSP11 | RAP1B | MATR3 | TRIM28 | SPRED2 |
| **ChE** | GBA | DDX27 | DPM1 | MARCKSL1 | TRIM28 | DUSP11 | ABCE1 | RSU1 | AKAP11 | DYNC1LI1 |
| **SQ** | GBA | CYCSP52 | DDX27 | CBX3 | MARCKSL1 | LYZ | DUSP11 | DPM1 | SLC11A1 | RAP1B |
| **SQepo** | RALGAPB | EIF3H | PAFAH1B2 | MRPL23 | SNRPF | FBXL3 | GBA | CDC123 | NCOA3 | POLDIP3 |
| **SQOOH** | ABCE1 | HDAC5 | EIF5B | PPP4C | RASGEF1B | PDE8A | SLC11A1 | MAP4K3 | RAP1B | KHSRP |
| **DAG** | KDM3A | NRARP | ERO1L | PPAP2A | GPATCH2 | PFDN1 | COPS3 | CYFIP2 | INF2 | VAMP2 |
| **TAG** | DDX27 | GBA | DUSP11 | NUDT16 | TRIM28 | AKAP 11 | DPM1 | YWHAG | TUBGCP6 | GPR108 |

### (Results)

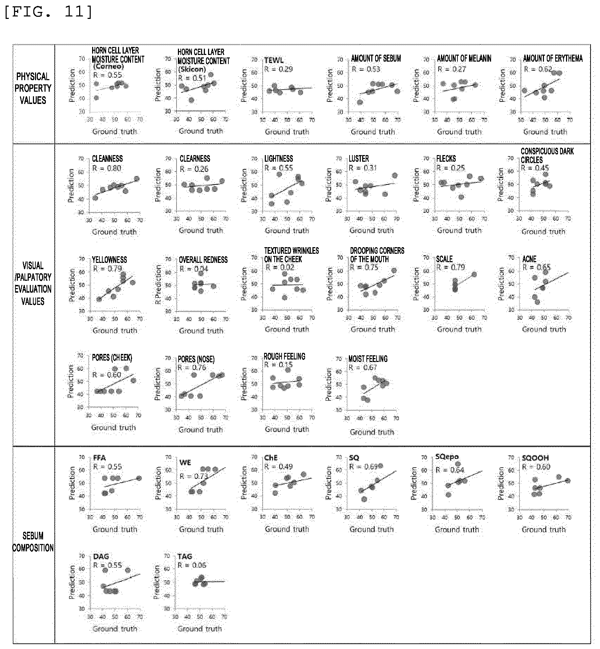

Table 9 shows the data division method giving the smallest RMSE, the algorism used and RMSE for each prediction target item. Figure 11 shows a scatter chart obtained by plotting the predicted and measured target values in the optimum prediction model. R in the figure represents a correlation coefficient in Pearson's correlational analysis of the predicted value and the measured value. In all the prediction target items, a positive correlation coefficient was obtained, so that it was possible to predict a skin condition using data of the expression level of SSL-derived RNA.

**[Table 9]**

| Prediction target item | Data set division method | Optimum algorism | RMSE |
|---|---|---|---|
| Horn cell layer moisture content (Corneo) | Division 1 | Lasso | 7.14 |
| Horn cell layer moisture content (Skicon) | Division 1 | Lasso | 7.89 |
| TEWL | Division 1 | SVM(rbf) | 11.05 |
| Amount of sebum | Division 1 | Randon forest | 11.88 |
| Amount of melanin | Division 1 | SVM(rbf) | 7.76 |
| Amount of erythema | Division 1 | SVM(rbf) | 6.60 |
| Cleanness | Division 2 | Randon forest | 10.67 |
| Clearness | Division 2 | SVM(rbf) | 9.94 |
| Lightness | Division 1 | SVM(linear) | 8.63 |
| Luster | Division 1 | Randon forest | 9.64 |
| Flecks | Division 2 | SVM(rbf) | 12.66 |
| Conspicuous dark circles | Division 1 | Lasso | 5.76 |
| Yellowness | Division 1 | Randon forest | 5.60 |
| Overall redness | Division 1 | SVM(rbf) | 6.22 |
| Textured wrinkles on the cheek | Division 1 | Randon forest | 9.35 |
| Drooping corners of mouth | Division 1 | SVM(linear) | 6.12 |
| Scale | Division 1 | SVM(linear) | 3.26 |
| Acne | Division 2 | Lasso | 7.48 |
| Pores (cheek) | Division 2 | SVM(rbf) | 9.27 |
| Pores (nose) | Division 1 | Neural net | 7.82 |
| Rough feeling | Division 1 | Lasso | 8.79 |
| Moist feeling | Division 1 | Randon forest | 6.81 |
| FFA | Division 1 | Linear model | 10.81 |
| WE | Division 1 | Neural net | 6.99 |
| ChE | Division 1 | Randon forest | 9.82 |
| SQ | Division 1 | SVM(linear) | 8.85 |
| SQepo | Division 1 | Linear model | 10.09 |
| SQOOH | Division 1 | Linear model | 10.45 |
| DAG | Division 2 | Randon forest | 13.27 |
| TAG | Division 1 | Lasso | 10.00 |

### Test Example 10: Prediction of blood cortisol concentration using SSL-derived RNA

### Subjects

128 healthy females (age: 20s to 50s) having no problem on the skin of the face, the fingers or the upper arms were selected as subjects.

### Collection of sebum and sequencing of SSL-RNA

Using an oil blotting film (5 cm × 8 cm, 3M Ltd.), sebum was collected from the entire face of each subject before washing of the face, and preserved as a sample for analysis of SSL-derived RNA at -80°C for about 1 month. From the preserved oil blotting film, RNA in SSL was extracted in accordance with the same procedure as in Test Example 3, a library was prepared, RNA species were identified through sequencing, and the expression levels of the RNA species were measured.

Measurement of blood cortisol concentration 15 mL of blood was collected from the arm of each subject using a vacuum blood collection tube, and serum was separated and preserved at -80°C. An external inspection organization (LSI Medience Corporation) was commissioned to determine the concentration of cortisol in the preserved serum by a chemiluminescent immunoassay method (CLIA method).

### Construction of machine learning model

### - Data used

As in Test Example 9, data with a read count of less than 10 in the data of the expression levels of SSL-derived RNAs from the subjects (read count values) was set as a missing value, and converted into a RPM value corrected for a difference in the total number of reads between samples, and the missing value was then supplemented by SVD imputation. Only genes for which expression data that is not a missing value had been obtained in 80% or more of all the subjects was used for analysis. Log₂ RPM values were used as the expression level data.

### - Division of data set

From the RNA profile data set obtained from the subjects, RNA profile data for 102 subjects, which amounts to 80% of the data set, was randomly extracted, and used as training data for blood cortisol concentration prediction models. RNA profile data for the other 26 subjects, which amounts to 20% of the data set, was used as test data for evaluation of model accuracy.

### - Selection of feature genes

1,000 genes having a large Pearson's correlation coefficient with the blood cortisol concentration in the training data were extracted.

### - Algorithms used (hyperparameter candidate values)

Support vector machine (C: [0.1, 1, 10], kernel: ['linear', 'rbf' and 'poly'])
Random forest (max depth: [1,2,3], max_features: [1,2], n_estimators: [10, 100])
Multilayer perceptron (solver: ['lbfgs', 'adam', alpha: [0.1,1,10])

### - Model construction

Model construction was performed using the machine learning library scikit-learn of Python.
-- The prediction model was made to learn by performing 10-fold cross validation, where the data of the expression level of SSL-derived RNA (Log₂ RPM value) in the training data was used as an explanatory variable, and the blood cortisol concentration was used as an objective variable.
-- In the cross validation, the data of the expression levels of 1,000 genes extracted as feature genes was compressed to first to tenths main components by main component analysis, and the model was then made to learn while grid search was performed for each algorism and hyperparameter candidate value.
-- The expression level of SSL-derived RNA (Log₂ RPM value) in the test data was input to each model after learning to calculate the predicted value, and the model giving the smallest RMSE of the difference between the predicted value and the measured value was selected as an optimum prediction model.

### (Results)

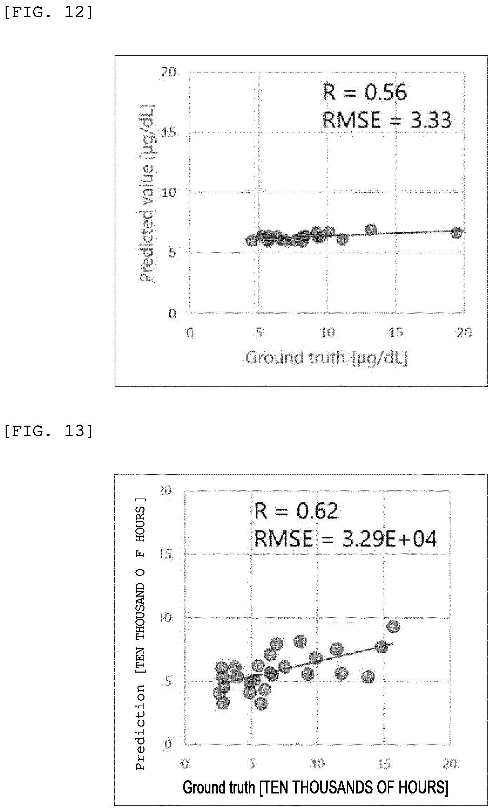

Figure 12 shows a scatter chart in which the predicted value of the blood cortisol concentration obtained by inputting the expression level of SSL-derived RNA (Log₂ RPM value) in the test data to the prediction model giving the smallest RMSE and using random forest (max_depth = 2, max_feature = 2 and n_estimator = 100) is plotted with respect to the measured value. The correlation coefficient (R) in Pearson's correlational analysis of the predicted value and the measured value, and the RMSE value are shown in the figure. As shown in the figure, a positive correlation coefficient was obtained, so that it was possible to predict the blood cortisol concentration using the data of the expression level of SSL-derived RNA.

### Test Example 11: Prediction of cumulative ultraviolet exposure time using SSL-derived RNA

130 healthy females (age: 20s to 50s) having no problem on the skin of the face, the fingers or the upper arms were selected as subjects.

### Collection of sebum and sequencing of SSL-RNA

Using an oil blotting film (5 cm × 8 cm, 3M Ltd.), sebum was collected from the entire face of each subject before washing of the face, and preserved as a sample for analysis of SSL-derived RNA at -80°C for about 1 month. From the preserved oil blotting film, RNA in SSL was extracted in accordance with the same procedure as in Test Example 3, a library was prepared, RNA species were identified through sequencing, and the expression levels of the RNA species were measured.

### Calculation of cumulative ultraviolet exposure time

A standard time during which subjects in a certain range of ages had been exposed to sunlight was predicted on the basis of questionary studies on the lifestyle habit and outdoor leisure activity, and the cumulative ultraviolet exposure time (hour) was calculated with consideration given to an actual age. The questionary items for the questionary studies were prepared on the basis of the questionnaire on the light exposure history which is published in National Cancer Institute (Arch. Dermatol. 144, 217-22 (2088)).

### Construction of Machine Learning Model

As in Test Example 9, data with a read count of less than 10 in the data of the expression levels of SSL-derived RNAs from the subjects (read count values) was set as a missing value, and converted into a RPM value corrected for a difference in the total number of reads between samples, and the missing value was then supplemented by SVD imputation. Only genes for which expression data that is not a missing value had been obtained in 80% or more of all the subjects was used for analysis. Log₂ RPM values were used as the expression level data.

### - Division of data set

From the RNA profile data set obtained from the subjects, RNA profile data for 104 subjects, which amounts to 80% of the data set, was randomly extracted, and used as training data for cumulative ultraviolet exposure time prediction models. RNA profile data for the other 26 subjects, which amounts to 20% of the data set, was used as test data for evaluation of model accuracy.

### - Selection of feature genes

1,000 genes having a large Pearson's correlation coefficient with the cumulative ultraviolet exposure time in the training data were extracted. In addition to these 1,000 genes, the ages of the subjects were added to the feature.

### - Algorithms used (hyperparameter candidate values)

Algorithms identical to those in Test Example 10 were used.

### - Model construction

10-fold cross validation was performed in the same manner as in Test Example 10, and the model giving the smallest RMSE of the difference from the measured value was selected as an optimum prediction model. In addition to the 1,000 genes selected above as features, the ages of the subjects were used.

### (Results)

Figure 13 shows a scatter chart in which the predicted value of the cumulative ultraviolet exposure time obtained by inputting the expression level of SSL-derived RNA (Log₂ RPM value) in the test data to the prediction model giving the smallest RMSE and using support vector machine (C = 10, kernel = 'linear') is plotted with respect to the calculated value based on the questionary studies. The correlation coefficient (R) in Pearson's correlational analysis of the predicted value and the measured value, and the RMSE value are shown in the figure. As shown in the figure, a positive correlation coefficient was obtained, so that use of the data of the expression level of SSL-derived RNA enabled prediction of the cumulative ultraviolet exposure time without depending on the questionary studies.

## Claims

1. A method for preparing a nucleic acid derived from a skin cell of a subject, the method comprising preserving at 0°C or lower an RNA-containing skin surface lipid collected from the subject.

2. A method for preparing a nucleic acid derived from a skin cell of a subject, the method comprising:
converting RNA has been contained in a skin surface lipid of the subject into cDNA by reverse transcription, and then subjecting the cDNA to multiplex PCR; and
purifying a reaction product of the PCR.

3. The method according to claim 2, wherein a temperature for annealing and elongation reaction in the multiplex PCR is 62°C ± 1°C.

4. The method according to claim 2 or 3, wherein the elongation reaction in the reverse transcription is carried out at 42°C ± 1°C for 60 minutes or more.

5. The method according to any one of claims 2 to 4, wherein the RNA has been contained in the skin surface lipid of the subject is prepared by separating the RNA from the skin surface lipid of the subject.

6. The method according to any one of claims 2 to 5, wherein the skin surface lipid of the subject is one preserved at 0°C or lower.

7. A method for analyzing a condition of a skin, a part other than the skin or the whole body of a subject, the method comprising analyzing a nucleic acid prepared by the method according to any one of claims 1 to 6.

8. The method according to claim 7, wherein the analysis is detection of a skin with redness, sensitive skin or atopic dermatitis or a skin without redness, sensitive skin or atopic dermatitis, or detection of a skin with a large or small amount of sebum or skin moisture content.

9. The method according to claim 7, wherein the analysis is estimation or prediction of a skin condition.

10. The method according to claim 9, wherein the estimation or prediction of the skin condition is estimation or prediction of a skin physical property, estimation or prediction of visual or palpatory evaluation of the skin, or prediction of a sebum composition.

11. The method according to claim 7, wherein the analysis is estimation or prediction of a cumulative ultraviolet exposure time.

12. A method for evaluating an effect or efficacy of a skin external preparation, an intracutaneously administered preparation, a patch, an oral preparation or an injection on a subject, the method comprising analyzing a nucleic acid prepared by the method according to any one of claims 1 to 6.

13. A method for analyzing a concentration of a component in the blood of a subject, the method comprising analyzing a nucleic acid prepared by the method according to any one of claims 1 to 6.

14. The method according to claim 13, wherein the component in the blood is a hormone, insulin, neutral fat, γ-GTP or LDL-cholesterol.
